(19)

Europäisches Patentamt
European Patent Office
Office européen des brevets

(11) **EP 4 484 956 A2**

(12) **EUROPEAN PATENT APPLICATION**

(43) Date of publication:
**01.01.2025 Bulletin 2025/01**

(21) Application number: **24210431.3**

(22) Date of filing: **29.01.2021**

(51) International Patent Classification (IPC):
**G01N 33/569** (2006.01)

(52) Cooperative Patent Classification (CPC):
**G01N 33/56983; G01N 33/68;** G01N 2333/165;
G01N 2333/522; G01N 2333/70575; G01N 2800/50;
G01N 2800/52; G01N 2800/56

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 29.01.2020 US 202062967409 P
13.02.2020 US 202062975802 P
08.04.2020 US 202063006758 P
23.04.2020 US 202063014214 P
04.05.2020 US 202063019451 P
28.05.2020 US 202063030937 P
30.09.2020 US 202063085189 P

(62) Document number(s) of the earlier application(s) in
accordance with Art. 76 EPC:
**21747659.7 / 4 097 485**

(71) Applicant: **MeMed Diagnostics Ltd.
3508504 Tirat HaCarmel (IL)**

(72) Inventors:
• **MASTBOIM, Niv Steven
3501801 Haifa (IL)**
• **SHAHAM, Oded
3440713 Haifa (IL)**
• **NAVON, Roy
6423313 Tel-Aviv (IL)**
• **ILAN BER, Tahel
4528051 Hod Hasharon (IL)**

• **LEV, Shaul
4668636 Herzliya (IL)**
• **BARASH, Eran
6424414 Tel Aviv (IL)**
• **GOTTLIEB, Tanya Michelle
6522012 Tel Aviv (IL)**
• **PAZ, Meital
3475957 Haifa (IL)**
• **BOICO, Olga
3033561 Atlit (IL)**
• **OVED, Kfir
3087500 Hof HaCarmel (IL)**
• **EDEN, Eran
3498233 Haifa (IL)**

(74) Representative: **Mathys & Squire
The Shard
32 London Bridge Street
London SE1 9SG (GB)**

Remarks:
•The complete document including Reference
Table(s) and the Sequence Listing(s) can be
downloaded from the EPO website
•This application was filed on 01-11-2024 as a
divisional application to the application mentioned
under INID code 62.

(54) **METHODS OF DIAGNOSING AND CLASSIFYING VIRAL INFECTIONS**

(57) A method of classifying the severity of a coronaviral infection of a subject is disclosed. The method comprises measuring the TRAIL and/or IP IO protein level in a body liquid sample of the subject, wherein the level is indicative of the severity of the coronaviral infection. Managing treatments for coronaviral infections are also disclosed as well as measuring contagiousness of infections.

FIG. 8A

EP 4 484 956 A2

**Description**

RELATED APPLICATIONS

This application claims the benefit of priority of US Patent Application No. 62/967,409 filed 29 January, 2020, US Patent Application No. 62/975,802 filed 13 February, 2020 and US Patent Application No. 63/019,451 filed 4 May, 2020, the contents of which are incorporated herein by reference in their entirety. This application further claims the benefit of priority of US Patent Application No. 63/006,758 filed 8 April, 2020, 63/014,214 filed 23 April, 2020, 63/030,937 filed 28 May, 2020 and 63/085,189 filed 30 September 2020.

SEQUENCE LISTING STATEMENT

The ASCII file, entitled 86297 Sequence Listing.txt, created on 29 January 2021, comprising 57,685 bytes, submitted concurrently with the filing of this application is incorporated herein by reference.

FIELD AND BACKGROUND OF THE INVENTION

The present invention, in some embodiments thereof, relates to methods of diagnosing and classifying the severity of viral infections and more specifically to coronaviral infections.

Disease assessment is one of the most important tasks in management of patients. Predicting patient prognosis may affect various aspects of patient management including treatment, diagnostic tests (e.g., microbiology, blood chemistry, radiology etc.), and admission. Timely identification of patients with higher chance for poor prognosis may result in more aggressive patient management procedures including for example, intensive care unit (ICU) admission, advanced therapeutics, invasive diagnostics or surgical intervention, which could reduce complications and mortality.

WO 2013/117746 teaches biomarkers including TNF-related apoptosis-inducing ligand (TRAIL) for distinguishing between a bacterial and viral infection.

Additional background art includes WO 2016/024278, WO2018/060998 and WO2018/060999.

SUMMARY OF THE INVENTION

According to an aspect of the present invention, there is provided a method of classifying the severity of a coronaviral infection of a subject, comprising measuring the TRAIL and/or IP10 protein level in a body liquid sample of the subject, wherein the level is indicative of the severity of the coronaviral infection.

According to embodiments of the invention the subject does not present with symptoms of an infectious disease.

According to embodiments of the invention the subject presents with symptoms of an infectious disease.

According to embodiments of the invention the symptoms comprise fever.

According to embodiments of the invention the method further comprises measuring the level of at least one determinant set forth in Table 1.

According to embodiments of the invention the at least one determinant is selected from the group consisting of PCT, IL-6 and CRP.

According to embodiments of the invention the method further comprises measuring the level of at least one determinant selected from the group consisting of ferritin, d-dimer and lactate.

According to embodiments of the invention the method further comprises measuring the level of CRP.

According to embodiments of the invention the method further comprises treating the subject who has been classified as severe with a management selected from the group consisting of oxygen therapy, non-invasive ventilation, mechanical ventilation, invasive monitoring, last-resort drug, sedation, intensive care admission, surgical intervention, hospital admittance, anti-viral drug, anti-viral regimen, anti-bacterial drug, anti-fungal drug, immune-globulin treatment isolation, glucocorticoid therapy, extracorporeal membrane oxygenation and kidney replacement therapy.

According to embodiments of the invention the coronavirus of the coronaviral infection is severe acute respiratory syndrome coronavirus (SARS-CoV-2) and Middle East respiratory syndrome coronavirus (MERS-CoV).

According to an aspect of the present invention, there is provided a method of managing treatment of a disease which brings about a dysregulation of an immune and/or inflammatory response of a subject, the subject already being medicated with a first treatment to treat the disease comprising:

(a) measuring an amount of IP10 and/or TRAIL in a sample of the subject; and
(b) altering the treatment or treatment course when the level of IP10 is above a predetermined threshold and/or when the level of TRAIL is below a predetermined threshold in the sample.

According to embodiments of the invention the disease is a viral disease.

According to embodiments of the invention the viral disease is a respiratory viral disease.

According to embodiments of the invention the respiratory viral disease is COVID-19.

According to embodiments of the invention the respiratory viral disease comprises acute respiratory distress syndrome (ARDS).

According to embodiments of the invention the virus of the respiratory viral disease is selected from the group consisting of RSV, Flu A, Flu B, HCoV and SARS-Cov-2.

According to embodiments of the invention the predetermined threshold for IP10 is above 1000 pg/ml.

According to embodiments of the invention the method further comprises determining the length of time for which the level of IP10 is above the predetermined threshold, wherein the length of time is indicative that the treatment or treatment course should be altered.

According to embodiments of the invention the predetermined threshold for TRAIL is below 25 pg/ml.

According to embodiments of the invention the method further comprises determining the length of time for which the level of TRAIL is below a predetermined threshold, wherein the length of time is indicative that the treatment or treatment course should be altered.

According to embodiments of the invention the subject is hospitalized.

According to embodiments of the invention the subject is non-hospitalized.

According to embodiments of the invention the subject is in intensive care.

According to embodiments of the invention the method further comprises re-measuring the amount of IP10 and/or TRAIL following the altering

According to embodiments of the invention the re-measuring is effected no more than 12 hours following the altering the treatment.

According to embodiments of the invention, steps (a) and (b) are repeated at least three times during the course of the disease.

According to embodiments of the invention the first treatment comprises administering a therapeutic agent.

According to embodiments of the invention the first therapeutic agent comprises a steroid agent.

According to embodiments of the invention the therapeutic agent comprises an agent which reduces the activity or amount of IL-6.

According to embodiments of the invention the altering the treatment comprises altering the dose of the therapeutic agent based on the level of IP10 and/or TRAIL.

According to embodiments of the invention step (b) is effected no more than 12 hours following step (a).

According to embodiments of the invention the altering the treatment comprises terminating the first treatment.

According to embodiments of the invention the altering the treatment comprises initiating a second treatment.

According to embodiments of the invention the altering the treatment further comprises initiating a second treatment.

According to an aspect of the present invention, there is provided a method of predicting onset of a cytokine storm in a subject having a disease which brings about a dysregulation of an immune and/or inflammatory response, the method comprising:

(a) measuring an amount of IP10 in a sample of the subject; and
(b) predicting a cytokine storm based on the level of IP10.

According to an aspect of the present invention, there is provided a method of distinguishing between a system shutdown diagnosis and a cytokine storm diagnosis in a subject with a viral disease, comprising measuring the expression level of TRAIL in a sample of the subject, wherein when the level of TRAIL is above a predetermined level, a cytokine storm diagnosis is ruled in and/or when the level of TRAIL is below a predetermined level, a system shutdown diagnosis is ruled in.

According to embodiments of the invention the method further comprises analyzing the level of at least one additional determinant set forth in Tables 1 or 2.

According to embodiments of the invention the amount of IP10 above 1000 pg/ml is indicative of onset of cytokine storm.

According to embodiments of the invention the predicting comprises an estimation of the time of onset.

According to embodiments of the invention the cytokine storm is the result of an immune-based therapy selected from the group consisting of Tumor-Infiltrating Lymphocyte (TIL) Therapy, Engineered T Cell Receptor (TCR) Therapy, Chimeric Antigen Receptor (CAR) T Cell Therapy and Natural Killer (NK) Cell Therapy.

According to embodiments of the invention the disease is selected from the group consisting of infectious disease, Ulcerative colitis, Crohn's disease, Rheumatoid arthritis, Sjogren syndrome, Kawasaki disease, Guillain-Barre syndrome, Lupus, Asthma, COPD, Multiple sclerosis, vasculitis and Psoriasis.

According to embodiments of the invention the infectious disease is a viral disease.

According to an aspect of the present invention, there is provided a method of diagnosing the severity of a viral disease of a subject, comprising measuring the expression level of a first determinant set forth in Table 1 and the level of a clinical measurement set forth in Table 2 in a sample of the subject, wherein the levels are indicative of the severity of the viral disease.

According to embodiments of the invention the virus of the viral infection is selected from the group consisting of Adenovirus, Bocavirus, Coronavirus, Enterovirus, Influenza virus, Metapneumovirus, Parainfluenza virus, Respiratory syncytial virus and Rhinovirus.

According to embodiments of the invention the viral disease is a respiratory viral disease.

According to embodiments of the invention the viral disease is transmitted via an influenza virus.

According to embodiments of the invention the viral disease is transmitted via a coronavirus.

According to embodiments of the invention the coronavirus is selected from the group consisting of severe acute respiratory syndrome coronavirus (SARS-CoV-2) and Middle East respiratory syndrome coronavirus (MERS-CoV).

According to embodiments of the invention the method further comprises measuring the amount of at least one additional determinant selected from the group consisting of IL-6, PCT and CRP.

According to an aspect of the present invention, there is provided a method of analyzing the contagiousness of a non-symptomatic subject suffering from an infection, comprising measuring the level of a determinant selected from the group consisting of TRAIL and IP10 in a body liquid sample of the subject, wherein:

> (i) when the level of TRAIL is higher than the level of TRAIL in a subject who does not have an infection, it is indicative that the non-symptomatic subject is contagious; and/or
> (ii) when the level of IP10 is higher than the level of IP-10 in a subject who does not have an infection, it is indicative that the non-symptomatic subject suffering from an infection is contagious.

According to an aspect of the present invention, there is provided a method of analyzing the degree of contagiousness of a test subject suffering from an infection comprising measuring the level of a determinant selected from the group consisting of TRAIL and IP10 in a body liquid sample of the subject, wherein the level of TRAIL and/or IP10 is proportional to the degree of contagiousness of the test subject suffering from the infection.

According to embodiments of the invention the test subject is non-symptomatic.

According to embodiments of the invention the test subject is symptomatic.

According to embodiments of the invention the non-symptomatic subject has been exposed or is suspected of being exposed to a subject known to have a viral infection.

According to embodiments of the invention the non-symptomatic subject is recovering from a viral infection.

According to embodiments of the invention the method further comprises recommending isolation of the subject upon diagnosis as being contagious.

According to embodiments of the invention the method further comprises treating the non-symptomatic subject who has been diagnosed as being contagious with a treatment selected from the group consisting of an anti-viral agent, mechanical ventilation, invasive monitoring, last-resort drug, sedation, intensive care admission, surgical intervention, hospital admittance and isolation.

According to embodiments of the invention:

> (i) when the level of TRAIL is at least 10 % higher than the level of TRAIL in a subject who does not have an infection, it is indicative that the non-symptomatic subject is contagious; and/or
> (ii) when the level of IP10 is at least 25 % higher than the level of TRAIL in a subject who does not have an infection, it is indicative that the non-symptomatic subject is contagious.

According to an aspect of the present invention, there is provided a method of analyzing biological data, the biological data containing expression values of the TRAIL protein, the IP10 protein, and the CRP protein in a body liquid sample of the subject, the method comprising:

> by a hardware processor, receiving the expression values, calculating a distance between a segment of a curved line and an axis defined by a direction, determining a score classifying a severity of a coronaviral infection of the subject based on the distance, and generating an output indicative of the score;
> wherein the distance is calculated at a point over the curved line defined by a coordinate $\delta$ along the direction, the coordinate $\delta$ being defined based on the expression values,
> wherein at least 90% of the segment is between a lower bound line $f(\delta)-\varepsilon_0$ and an upper bound line $f(\delta)+\varepsilon_1$, wherein each of the $\varepsilon_0$ and the $\varepsilon_1$ is less than 0.5, and wherein the $f(\delta)$ comprises a multiplication between a saturation function of the $\delta$ coordinate, and a saturation function of the expression value of the CRP protein.

According to embodiments of the invention the saturation function of the $\delta$ coordinate is linearly proportional to $1/(1+Exp(-\delta))$.

According to embodiments of the invention the saturation function of the expression value of the CRP protein is linearly proportional to $1/(1+(CRP/CRP_0)^{-h})$, where $CRP_0$ and $h$ are predetermined shift and width parameters.

According to embodiments of the invention the predetermined shift parameter $CRP_0$ is from about 1 mg/L to about 1000 mg/L.

According to embodiments of the invention the predetermined width parameter $h$ is from about 2 to about 100.

According to embodiments of the invention the $f(\delta)$ equals $p(1-w)+w$, the $p$ being the saturation function of the $\delta$ coordinate, and the $w$ being the saturation function of the expression value of the CRP protein.

According to embodiments of the invention the coordinate $\delta$ is defined at least based on a linear combination of the expression values.

According to embodiments of the invention the method comprises comparing each of the expression values to at least one respective threshold, wherein the coordinate $\delta$ is defined at least based on a result of the comparison.

According to embodiments of the invention the offset term of the linear combination is from about $0.9-\kappa$ to about $0.9+\kappa$, the $\kappa$ being defined as $\log(p_{max}/(1-p_{max})) - \log(p_{min}/(1-p_{min}))$, the $p_{max}$ being more than 0.9 and less than 1, and the $p_{min}$ being from about 0.01 to about 0.5.

According to embodiments of the invention the coefficient of the expression value of the TRAIL protein in the linear combination is from about $-0.04-\kappa/TRAIL_{min}$ ml/pg to about $-0.04+\kappa/TRAIL_{min}$ ml/pg, the $\kappa$ being defined as $\log(p_{max}/(1-p_{max})) - \log(p_{min}/(1-p_{min}))$, the $p_{max}$ being more than 0.9 and less than 1, the $p_{min}$ being from about 0.01 to about 0.5, and the $TRAIL_{min}$ being from about 0 to about 20 pg/ml.

According to embodiments of the invention the coefficient of the expression value of the IP-10 protein in the linear combination is from about $0.0006-\kappa/IP10_{min}$ ml/pg to about $0.0006+\kappa/IP10_{min}$ ml/pg, the $\kappa$ being defined as $\log(p_{max}/(1-p_{max})) - \log(p_{min}/(1-p_{min}))$, the $p_{max}$ being more than 0.9 and less than 1, the $p_{min}$ being from about 0.01 to about 0.5, and the $IP10_{min}$ being from about 0 to about 150 pg/ml.

According to embodiments of the invention the coefficient of the expression value of the CRP protein in the linear combination is from about $0.003-\kappa/CRP_{min}$ L/mg to about $0.003+\kappa/CRP_{min}$ L/mg, the $\kappa$ being defined as $\log(p_{max}/(1-p_{max})) - \log(p_{min}/(1-p_{min}))$, the $p_{max}$ being more than 0.9 and less than 1, the $p_{min}$ being from about 0.01 to about 0.5, and the $CRP_{min}$ being from about 0 to about 1.5 mg/L.

According to yet another aspect of the present invention there is provided a method of diagnosing a non-symptomatic subject with a viral infection, comprising measuring the level of a determinant selected from the group consisting of TRAIL and IP10 in a body liquid sample of the subject, wherein when the level of TRAIL is greater than 100 pg/ml, it is indicative that the non-symptomatic subject has a viral infection, and/or wherein when the level of IP10 is greater than 200 pg/ml, it is indicative that the non-symptomatic subject has a viral infection.

According to further embodiments, the method further comprises measuring the protein level of CRP in the body liquid of the subject, wherein when the level of CRP is greater than 5 mg/ml, it is indicative that the non-symptomatic subject has a viral infection.

According to further embodiments, the method further comprises measuring the level of IP10 and CRP in the body liquid of the subject.

According to yet another aspect of the present invention there is provided a method of diagnosing a non-symptomatic subject with a viral infection, comprising measuring the level of a determinant selected from the group consisting of TRAIL and IP10 in a body liquid sample of the subject, wherein when the level of TRAIL is at least 10 % higher than a control subject that does not have an infection, it is indicative that the non-symptomatic subject has a viral infection, and/or wherein when the level of IP10 is at least 25 % higher than a control subject that does not have an infection, it is indicative that the non-symptomatic subject has a viral infection.

According to further embodiments, when the level of TRAIL is at least 25 % higher than a control subject that does not have an infection, it is indicative that the non-symptomatic subject has a viral infection.

According to further embodiments, when the IP10 is at least 50 % higher than a control subject that does not have an infection, it is indicative that the non-symptomatic subject has a viral infection.

According to further embodiments, the non-symptomatic subject is contagious.

According to further embodiments, the method further comprises treating the non-symptomatic subject who has been diagnosed as having the viral infection with an anti-viral agent, anti-viral regimen, hospital admittance, mechanical ventilation, invasive monitoring, last-resort drug, sedation, intensive care admission, surgical intervention, hospital admittance and isolation.

According to further embodiments, the method further comprises isolating the non-symptomatic subject who has been diagnosed with the viral infection.

According to further embodiments, a virus of the viral infection is selected from the group consisting of Adenovirus, Bocavirus, Coronavirus, Enterovirus, Influenza virus, Metapneumovirus, Parainfluenza virus, Respiratory syncytial virus and Rhinovirus.

According to further embodiments, the method further comprises measuring the level of at least one determinant set forth in Tables 1, 2 or 7.

According to further embodiments, the method further comprises measuring measuring the level of at least one RNA determinant set forth in Table 12.

According to further embodiments, the method further comprises measuring further comprising measuring the level of at least one determinant selected from the group consisting of PCT, IL-6 and CRP.

According to yet another aspect of the present invention there is provided a method of diagnosing a non-symptomatic subject with a viral infection, comprising measuring the level of at least one pair of determinants set forth in Tables 8, 9 or 24 or a triplet of determinants set forth in Tables 10, 11 or 25 in a body liquid sample of the subject, wherein when the levels of determinants are statistically significantly altered compared to the levels in a subject who does not have an infection, it is indicative that the non-symptomatic subject has a viral infection.

According to yet another aspect of the present invention there is provided a method of diagnosing a subject that has been infected by a coronavirus, comprising measuring the TRAIL protein level in a body liquid sample of the subject, wherein the level is indicative whether the subject has been infected by the coronavirus.

According to yet another aspect of the present invention there is provided a method of diagnosing a subject that has been infected by a coronavirus, comprising measuring the IP10 protein level in a body liquid sample of the subject, wherein the level is indicative whether the subject has been infected by the coronavirus.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIGs. 1A-C - TRAIL (Figure 1A), IP-10 (Figure 1B) and CRP (Figure 1C) serial measurements on a non-symptomatic patient. Plot indicates the % increase from basal level. The hour 0 represents the time of symptoms onset. TRAIL, IP-10 and CRP were shown to increase prior to symptom onset.

FIGs. 2A-B. TRAIL (Figure 2A) and IP-10 (Figure 2B) levels in individuals with high and low viral loads. Both protein levels correlate with viral load status in non-symptomatic patients, a surrogate for patient infectivity status.

FIG.3 is a graph of the IP10 levels of a Covid 19 patient throughout the course of the disease.

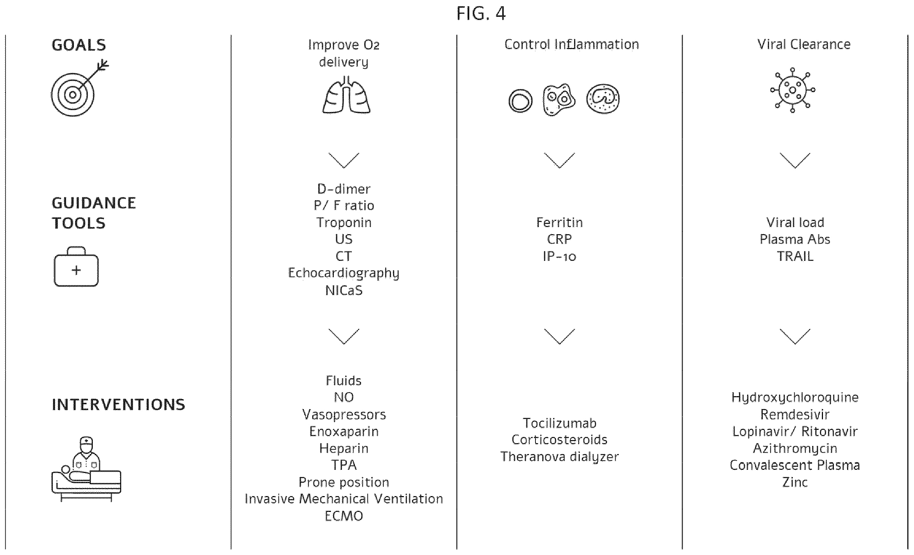

FIG. 4 is an overview of dynamic clinical decision support protocol employed for managing SARS-CoV-2 patients admitted to a COVID-19 dedicated medical center (Rabin Medical Center, HaSharon, Israel). P/F ratio, Ratio of arterial oxygen partial pressure (PaO2) to fractional inspired oxygen (FiO2); US, Ultrasound; CT, Computed tomography; NICaS, Non-Invasive Cardiac system; NO, Nitric oxide; TPA, Tissue plasminogen activator; ECMO, Extracorporeal membrane oxygenation; CRP, C-reactive protein; IP-10, interferon-γ induced protein 10 (also known as CXCL-10); Plasm Abs, Plasm antibodies; TRAIL, TNF-related apoptosis inducing ligand.

FIG. 5 is a graph illustrating that initiation of corticosteroid therapy was reflected by a decrease in IP-10 levels (ICU patients, n = 12). IP-10 was measured using MeMed Key™ at the indicated time points during the stay of the patients in the ICU. Day 0 = initiation of corticosteroid therapy. Pink area indicates IP-10 ≥ 1,000 pg/ml. Patients #2, #8 and #11 exhibited subsequent surges in IP-10 expression and are detailed in Figures 4A-C.

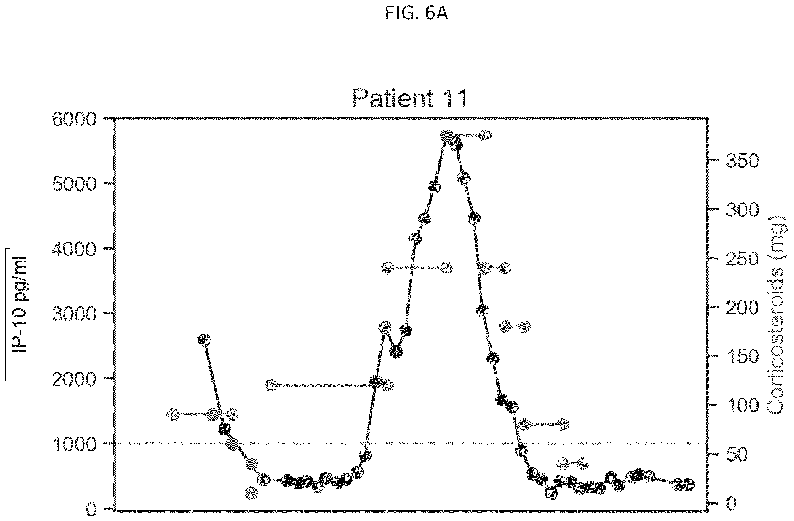

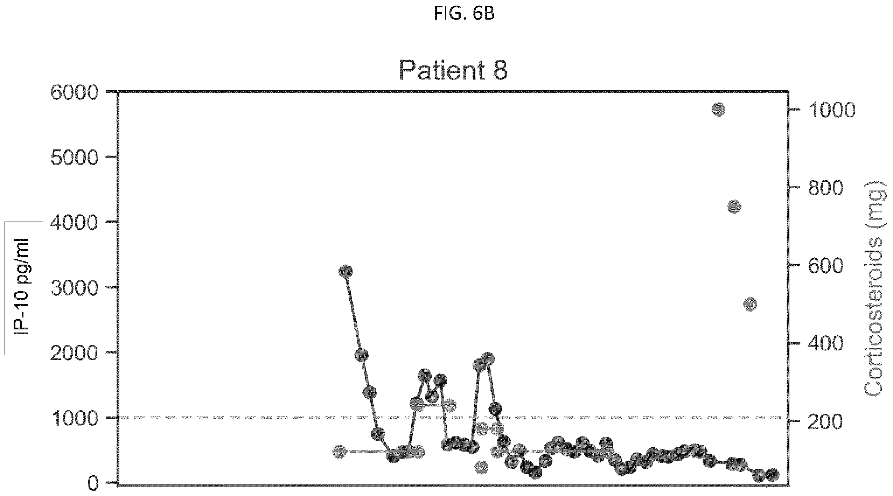

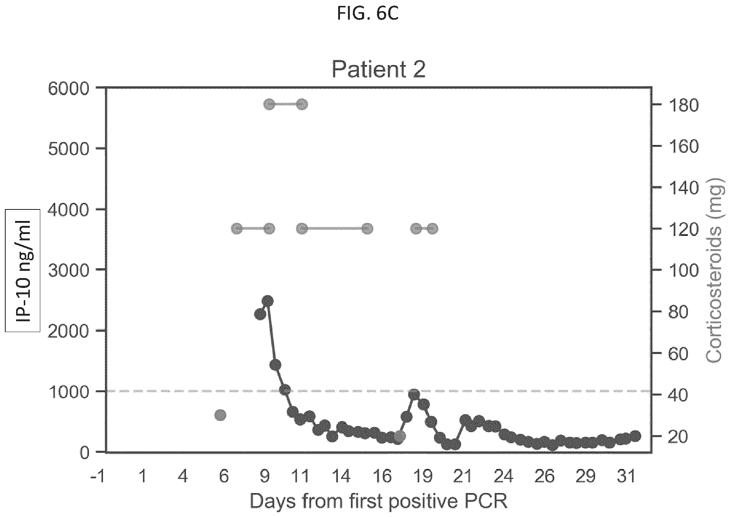

FIGs. 6A-C are graphs illustrating that IP-10 levels reflect personalized corticosteroid dosing (both increase and decrease). These 3 patients had a relapse of IP-10 levels >1,000 pg/ml, after initial treatment with corticosteroids. Right Y-axis shows the normalized levels of corticosteroids administered (hydrocortisone was converted to methylprednisolone). Left Y-axis shows the levels of IP-10 measured by MeMed Key™ at the indicated time points. X-axis shows the days from the first positive SARS-CoV-2 PCR. Lines connecting the doses indicate that the dose was given in each of the intervening day. Pink area indicates IP-10 > 1,000 pg/ml. Patient 2 survived, patients 8 and 11 died.

FIG. 7 is a Flow Diagram of Study Population for Derivation Cohort (as exemplified in Example 6).

FIGs. 8A-B are Kaplan-Meier Survival Curves of 14-day Mortality Across COVID-19 Severity algorithm Score Bins according to embodiments of the present invention compared to other markers.

FIG. 9 is a graph illustrating the AUROC Performance of COVID-19 Severity algorithm according to embodiments of the present invention compared to other markers.

FIG. 10 is a flowchart diagram of a method suitable for analyzing biological data obtained from a subject, according to various exemplary embodiments of the present invention.

FIGs. 11 is a schematic illustration describing a procedure for calculating a distance of a curved line from an axis according to some embodiments of the present invention.

FIGs. 12A-D are schematic illustrations describing a procedure for obtaining the smooth version of a segment of a curved line, according to some embodiments of the present invention.

FIG. 13 is a schematic illustration of a client-server computer configuration according to some embodiments of the present invention.

## DESCRIPTION OF SPECIFIC EMBODIMENTS OF THE INVENTION

The present invention, in some embodiments thereof, relates to methods of diagnosing and classifying the severity of viral infections and more specifically to coronaviral infections.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

In most SARS-CoV-2-positive patients, a localized, short-lasting immune response is sufficient to clear the virus from the lungs, following which the immune response recedes and the patient recovers. However, in as many as 14% of patients, a dysregulated immune response ensues, triggering a hyperinflammatory state that can lead to acute lung injury, acute respiratory distress syndrome CARDS), multiple organ failure, and mortality. The identification of markers or patterns that can be used to help stratify the risk of COVID-19 disease progression would improve the outcome of infected individuals and enable better resource allocation, thereby relieving the burden on the US healthcare system. In this way, individuals likely to deteriorate can be provided with higher level care, and those unlikely to progress to poor outcomes can have care de-escalated.

Current prognostic tools for infection severity have one or more of the following limitations: the scoring systems are complex and/or subjective and require data rich input; individual biomarkers predominantly relate to bacterial infection and were derived based on elderly cohorts; and the severity tools were not validated for early identification of the potential to deteriorate. Notably, the seemingly unusual pathophysiology of SARS-CoV-2 infection may require a dedicated tool for stratifying the likelihood of poor outcomes.

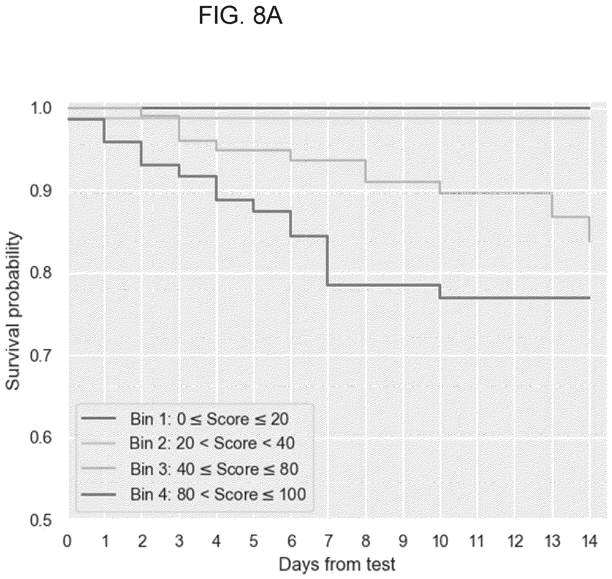

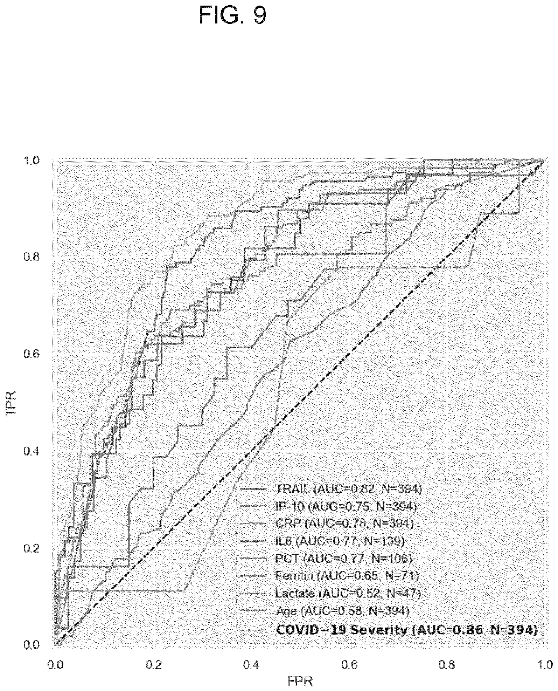

The present inventor have now shown that a multiplex host-protein signature encompassing proteins from distinct biological pathways has potential to serve as a rapid, objective and accurate triage test for early detection of severe SARS-CoV-2 infection outcomes. The blood levels of each of TRAIL, CRP and IP10 have been shown to be differentially responsive to the severity of infection, and specifically to the severity of COVID-19 infection (Figures 8A-B and Figure 9).

Thus, according to a first aspect of the present invention there is provided a method of classifying the severity of a coronaviral infection of a subject, comprising measuring the TRAIL and/or IP10 protein level in a body liquid sample of the subject, wherein the level is indicative of the severity of the coronaviral infection.

A "subject" in the context of the present invention may be a mammal (e.g. human, dog, cat, horse, cow, sheep, pig or goat). According to another embodiment, the subject is a bird (e.g. chicken, turkey, duck or goose). According to a particular embodiment, the subject is a human. The subject may be male or female. The subject may be an adult (e.g. older than 18, 21, or 22 years or a child (e.g. younger than 18, 21 or 22 years). In another embodiment, the subject is an adolescent (between 12 and 21 years), an infant (29 days to less than 2 years of age) or a neonate (birth through the first 28 days of life).

For this aspect of the invention, the subject may present with symptoms of the coronavirus infection (e.g. fever, cough, sore throat) or not present with symptoms of the coronavirus infection.

Examples of coronaviruses include: human coronavirus 229E, human coronavirus OC43, SARS-CoV, HCoV NL63, HKU1, MERS-CoV and SARS-CoV-2.

According to a particular embodiment, the coronavirus is SARS-CoV-2.

In one embodiment, the severity test is carried out on a subject who has been pre-diagnosed as having a coronaviral infection. In one embodiment, the severity test is carried out no more than 1, 2, 3, 4, 5, 6, 7, 8, 9, 10, 12, 14 days following confirmation of the coronaviral infection. In another embodiment, the severity test is carried out on a subject who does not yet show symptoms of a severe infection.

The term "determining the severity" refers to as assignment of the severity of the disease which may in one embodiment, relate to the probability to experience certain adverse events (e.g. death, hospitalization or admission to ICU) to an individual. Hereby, the individual may be accounted to a certain severity category, wherein categories comprise for instance high severity versus low severity, or severity categories based on numeral values, such as severity category 1, 2, 3, 4, 5 etc.

In one embodiment, a subject who is categorized as having a severe coronaviral infection is one predicted to die, require ICU, require ventilation and/or require Extracorporeal membrane oxygenation (ECMO) treatment within 14 days of the test. In another embodiment, the subject who is categorized as having a severe coronaviral infection is one that will require high glow nasal cannul (HFNC), bilevel positive airway pressure (BPAP) treatment, or continues positive air pressure (CPAP) support.

A subject who is categorized as having a non severe coronaviral infection is one (according to one embodiment) that is predicted not to die from the disease, not to require ICU, not to require ventilation and/or not to require Extracorporeal membrane oxygenation (ECMO) treatment within 14 days of the test. In another embodiment, the subject who is categorized as having a non-severe coronaviral infection is one that will not require high flow nasal cannul (HFNC) or bilevel positive airway pressure (BPAP) treatment, or continues positive air pressure (CPAP) support.

Additional deteminants that can be used in conjuction with TRAIL and IP10 to classify the severity of the coronaviral disease are set forth in Table 1. The level of these determinants can be measured at the protein or RNA level. Non-protein determinants that can be used in conjuction with TRAIL and IP10 to classify the severity of the coronaviral disease are set forth in Table 2.

*Table 1*

| |
|---|
| TRAIL |
| IP-10 |
| CRP |
| IL-6 |
| PCT |
| Pro-adrenomedullin |
| IL1R/IL1R1/IL1RA |
| SAA/ SAA1 |
| TREM1 |
| TREM2 |
| RSAD2 |
| NGAL |
| IL1R/IL1R1/IL1RA |
| IL-1-beta |
| SAA/ SAA1 |
| SAA/L |
| MMP8 |
| MX1 |

(continued)

| Neopterin |
| --- |
| Presepsin |
| TNF-alpha |
| IL-10 |
| IL-7 |
| MCP-1 |
| MCP-3 |
| MIP-1-alpha |
| G-CSF |

*Table 2*

| WBC |
| --- |
| ANC |
| % Lymphocytes |
| % Neutrophils |
| Neutrophil-to-Lymphocyte Ratio |
| Fibrinogen |
| Triglyceride |
| D-dimer |
| Ferritin |
| AST |
| ALT |
| PCR-Ct (viral shedding levels) |
| % Saturation |
| PaO2/FiO2 ratio |

According to a specific embodiment, the TRAIL, IP10, CRP, IL-6 and/or PCT protein level is used to stratify the subject having a coronaviral disease into one of four levels of likelihood for severe outcome - very low, low, moderate and high.

According to a specific embodiment, the TRAIL, IP10 and CRP protein level is used to stratify the subject having a coronaviral disease into one of four levels of likelihood for severe outcome - very low, low, moderate, high.

According to a specific embodiment, TRAIL, IP10 and CRP are measured to classify the severity of a SARS-CoV-2 infection.

As mentioned, when a patient has been diagnosed as having a severe infection, the management course is typically more aggressive than if he had not been assessed as having a severe infection. Thus, treatment options (for a respiratory disease) such as mechanical ventilation, life support, catheterization, hemofiltration, invasive monitoring, sedation, intensive care admission, surgical intervention, drug of last resort and hospital admittance may be selected which may otherwise not have been considered the preferred method of treatment if the patient had not been assessed as having a severe respiratory infection.

Treatment of a subject classified as having a respiratory infection with very low likelihood for severe outcome may include oxygen administration (between 1-6 L/minute). The $FiO_2$ of the inhaled gas is typically between 24-50 %. This may be carried out using a nasal cannula.

Treatment of a subject classified as having a respiratory infection with low likelihood for severe outcome may include oxygen administration (between 5-10 L/minute). The $FiO_2$ of the inhaled gas is typically between 40-60 %. This may be carried out using a nasal cannula or a simple face mask.

Treatment of a subject classified as having a respiratory infection with moderate likelihood for severe outcome may include oxygen administration (about 15 L/minute). The $FiO_2$ of the inhaled gas is typically between 60-90 %. This may be carried out using a reservoir mask.

Treatment of a subject classified as having a respiratory infection with high likelihood for severe outcome may include oxygen administration (up to 70 L/minute). The $FiO_2$ of the inhaled gas is up to 100 %. This may be carried out using nasal high flow oxygen or CPAP. Treatment may further include intubation and ECMO.

Other exemplary treatments that can be used once the classification has been made include oxygen therapy, non-invasive ventilation, mechanical ventilation, invasive monitoring, last-resort drug, sedation, intensive care admission, surgical intervention, hospital admittance, anti-viral drug, anti-viral regimen, anti-fungal drug, immune-globulin treatment, glucocorticoid therapy, extracorporeal membrane oxygenation, kidney replacement therapy and isolation.

Exemplary agents that may be used to treat coronavirus (e.g. SARS-CoV-2) infections include an antiviral agent, immunomodulatorty therapy (e.g. corticosteroid), an IV fluid, a vasopressor, an inotrope and a diuretic.

Secondary infections due to coronavirus can be treated using antibiotic or other antibacterial agents.

In one embodiment, the severity assessment is made in the emergency department of a hospital.

Emergency departments (ED) are progressively overwhelmed by patients with both urgent and non-urgent problems. This leads to overfilled ED waiting rooms with long waiting times, detrimental outcomes and unsatisfied patients. As a result, patients needing urgent care may not be treated in time, whereas patients with non-urgent problems may unnecessarily receive expensive and dispensable treatments. Time to effective treatment is among the key predictors for outcomes across different medical conditions, including patients with septicaemia, pneumonia, stroke and myocardial infarction. For these reasons, the present inventors propose use of the presently disclosed severity stratification system in the ED is essential for an optimal initial triage of medical patients.

In another embodiment, the severity assessment is made in the internal ward of a hospital.

In another embodiment, the severity assessment is made in the intensive care unit of a hospital.

The severity measurement may be used to determine a management course for the patient. The severity measurement may aid in selection of treatment priority and also site-of-care decisions (i.e. outpatient vs. inpatient management) and early identification and organization of post-acute care needs.

The present inventors have further shown that the levels of TRAIL and IP10 accurately reflect the course of an immune-related or inflammation-related disease and continual monitoring thereof aids in selection of alternative treatments throughout the disease.

According to another aspect of the present invention there is provided a method of managing treatment of a disease which brings about a dysregulation of an immune and/or inflammatory response of a subject, said subject already being medicated with a first treatment to treat said disease comprising:

(a) measuring an amount of IP10 and/or TRAIL in a sample of the subject; and
(b) altering the treatment or treatment course when the level of IP10 is above a predetermined threshold and/or when the level of TRAIL is below a predetermined threshold in said sample.

Examples of diseases which bring about a dysregulation of an immune and or inflammatory response include but are not limited to an infectious disease, ulcerative colitis, Crohn's disease, rheumatoid arthritis, Sjogren syndrome, Kawasaki disease, Guillain-Barre syndrome, lupus, asthma, COPD, Multiple sclerosis, vasculitis and psoriasis.

In one embodiment, the disease is acute respiratory distress syndrome (ARDS).

In one embodiment, the infectious disease is a viral infection.

Exemplary viruses that cause disease include those set forth in Table 3 herein below.

*Table 3*

| Family | Baltimore group | Important species | envelopment |
|---|---|---|---|
| Adenoviridae | Group I (dsDNA) | Adenovirus | non-enveloped |
| Herpesviridae | Group I (dsDNA) | Herpes simplex, type 1, Herpes simplex, type 2, Varicella-zoster virus, Epstein-Barr virus, Human cytomegalovirus, Human herpesvirus, type 8 | enveloped |
| Papillomaviridae | Group I (dsDNA) | Human papillomavirus | non-enveloped |

(continued)

| Family | Baltimore group | Important species | envelopment |
|---|---|---|---|
| Polyomaviridae | Group I (dsDNA) | BK virus, JC virus | non-enveloped |
| Poxviridae | Group I (dsDNA) | Smallpox | enveloped |
| Hepadnaviridae | Group VII (dsDNA-RT) | Hepatitis B virus | enveloped |
| Parvoviridae | Group II (ssDNA) | Parvovirus B19 | non-enveloped |
| Astroviridae | Group IV (positive-sense ssRNA) | Human astrovirus | non-enveloped |
| Caliciviridae | Group IV (positive-sense ssRNA) | Norwalk virus | non-enveloped |
| Picomaviridae | Group IV (positive-sense ssRNA) | coxsackievirus, hepatitis A virus, poliovirus, rhinovirus | non-enveloped |
| Coronaviridae | Group IV (positive-sense ssRNA) | Severe acute respiratory syndrome virus | enveloped |
| Flaviviridae | Group IV (positive-sense ssRNA) | Hepatitis C virus, yellow fever virus, dengue virus, West Nile virus, TBE virus | enveloped |
| Togaviridae | Group IV (positive-sense ssRNA) | Rubella virus | enveloped |
| Hepeviridae | Group IV (positive-sense ssRNA) | Hepatitis E virus | non-enveloped |
| Retroviridae | Group VI (ssRNA-RT) | Human immunodeficiency virus (HIV) | enveloped |
| Orthomyxoviridae | Group V (negative-sense ssRNA) | Influenza virus | enveloped |
| Arenaviridae | Group V (negative-sense ssRNA) | Lassa virus | enveloped |
| Bunyaviridae | Group V (negative-sense ssRNA) | Crimean-Congo hemorrhagic fever virus, Hantaan virus | enveloped |
| Filoviridae | Group V (negative-sense ssRNA) | Ebola virus, Marburg virus | enveloped |
| Paramyxoviridae | Group V (negative-sense ssRNA) | Measles virus, Mumps virus, Parainfluenza virus, Respiratory syncytial virus, | enveloped |
| Rhabdoviridae | Group V (negative-sense ssRNA) | Rabies virus | enveloped |
| Unassigned | Group V (negative-sense ssRNA) | Hepatitis D | enveloped |
| Reoviridae | Group III (dsRNA) | Rotavirus, Orbivirus, Coltivirus, Banna virus | non-enveloped |

According to a specific embodiment, the virus is SARS-CoV-2.

According to another specific embodiment, the virus is Human metapneumovirus, Bocavirus or Enterovirus.

Exemplary viral diseases which can be managed according to this aspect of the present invention include those set forth in Table 4, herein below.

*Table 4*

| Diseases |
|---|
| • gastroenteritis<br>• keratoconjunctivitis<br>• pharyngitis<br>• croup<br>• pharyngoconjunctival fever<br>• pneumonia<br>• cystitis |
| • Hand, foot and mouth disease<br>• pleurodynia<br>• aseptic meningitis<br>• pericarditis<br>• myocarditis |
| • infectious mononucleosis<br>• Burkitt's lymphoma<br>• Hodgkin's lymphoma<br>• nasopharyngeal carcinoma |
| • acute hepatitis |
| • chronic hepatitis<br>• hepatic cirrhosis<br>• hepatocellular carcinoma |
| • herpes labialis, cold sores - can recur by latency<br>• gingivostomatitis in children<br>• tonsillitis & pharyngitis in adults<br>• keratoconjunctivitis |
| • Aseptic meningitis |
| • infectious mononucleosis<br>• Cytomegalic inclusion disease |
| • Kaposi sarcoma<br>• multicentric Castleman disease<br>• primary effusion lymphoma |
| • AIDS |
| • influenza<br>• (Reye syndrome) |
| • measles<br>• postinfectious encephalomyelitis |
| • mumps |
| • hyperplastic epithelial lesions (common, flat, plantar and anogenital warts, laryngeal papillomas, epidermodysplasia verruciformis<br>• Malignancies for some species (cervical carcinoma, squamous cell carcinomas) |
| • croup<br>• pneumonia<br>• bronchiolitis<br>• common cold[ |
| • poliomyelitis |

(continued)

| Diseases |
| --- |
| • rabies (fatal encephalitis) |
| • congenital rubella<br>• German measles |
| • chickenpox<br>• herpes zoster<br>• Congenital varicella syndrome |

Particular examples of viruses that cause viral infections are respiratory viruses including but not limited to RSV, Flu A, Flu B, HCoV and SARS-Cov-2 and those described herein above.

In a particular embodiment, the disease which is managed according to this aspect of the present invention is COVID-19.

In another embodiment, the infectious disease which can be managed according to this aspect of the present invention is a bacterial infection.

The subjects of this aspect of the present invention are described herein above.

In one embodiment, the subject is hospitalized (e.g, in the ICU). In another embodiment, the subject is not hospitalized.

The subjects of this aspect of the present invention are already being treated for the disease.

The type of treatment which the subject is being treated is dependent on the particular disease.

In one embodiment, the subject is already being treated with an immunosuppressant.

Exemplary immunosuppressants are provided in Table 5, herein below:

### Table 5

### Immunosuppressants.

| FAMILY | DRUG | PHARMACOLOGICAL EFFECT |
| --- | --- | --- |
| Drugs that bind to immunophilins | Cyclosporine A, Tacrolimus and Sirolimus | Inhibition gene transcription of cytokines (e.g., IL-2) in T lymphocytes (blocking their proliferation), Inhibition of cytokines of T lymphocytes |
| Glucocorticoids | Prednisone and dexamethasone | Inhibition of transcription of cytokines into T lymphocytes and macrophages |
| Cytostatics | Azathioprine, Cyclophosphamide, Mophetil mycophenolate and Leflunomide | Inhibition of cell proliferation, Inhibition of proliferation of T and B lymphocytes, Inhibition of cell proliferation |
| Antilymphocyte antibodies | Polyclonal antibodies Anti-thymocytes | Triggering effector phase of specific immunity against lymphocytes |
| Monoclonal antibodies | Muromonab (OKT3) Anti-cytokines and anti-receptors | Destruction of CD3+ cells (T lymphocytes),Neutralization or destruction of molecules of the immune system |
| Hyposensitization | Allergens | Reversal of response from type IgE to IgG (from Th2 to Th1), Reduction is reactivity to allergen |

In another embodiment, the subject is already being treated with an immunostimulator, examples of which are provided in Table 6, herein below.

*Table 6*

**Immunostimulators.**

| FAMILY | DRUG | PHARMACOLOGICAL EFFECT |
|---|---|---|
| Bacterial and fungal products | Bacillus Calmette-Guérin (BCG) | Activation of macrophages (APC), NK cells, and B lymphocytes |
| | Muramyl dipeptide (MDP) L-MTP-PE | Activation of macrophages (APC and phagocytosis) Activation of macrophages (APC and phagocytosis) |
| | Lipopolysaccharides (LPS) | Activation of macrophages and B lymphocytes |
| | Propionibacterium species | APC, phagocytosis, Activation of Tc and B lymphocytes |
| | Glucan | Phagocytosis |
| Thymic factors | Thymosins | Maturation of thymocytes into T lymphocytes |
| Synthetic drugs | Levamisole Isoprinosine | Maturation and activation of T lymphocytes, phagocytosis, and chemotaxis Proliferation of T lymphocytes; activation of Th, Tc, NK, phagocytosis and chemotaxis |
| Polyclonal antibodies | Specific antibodies | Triggering effector phase of specific immunity against various antigens |
| Recombinant cytokines | Interleukin 2 (IL-2) | Activation of lymphocytes Th (proliferation), |
| | Interleukin 5 (IL-5) | Tc (lysis), and B |
| | Interleukin 12 (IL-12) | Activation of Th lymphocytes |
| | Interferon gamma (IFN-γ) | Proliferation of monocytes Activation of macrophages, lymphocytes, and NK cells, increase in expression of MHC II |
| Monoclonal antibodies | Specific antibodies | Triggering effector phase of specific immunity against antigen (e.g. tumor) |
| Vaccines | Antigens | Triggering of specific immunity (phases of recognition, activation, and effector) |

In yet another embodiment, the subject whose disease is being managed according to this aspect of the present invention is in a cytokine storm and is already being treated with a suitable agent (e.g. a steroid agent).

According to this aspect of the present invention, the present inventors contemplate measuring the level (e.g. the expression) of TRAIL and/or IP10 and on the basis of their levels altering the treatment which is already in use for treatment of the subject.

The TRAIL and IP-10 may be measured on the RNA level or the protein level.

Methods of measuring TRAIL or IP10 are described herein below.

Thus, in one embodiment, the subject is already being medicated with a first therapeutic agent and on the basis of the level of TRAIL and/or IP10, administration of a second therapeutic agent is administered. The first therapeutic agent may be stopped or the two agents may be administered together.

In another embodiment, the subject is being medicated with a first therapeutic agent and on the basis of the level of TRAIL and/or IP10, the dose or treatment regimen using the first therapeutic agent is altered.

The TRAIL and/or IP 10 level may be monitored more than one time throughout the course of the disease - e.g. once a week, once every two days, once a day, twice a day, every 12 hours, every 6 hours, every 3 hours. In another embodiment, the level of TRAIL and or IP/10 is monitored continuously (e.g. in real time).

According to another embodiment, the TRAIL and/or IP10 level is monitored at least 12 hours after the start of a new treatment, at least 6 hours, at least 3 hours or even 1 hour after the start of the new treatment.

In one embodiment, when the IP10 has increased above 1000 pg/ml, 1200 pg/ml, 1500 pg/ml, 1750 pg/ml, 2000 pg/ml, 2500 pg/ml, it is indicative that a new treatment or dosing is called for.

In another embodiment, when the TRAIL level has decreased below 25 pg/ml, it is indicative that a new treatment or dosing is called for.

In still another embodiment, the level of IP10 together with the level of TRAIL is measured at least twice a day, the IP10 level providing information on the immune status of the subject, and the TRAIL level providing information on the amount of virus present in the subject. The present inventors envisage balancing these two parameters to bring about optimal treatment for the subject.

As well as determining the level of IP10 and/or TRAIL, the present inventors further contemplate analyzing the length of time these determinants are above their relevant thresholds.

Thus, for IP10, the present inventors contemplate analyzing for how long its levels are above 1000 pg/ml or 1500 pg/ml. If the length of time is beyond a predetermined length (e.g. 1 day, two days, three days, four days etc.), it is indicative of a worst prognosis and a more extreme response.

For TRAIL, the present inventors contemplate analyzing for how long its levels are below 25 pg/ml. If the length of time is beyond a predetermined length (e.g. 1 day, two days, three days, four days etc.), it is indicative of a worst prognosis and a more extreme response.

The immune system has an exquisite mechanism capable of responding to various pathogens. Normal anti-viral immune response requires the activation of the inflammatory pathways of the immune system; however, aberrant or exaggerated response of the host's immune system can cause severe disease if remains uncontrolled. Cytokines are an essential part of the inflammatory process. During an innate immune response to a viral infection, inflammatory cytokines, together with chemokines and adhesion molecules aid to recruit leukocytes and plasma proteins to the site of infection where they perform various effector functions that serve to combat the triggering infection.

The cytokine storm results from a sudden acute increase in circulating levels of different pro-inflammatory cytokines including IL-6, IL-1, TNF-$\alpha$ and interferon. This increase in cytokines results in influx of various immune cells such as macrophages, neutrophils, and T cells from the circulation into the site of infection with destructive effects on human tissue resulting from destabilization of endothelial cell to cell interactions, damage of vascular barrier, capillary damage, diffuse alveolar damage, multiorgan failure, and ultimately death. Lung injury is one consequence of the cytokine storm that can progress into acute lung injury or its more severe form ARDS.

The present inventors propose that the level of IP 10 can be used as a gauge for predicting the onset of a cytokine storm.

According to yet another aspect of the present invention there is provided a method of predicting onset of a cytokine storm in a subject having a disease which brings about a dysregulation of an immune and/or inflammatory response, the method comprising:

(a) measuring an amount of IP 10 in a sample of the subject; and
(b) predicting a cytokine storm based on the level of IP10.

In one embodiment, the cytokine storm is the result of an immune-based therapy selected from the group consisting of Tumor-Infiltrating Lymphocyte (TIL) Therapy, Engineered T Cell Receptor (TCR) Therapy, Chimeric Antigen Receptor (CAR) T Cell Therapy and Natural Killer (NK) Cell Therapy.

Diseases which are known to bring about cytokine storms include but are not limited to infectious disease (e.g. viral infections), Ulcerative colitis, Crohn's disease, Rheumatoid arthritis, Sjogren syndrome, Kawasaki disease, Guillain-Barre syndrome, Lupus, Asthma, COPD, Multiple sclerosis, vasculitis, Psoriasis and graft-vs-host disease (GVHD).

According to a particular embodiment, the viral disease which may bring about a cytokine storm is a respiratory viral disease, such as those transmitted via an influenza virus, Ebola, smallpox, or a coronavirus (e.g. severe acute respiratory syndrome coronavirus (SARS-CoV-2) and Middle East respiratory syndrome coronavirus (MERS-CoV).

Additional proteins which may be used to predict a cytokine storm include, but are not limited to IL-6, PCT and CRP. In another embodiment, TRAIL used together with IP 10 to predict the onset of a cytokine storm.

Additional determinants which may be used to predict a cytokine storm include, but are not limited to those set forth in Tables 1 or 2.

In one embodiment, when the IP10 has increased above 1000 pg/ml, 1200 pg/ml, 1500 pg/ml, 1750 pg/ml, 2000 pg/ml, 2500 pg/ml, it is indicative that the subject is about to be in cytokine storm.

In one embodiment, a cytokine storm is defined as an increase in at least three, four, five, six, seven or all of the following cytokines: interleukin (IL)-6, IL-2, IL-7, granulocyte colony stimulating factor, interferon-$\gamma$ induced protein 10, monocyte chemoattractant protein 1, macrophage inflammatory protein 1-$\alpha$, and tumor necrosis factor-$\alpha$.

The predicting may be carried out 1 hour before the event, 2 hours before the event, 3 hours before the event, 6 hours before the event, 12 hours before the event or even 24 hours before the event.

The immune system serves in part to protect organisms from infectious pathogens. However, during infection, effector functions, such as cellular or antibody mediated cytotoxicity, and inflammatory cytokines can be harmful to the host. To limit damage to healthy tissue, mechanisms have evolved to shut down immune responses, including immune cell inactivation and cell death. This is termed a "system shutdown". In contrast, a cytokine storm is the excessive, uncontrolled release of pro-inflammatory proteins.

The present inventors further propose that TRAIL may be used as a marker for distinguishing between a system shutdown diagnosis and a cytokine storm diagnosis.

Thus, according to another aspect of the present invention there is provided a method of distinguishing between a system shutdown diagnosis and a cytokine storm diagnosis in a subject with a viral disease, comprising measuring the expression level of TRAIL in a sample of the subject, wherein when said level of TRAIL is above a predetermined level, a cytokine storm diagnosis is ruled in and/or when said level of TRAIL is below a predetermined level, a system shutdown diagnosis is ruled in.

To distinguish between system shutdown and cytokine storm, the present inventors further contemplate analyzing the level of at least one additional determinant set forth in Tables 1 or 2, herein above.

The subject for whom the classification is being carried out typically presents with symptoms of an infectious disease (e.g. fever, cough, sputum production, myalgia, fatigue, headache, anorexia, dyspnea, diarrhea, nausea, dizziness, headache, vomiting, abdominal pain, sore throat, nasal congestion, hemoptysis and chills).

Viruses and viral diseases which induce system shutdown or cytokine storms are listed in Tables 3 and 4 herein above.

In a particular embodiment, the subject is infected with a coronavirus (e.g. SARS-CoV-2).

According to yet another aspect of the present invention there is provided a method of determining the severity of a viral infection of a subject, comprising measuring the expression level of a first determinant set forth in Table 1 and the level of a clinical measurement set forth in Table 2 in a sample of the subject, wherein said levels are indicative of the severity of the viral infection.

The subject of this aspect of the present invention has typically been diagnosed with a viral infection.

In one embodiment, the subject has been diagnosed with a particular viral disease - e.g. a respiratory viral disease such as COVID-19.

Other viruses and viral diseases are disclosed herein above in Tables 3 and 4, herein above.

Methods of diagnosing viral diseases include for example clinical assessment, PCR analysis, sequencing analysis, viral culture, antibody or antigen testing, or by use of host immune response measurements.

The subject may be symptomatic for the viral disease or non-symptomatic for the viral disease.

The subject may be contagious or non-contagious.

In one embodiment, the subject is hospitalized. In another embodiment, the subject is not hospitalized.

According to some embodiments of the invention, the method further comprises informing the subj ect of results of the severity testing.

As used herein the phrase "informing the subject" refers to advising the subject that based on the diagnosis the subject should seek a suitable treatment regimen.

Once the diagnosis is determined, the results can be recorded in the subject's medical file, which may assist in selecting a treatment regimen and/or determining prognosis of the subject.

As mentioned above, once the severity of the virus has been determined, the subject may be treated in a particular way, as further detailed herein above.

Exemplary managements for viral infections include, but are not limited to oxygen therapy, non-invasive ventilation, mechanical ventilation, invasive monitoring, last-resort drug, sedation, intensive care admission, surgical intervention, hospital admittance, anti-viral drug, anti-viral regimen, anti-fungal drug, immune-globulin treatment, glucocorticoid therapy, extracorporeal membrane oxygenation, kidney replacement therapy and isolation.

Exemplary agents that may be used to treat coronavirus (e.g. SARS-CoV-2) infections include an antiviral agent, an IV fluid, a vasopressor, an inotrope and a diuretic.

The antiviral drug may be selected from the group consisting of remdesivir, an interferon, ribavirin, adefovir, tenofovir, acyclovir, brivudin, cidofovir, fomivirsen, foscarnet, ganciclovir, penciclovir, amantadine, rimantadine and zanamivir.

Also contemplated are plasma treatments from infected persons who survived and/or anti-HIV drugs such as lopinavir and ritonavir, as well as chloroquine.

Specific examples for drugs that are routinely used for the treatment of COVID-19 include, but are not limited to, Lopinavir /Ritonavir, Nucleoside analogues, Neuraminidase inhibitors, Remdesivir, polypeptide (EK1), abidol, RNA synthesis inhibitors (such as TDF, 3TC), antiinflammatory drugs (such as hormones and other molecules), Chinese traditional medicine, such ShuFengJieDu Capsules and Lianhuaqingwen Capsule, could be the drug treatment options for COVID19.

The present inventors contemplate that at least two determinants are used to determine severity of the viral disease.

According to another embodiment, at least three determinants are used to rule in severity of the disease.

Exemplary combinations for this aspect of the present invention are set forth herein below.

TRAIL and IL-6;
TRAIL and %lymph;
TRAIL, PCT and IL-6;
TRAIL, CRP and IL-6;
TRAIL, IP-10 and IL-6TRAIL, IL-6 and % Lymph;
TRAIL, IL-6, % Lymph and PCT;
TRAIL, IL-6, PCT and CRP.
IP-10 and IL-6
IP-10 and PCT

IP-10, PCT and IL-6

According to a particular embodiment, TRAIL and IP10 are measured.

According to a particular embodiment, TRAIL and IL-6 are measured.

According to a particular embodiment, TRAIL and PCT are measured.

According to still another embodiment, TRAIL and CRP are measured

According to still another embodiment, TRAIL and % Lymphocytes are measured

According to a particular embodiment, TRAIL, CRP and IP10 are measured.

According to a particular embodiment, TRAIL, IL-6 and %Lymph are measured.

According to another embodiment, TRAIL, IL-6, % Lymph and PCT are measured.

According to another embodiment, TRAIL, CRP, IP10 and IL-6 are measured.

According to another embodiment, TRAIL, CRP, IP10, IL-6 and PCT are measured.

According to a particular embodiment, TRAIL and IP10 are measured together with at least one determinant set forth in Table 1 to determine the severity of the viral infection.

According to another embodiment, CRP and IP10 are measured together with at least one determinant set forth in Table 1 to determine the severity of the viral infection.

According to another embodiment, CRP and IL6 are measured together with at least one determinant set forth in Table 1 to determine the severity of the viral infection.

According to still another embodiment, CRP and PCT are measured together with at least one determinant set forth in Table 1 to determine the severity of the viral infection.

According to still another embodiment, TRAIL and CRP are measured together with at least one determinant set forth in Table 1 to determine the severity of the viral infection.

According to still another embodiment, TRAIL and IL-6 are measured together with at least one determinant set forth in Table 1 to determine the severity of the viral infection.

According to still another embodiment, IL-6 and IP-10 are measured together with at least one determinant set forth in Table 1 to determine the severity of the viral infection.

According to still another embodiment, TRAIL and PCT are measured together with at least one determinant set forth in Table 1 to determine the severity of the viral infection.

According to still another embodiment, CRP and PCT are measured together with at least one determinant set forth in Table 1 to determine the severity of the viral infection.

According to still another embodiment, IP-10 and PCT are measured together with at least one determinant set forth in Table 1 to determine the severity of the viral infection.

According to a particular embodiment, TRAIL, CRP and IP10 are measured together with at least one determinant set forth in Table 1 to determine the severity of the viral infection.

According to a particular embodiment, TRAIL, CRP and IL-6 are measured together with at least one determinant set forth in Table 1 to determine the severity of the viral infection.

According to a particular embodiment, TRAIL, IP-10 and IL-6 are measured together with at least one determinant set forth in Table 1 to determine the severity of the viral infection.

According to another embodiment, TRAIL, CRP, IP10 and PCT are measured together with at least one determinant set forth in Table 1 to determine the severity of the viral infection.

According to another embodiment, TRAIL, CRP, IP10 and IL-6 are measured together with at least one determinant set forth in Table 1 to determine the severity of the viral infection.

According to another embodiment, TRAIL, CRP, IP10, IL-6 and PCT are measured together with at least one determinant set forth in Table 1 to determine the severity of the viral infection.

Virally infected individuals are often contagious prior to onset of clinical symptoms, and sometimes even in their absence. Currently, these contagious yet asymptomatic individuals are unidentifiable in real-time, and can only be detected if symptoms manifest, when they have already potentially infected others.

The present inventors have now shown that particular determinants (e.g. TRAIL, CRP and IP10) and combinations thereof exhibit a differential pattern between non-virally infected subjects and virally infected subjects at "time 0", wherein "time 0" is defined as the time of symptom onset. At "time 0" these determinants are already highly elevated in response to infection. Accordingly, the present inventors propose that these determinants and others are upregulated prior to day 0, and therefore can be used to distinguish between non-virally infected subjects and virally infected subjects at the non-symptomatic phase of their infection. These markers were shown to accurately distinguish between these two states for a myriad of different virus types including, but not limited to influenza, RSV and Coronavirus, across different age groups, clinical syndromes, etc.

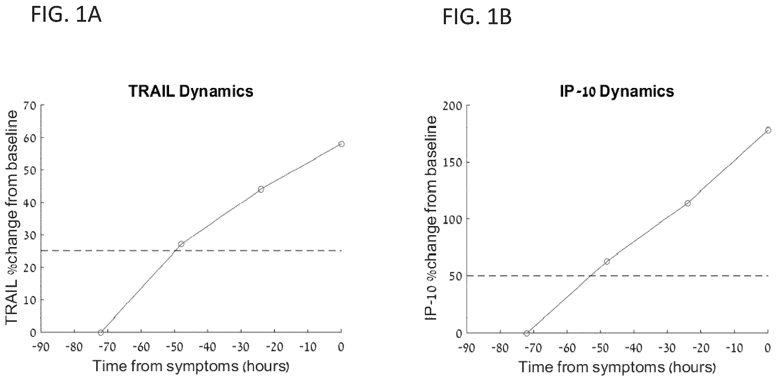

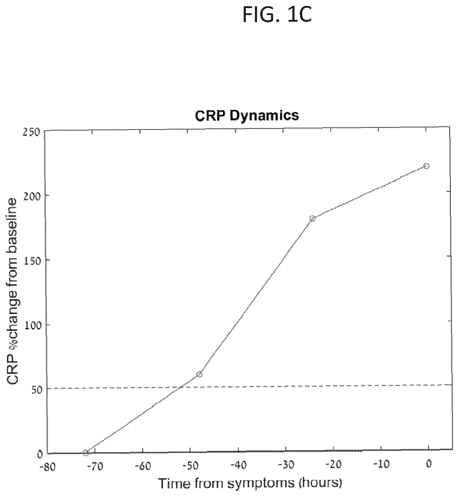

In addition, serial measurements of TRAIL and IP-10 were performed on a non-symptomatic individual who had been in close contact with a virally infected patient. On day 3 the patient developed upper respiratory tract symptoms. TRAIL and IP-10 levels were shown to increase from basal level prior to symptoms onset. More specifically, a 25% and 50% increase in TRAIL and IP-10 levels, respectively, were found to accurately detect the non-symptomatic individual prior to symptoms onset (Figures 1A-B).

The present inventors propose that knowledge of viral infection at the non-symptomatic stage (e.g. prior to fever onset) will help guide triage of infectious/infected individuals in hospital/health care settings. Furthermore, this knowledge can be used to flag non-symptomatic individuals in public settings such as border-control/airport/train station etc. In addition, the present invention can be used for providing ongoing infectivity and infectious monitoring for healthcare/public servants/other individuals with high exposure to infectious diseases, as well as to guide treatment regimens e.g. antiviral treatment (with/without relation to infective status). Lastly, the ability to diagnose viral infections at the non-symptomatic stage allows for the stratification of individuals according to likelihood of viral infection and therefore will aid in quarantine/segregation decisions.

Thus, according to still another aspect of the present invention there is provided a method of diagnosing a non-symptomatic subject with a viral infection, comprising measuring the level of a determinant selected from the group consisting of TRAIL, IP10, CRP, IL-6 and PCT in a body liquid sample of the subject, wherein the level is indicative whether the non-symptomatic subject has a viral infection.

According to a particular embodiment, TRAIL and IP10 are measured in the non-symptomatic subject.

According to another embodiment, CRP and IP10 are measured in the non-symptomatic subject.

According to another embodiment, CRP and IL6 are measured in the non-symptomatic subject.

According to still another embodiment, CRP and PCT are measured in the non-symptomatic subject.

According to still another embodiment, TRAIL and CRP are measured in the non-symptomatic subject.

According to still another embodiment, TRAIL and IL-6 are measured in the non-symptomatic subject.

According to still another embodiment, IL-6 and IP-10 are measured in the non-symptomatic subject.

According to still another embodiment, TRAIL and PCT are measured in the non-symptomatic subject.

According to still another embodiment, CRP and PCT are measured in the non-symptomatic subject.

According to still another embodiment, IP-10 and PCT are measured in the non-symptomatic subject.

According to a particular embodiment, TRAIL, CRP and IP10 are measured in the non-symptomatic subject.

According to a particular embodiment, TRAIL, CRP and IL-6 are measured in the non-symptomatic subject.

According to a particular embodiment, TRAIL, IP-10 and IL-6 are measured in the non-symptomatic subject.

According to another embodiment, TRAIL, CRP, IP10 and PCT are measured in the non-symptomatic subject.

According to another embodiment, TRAIL, CRP, IP10 and IL-6 are measured in the non-symptomatic subject.

According to another embodiment, TRAIL, CRP, IP10, IL-6 and PCT are measured in the non-symptomatic subject.

The term "non-symptomatic" according to this aspect of the present invention refers to a subject that does not have a fever, but is afflicted with the viral disease. The non-symptomatic subject may be in the early phase of the disease and may later show other clinical symptoms of the disease, such as fever. This subject may or may not be contagious. Alternatively, the non-symptomatic subject may be one that never develops other clinical symptoms of the disease, yet is contagious. This subject may be in the early, middle or late stages of the disease. Still alternatively, the subject may be in the late stages (e.g. recovery stage) of the disease, where his fever has already gone down to normal, but is still contagious.

The non-symptomatic subject may or may not show minor symptoms of a viral disease.

In one embodiment, the non-symptomatic subject, as well as not having fever, does not show respiratory symptoms - e.g. coughing, sputum production, sore throat or nasal congestion.

In another embodiment, the non-symptomatic subject, as well as not having fever, presents with at least one respiratory symptom.

According to still another embodiment, the non-symptomatic subject as well as not having fever, presents with none, one, two, three or four of the below symptoms: cough, sputum production, myalgia, fatigue, headache, anorexia, dyspnea, diarrhea, nausea, dizziness, headache, vomiting, abdominal pain, sore throat, nasal congestion, hemoptysis and chills.

The non-symptomatic subject may be contagious or non-contagious.

In another embodiment, a non-symptomatic subject may be diagnosed as having a viral infection if the level of TRAIL is at least 25 %, 30 %, 35 %, 40 %, 45 %, or 50 % higher than a control subject who does not have an infection.

According to a particular embodiment, the control level of TRAIL (based on data from non-infected subjects) is about 75 pg/ml.

According to another embodiment, the control level of TRAIL may be the level of TRAIL of that subject when he is healthy (e.g. not under any suspicion of having an infectious disease).

According to another embodiment, the control level of TRAIL may be the average level of TRAIL of a group of at least 100 healthy subjects (e.g. subj ect who are not under any suspicion of having an infectious disease, do not have an immune related disorder and/or do not have a cardiac di sorder).

A non-symptomatic subject may be diagnosed as having a viral infection if the level of IP10 is at least 50 % higher than a control subject who does not have an infection.

According to a particular embodiment, the control level of IP10 (based on data from non-infected subjects) is about 90 pg/ml.

The control level of IP10 may be the level of IP10 of that subject when he is healthy (e.g. not under any suspicion of having an infectious disease).

According to another embodiment, the control level of IP10 may be the average level of IP10 of a group of at least 100 healthy subjects (e.g. subject who are not under any suspicion of having an infectious disease, do not have an immune related disorder and/or do not have a cardiac disorder).

In one embodiment, the level of CRP in a body liquid sample of the subject is indicative whether the non-symptomatic subject has a viral infection.

For example, when the level of CRP is greater than 5 mg/ml, 6 mg/ml, 7 mg/ml, 8 mg/ml, 9 mg/ml, 10 mg/ml, 11 mg/ml, 12 pg/ml, 13 pg/ml, 14 mg/ml, 15 mg/ml, 16 mg/ml, 17 mg/ml, 18 mg/ml, 19 mg/ml, 20 mg/ml, it is indicative that the non-symptomatic subject has a viral infection.

For example, a non-symptomatic subject may be diagnosed as having a viral infection if the level of CRP is at least 50 % higher than a control subject who does not have an infection.

The control level of CRP may be the level of CRP of that subject when he is healthy (e.g. not under any suspicion of having an infectious disease).

According to another embodiment, the control level of CRP may be the average level of CRP of a group of at least 100 healthy subjects (e.g. subject who are not under any suspicion of having an infectious disease, do not have an immune related disorder and/or do not have a cardiac disorder).

According to a specific embodiment, the control level of CRP (based on data from non-infectious subjects) is about 5 mg/ml.

As used herein, the term "diagnosing" refers to determining presence or absence of the virus, monitoring virus progression, forecasting an outcome of a pathology and/or prospects of recovery and/or screening of a subject for the virus.

According to some embodiments of the invention, diagnosing of the subject for the virus is followed by substantiation of the screen results using gold standard methods.

For example, once a non-symptomatic subject has been diagnosed as having a virus, the viral infection can be substantiated using a clinical assessment, PCR analysis, sequencing analysis, viral culture, antibody or antigen testing, or by use of host immune response measurements.

According to some embodiments of the invention, the method further comprises informing the subject of the diagnosis.

Exemplary viruses that cause disease and the diseases themselves are provided in Tables 3 and 4 herein above.

According to a specific embodiment, the viral disease which is diagnosed in the non-symptomatic subject is a respiratory disease such as COVID-19.

The diagnosis of a non-symptomatic subject with a viral disease may be used to make decisions as to whether the subject should be isolated or not (i.e isolated from subjects who do not have the disease). In addition, the diagnosis can be used to determine what treatment should be used. Such a treatment may include use of an anti-viral agent or an anti-viral regime such as those described herein above. The diagnosis may further be used to determine when the subject should be retested.

Additional determinants that can be used to rule in a viral disease in a non-symptomatic subject are listed in Tables 1 and 2, herein above and Table 7, herein below.

*Table 7- singlets: data derived from the comparison of viral patients tested at time from symptoms <=1 (day 0 or day 1) to non-infectious patients. The analysis was performed on proprietary data.*

| Feature #1 | AUC | Feature #1 | AUC |
|---|---|---|---|
| GDF15 | 1 | PCSK9 | 0.686 |
| IP-10 | 0.961 | a1 Acid Glycoprotein | 0.686 |
| MBL | 0.914 | Angiopoietin2 | 0.657 |
| Neopterin | 0.914 | Dkk1 | 0.657 |
| CRP | 0.896 | SLPI | 0.657 |
| CD27 | 0.857 | Vitamin D Binding Protein | 0.657 |
| MMP2 | 0.857 | Angiogenin | 0.629 |

(continued)

| Feature #1 | AUC | Feature #1 | AUC |
|---|---|---|---|
| Resistin | 0.857 | CD95 | 0.629 |
| IL-6 | 0.84 | Cvstatin C | 0.6 |
| RSAD2 | 0.827 | Endostatin | 0.6 |
| MX1 | 0.813 | LIGHT | 0.6 |
| Tie2 | 0.8 | MIF | 0.6 |
| VCAM1 | 0.8 | RBP4 | 0.6 |
| CD14 | 0.771 | PCT | 0.589 |
| IGFBP3 | 0.743 | Complement factor D | 0.571 |
| Thrombospondin2 | 0.743 | Osteoprotegerin | 0.571 |
| PTEN | 0.735 | uPAR | 0.571 |
| ICAM1 | 0.714 | Fetuin A | 0.543 |
| APRIL | 0.686 | Leptin | 0.514 |
| Clusterin | 0.686 | IL7R | 0.489 |
| IL18 | 0.686 | CXCL13 | 0.486 |
| IL19 | 0.686 | GCP2 | 0.457 |
| IL1R | 0.686 | Adiponectin | 0.371 |
| P Selectin | 0.686 | | |

The present inventors have also uncovered particular protein pairs and triplets which should be able to detect viruses in non-symptomatic subjects.

In one embodiment, the pair of determinants which is used to rule in a viral infection in a non-symptomatic subject is set forth in Table 8 and the triplet of determinants is set forth in Table 10.

*Table 8- pairs: **data** derived from the comparison of viral patients tested at time from symptoms <=1 (day 0 or day 1) to non-infectious patients. The analysis was performed on proprietary **data.***

| Feature #1 | Feature #2 | AUC |
|---|---|---|
| IL-6 | RSAD2 | 1 |
| APRIL | IP-10 | 1 |
| Adiponectin | IP-10 | 1 |
| Angiogenin | IP-10 | 1 |
| Angiopoietin2 | IP-10 | 1 |
| CD27 | IP-10 | 1 |
| CD95 | IP-10 | 1 |
| Clusterin | GDF15 | 1 |
| Complement factor D | IP-10 | 1 |
| Cystatin C | IP-10 | 1 |
| Dkk1 | IP-10 | 1 |
| Endostatin | IP-10 | 1 |
| Fetuin A | IP-10 | 1 |
| Fetuin A | MMP2 | 1 |

(continued)

| Feature #1 | Feature #2 | AUC |
|---|---|---|
| GCP2 | IP-10 | 1 |
| GDF15 | IGFBP3 | 1 |
| GDF15 | MIF | 1 |
| GDF15 | Osteoprotegerin | 1 |
| GDF15 | TRAIL | 1 |
| GDF15 | uPAR | 1 |
| ICAM1 | IP-10 | 1 |
| IGFBP3 | IP-10 | 1 |
| IL19 | IP-10 | 1 |
| IL1R | IP-10 | 1 |
| IP-10 | LIGHT | 1 |
| IP-10 | Leptin | 1 |
| IP-10 | MIF | 1 |
| IP-10 | Neopterin | 1 |
| IP-10 | Osteoprotegerin | 1 |
| IP-10 | P Selectin | 1 |
| IP-10 | RBP4 | 1 |
| IP-10 | Resistin | 1 |
| IP-10 | Thrombospondin2 | 1 |
| IP-10 | Tie2 | 1 |
| IP-10 | VCAM1 | 1 |
| IP-10 | Vitamin D Binding Protein | 1 |
| IP-10 | uPAR | 1 |
| P Selectin | Resistin | 1 |
| Resistin | TRAIL | 1 |
| MX1 | TRAIL | 0.99 |
| IP-10 | RSAD2 | 0.988 |
| CD27 | TRAIL | 0.971 |
| CXCL13 | IP-10 | 0.971 |
| Clusterin | IP-10 | 0.971 |
| GDF15 | IL1R | 0.971 |
| GDF15 | MBL | 0.971 |
| IL18 | IP-10 | 0.971 |
| IP-10 | PCSK9 | 0.971 |
| LIGHT | Resistin | 0.971 |
| MIF | VCAM1 | 0.971 |
| Neopterin | Thrombospondin2 | 0.971 |
| TRAIL | uPAR | 0.971 |

(continued)

| Feature #1 | Feature #2 | AUC |
|---|---|---|
| CRP | IP-10 | 0.968 |
| IP-10 | PCT | 0.968 |
| IL-6 | IP-10 | 0.967 |
| CRP | RSAD2 | 0.944 |
| CD14 | IP-10 | 0.943 |
| Dkk1 | GDF15 | 0.943 |
| GDF15 | Neopterin | 0.943 |
| IGFBP3 | VCAM1 | 0.943 |
| MMP2 | Resistin | 0.943 |
| IL7R | IP-10 | 0.94 |
| CRP | IP-10 | 0.937 |
| CRP | TRAIL | 0.937 |
| IP-10 | TRAIL | 0.935 |
| CRP | TRAIL | 0.932 |
| Adiponectin | GDF15 | 0.929 |
| GDF15 | IP-10 | 0.929 |
| GDF15 | P Selectin | 0.929 |
| IP-10 | MBL | 0.929 |
| PCT | RSAD2 | 0.924 |
| CRP | IL-6 | 0.915 |
| Angiopoietin2 | GDF15 | 0.914 |
| Cystatin C | GDF15 | 0.914 |
| Fetuin A | Neopterin | 0.914 |
| GDF15 | IL18 | 0.914 |
| GDF15 | Leptin | 0.914 |
| GDF15 | Tie2 | 0.914 |
| IGFBP3 | Neopterin | 0.914 |
| IP-10 | SLPI | 0.914 |
| IP-10 | a1 Acid Glycoprotein | 0.914 |
| IP-10 | PTEN | 0.906 |
| APRIL | Resistin | 0.9 |
| CD95 | TRAIL | 0.9 |
| IP-10 | MX1 | 0.899 |
| CD95 | GDF15 | 0.886 |
| CRP | GDF15 | 0.886 |
| Fetuin A | GDF15 | 0.886 |
| GDF15 | IL19 | 0.886 |
| GDF15 | MMP2 | 0.886 |

(continued)

| Feature #1 | Feature #2 | AUC |
|---|---|---|
| GDF15 | VCAM1 | 0.886 |
| GDF15 | Vitamin D Binding Protein | 0.886 |
| IGFBP3 | MBL | 0.886 |
| IP-10 | MMP2 | 0.886 |
| MBL | TRAIL | 0.886 |
| MMP2 | TRAIL | 0.886 |
| RSAD2 | TRAIL | 0.877 |
| IL-6 | TRAIL | 0.876 |
| CRP | PCT | 0.875 |
| GDF15 | ICAM1 | 0.871 |
| ICAM1 | Thrombospondin2 | 0.871 |
| IL19 | Neopterin | 0.871 |
| MX1 | PTEN | 0.87 |
| IL-6 | MX1 | 0.869 |
| IP-10 | TRAIL | 0.865 |
| CRP | IL7R | 0.859 |
| Angiogenin | GDF15 | 0.857 |
| Clusterin | MBL | 0.857 |
| GCP2 | GDF15 | 0.857 |
| GDF15 | PCSK9 | 0.857 |
| CXCL13 | GDF15 | 0.843 |
| LIGHT | Neopterin | 0.843 |
| CD27 | GDF15 | 0.829 |
| GDF15 | RBP4 | 0.829 |
| Neopterin | TRAIL | 0.829 |
| TRAIL | Tie2 | 0.829 |
| Complement factor D | GDF15 | 0.814 |
| Neopterin | Resistin | 0.814 |
| SLPI | TRAIL | 0.814 |
| TRAIL | a1 Acid Glycoprotein | 0.814 |
| PTEN | TRAIL | 0.813 |
| IL-6 | PCT | 0.805 |
| IL7R | MX1 | 0.804 |
| CD27 | ICAM1 | 0.8 |
| LIGHT | MMP2 | 0.8 |
| CRP | MX1 | 0.788 |
| IL-6 | PTEN | 0.781 |
| IL7R | RSAD2 | 0.779 |

(continued)

| Feature #1 | Feature #2 | AUC |
|---|---|---|
| MX1 | RSAD2 | 0.779 |
| PTEN | RSAD2 | 0.775 |
| MX1 | PCT | 0.773 |
| CD14 | Clusterin | 0.771 |
| CD14 | GDF15 | 0.771 |
| GDF15 | LIGHT | 0.771 |
| GDF15 | SLPI | 0.771 |
| GDF15 | Thrombospondin2 | 0.771 |
| GDF15 | a1 Acid Glycoprotein | 0.771 |
| ICAM1 | MBL | 0.771 |
| ICAM1 | VCAM1 | 0.771 |
| Neopterin | Vitamin D Binding Protein | 0.757 |
| IL-6 | IL7R | 0.744 |
| IL7R | PCT | 0.731 |
| APRIL | GDF15 | 0.729 |
| Clusterin | VCAM1 | 0.729 |
| Endostatin | GDF15 | 0.729 |
| ICAM1 | TRAIL | 0.729 |
| IL7R | TRAIL | 0.705 |
| CD14 | TRAIL | 0.7 |
| GDF15 | Resistin | 0.7 |
| IL7R | PTEN | 0.687 |
| PCT | TRAIL | 0.678 |
| CRP | PTEN | 0.602 |
| PCT | PTEN | 0.5 |

In one embodiment, at least one of the members of the pairs is PCT, TRAIL, IL-6 or CRP. Such pairs are summarized in Table 9 herein below.

*Table 9*

| Feature #1 | Feature #2 | AUC- new | Sensitivity | Specificity | PPV | NPV |
|---|---|---|---|---|---|---|
| IL-6 | RSAD2 | 1 | 1 | 1 | 1 | 1 |
| GDF15 | TRAIL | 1 | 1 | 1 | 1 | 1 |
| Resistin | TRAIL | 1 | 1 | 1 | 1 | 1 |
| MX1 | TRAIL | 0.99 | 1 | 0.909 | 0.9 | 1 |
| CD27 | TRAIL | 0.971 | 0.857 | 1 | 1 | 0.833 |
| TRAIL | uPAR | 0.971 | 0.857 | 1 | 1 | 0.833 |
| CRP | IP-10 | 0.968 | 0.895 | 0.944 | 0.879 | 0.952 |
| IP-10 | PCT | 0.968 | 0.911 | 0.936 | 0.879 | 0.953 |

(continued)

| Feature #1 | Feature #2 | AUC- new | Sensitivity | Specificity | PPV | NPV |
|---|---|---|---|---|---|---|
| IL-6 | IP-10 | 0.967 | 0.947 | 0.928 | 0.857 | 0.975 |
| CRP | RSAD2 | 0.944 | 0.889 | 1 | 1 | 0.9 |
| CRP | IP-10 | 0.937 | 0.889 | 1 | 1 | 0.875 |
| CRP | TRAIL | 0.937 | 0.889 | 1 | 1 | 0.875 |
| IP-10 | TRAIL | 0.935 | 0.895 | 0.896 | 0.797 | 0.949 |
| CRP | TRAIL | 0.932 | 0.877 | 0.928 | 0.847 | 0.943 |
| PCT | RSAD2 | 0.924 | 0.875 | 0.889 | 0.875 | 0.889 |
| CRP | IL-6 | 0.915 | 0.825 | 0.888 | 0.77 | 0.917 |
| CD95 | TRAIL | 0.9 | 0.857 | 1 | 1 | 0.833 |
| CRP | GDF15 | 0.886 | 0.857 | 0.8 | 0.857 | 0.8 |
| MBL | TRAIL | 0.886 | 1 | 0.8 | 0.875 | 1 |
| MMP2 | TRAIL | 0.886 | 0.857 | 0.8 | 0.857 | 0.8 |
| RSAD2 | TRAIL | 0.877 | 0.778 | 1 | 1 | 0.818 |
| IL-6 | TRAIL | 0.876 | 0.789 | 0.936 | 0.849 | 0.907 |
| CRP | PCT | 0.875 | 0.786 | 0.853 | 0.733 | 0.886 |
| IL-6 | MX1 | 0.869 | 0.889 | 0.909 | 0.889 | 0.909 |
| IP-10 | TRAIL | 0.865 | 0.889 | 0.857 | 0.889 | 0.857 |
| CRP | IL7R | 0.859 | 0.889 | 0.846 | 0.8 | 0.917 |
| Neopterin | TRAIL | 0.829 | 0.714 | 1 | 1 | 0.714 |
| TRAIL | Tie2 | 0.829 | 0.857 | 0.8 | 0.857 | 0.8 |
| SLPI | TRAIL | 0.814 | 0.857 | 0.8 | 0.857 | 0.8 |
| TRAIL | a1 Acid Glycoprotein | 0.814 | 0.714 | 0.8 | 0.833 | 0.667 |
| PTEN | TRAIL | 0.813 | 0.875 | 0.75 | 0.778 | 0.857 |
| IL-6 | PCT | 0.805 | 0.75 | 0.927 | 0.84 | 0.878 |
| CRP | MX1 | 0.788 | 0.778 | 1 | 1 | 0.846 |
| IL-6 | PTEN | 0.781 | 0.875 | 0.875 | 0.875 | 0.875 |
| MX1 | PCT | 0.773 | 0.75 | 0.818 | 0.75 | 0.818 |
| IL-6 | IL7R | 0.744 | 0.778 | 0.923 | 0.875 | 0.857 |
| IL7R | PCT | 0.731 | 0.875 | 0.615 | 0.583 | 0.889 |
| ICAM1 | TRAIL | 0.729 | 0.857 | 0.6 | 0.75 | 0.75 |
| IL7R | TRAIL | 0.705 | 0.778 | 0.846 | 0.778 | 0.846 |
| CD14 | TRAIL | 0.7 | 0.857 | 0.6 | 0.75 | 0.75 |
| PCT | TRAIL | 0.678 | 0.607 | 0.78 | 0.586 | 0.794 |
| CRP | PTEN | 0.602 | 0.625 | 0.875 | 0.833 | 0.7 |
| PCT | PTEN | 0.5 | 0.625 | 0.5 | 0.556 | 0.571 |

Exemplary triples of proteins that can be used to rule in a viral infection of non-symptomatic patients are disclosed in Table 10, herein below.

EP 4 484 956 A2

*Table 10- triples: data derived from the comparison of viral patients tested at time from symptoms <=1 (day 0 or day 1) to non-infectious patients. The analysis was performed on proprietary data.*

| Feature #1 | Feature #2 | Feature #3 | AUC | Sensitivity | Specificity | PPV | NPV |
|---|---|---|---|---|---|---|---|
| CRP | IL7R | TRAIL | 1 | 1 | 1 | 1 | 1 |
| CRP | PTEN | TRAIL | 1 | 1 | 1 | 1 | 1 |
| CRP | RSAD2 | TRAIL | 1 | 1 | 1 | 1 | 1 |
| IL-6 | IL7R | RSAD2 | 1 | 1 | 1 | 1 | 1 |
| IL-6 | MX1 | RSAD2 | 1 | 1 | 1 | 1 | 1 |
| IL-6 | PTEN | RSAD2 | 1 | 1 | 1 | 1 | 1 |
| IL7R | IP-10 | PCT | 0.99 | 1 | 0.923 | 0.889 | 1 |
| IP-10 | RSAD2 | TRAIL | 0.988 | 0.889 | 1 | 1 | 0.9 |
| IL-6 | PTEN | TRAIL | 0.984 | 0.875 | 1 | 1 | 0.889 |
| CRP | IL-6 | IL7R | 0.983 | 1 | 0.923 | 0.9 | 1 |
| IL-6 | IL7R | TRAIL | 0.983 | 0.889 | 1 | 1 | 0.929 |
| CRP | IP-10 | PCT | 0.974 | 0.929 | 0.936 | 0.881 | 0.962 |
| CRP | IL-6 | IP-10 | 0.973 | 0.93 | 0.952 | 0.898 | 0.967 |
| IP-10 | PCT | PTEN | 0.969 | 0.875 | 1 | 1 | 0.889 |
| IP-10 | PTEN | TRAIL | 0.969 | 1 | 0.875 | 0.889 | 1 |
| IL-6 | IP-10 | PCT | 0.966 | 0.929 | 0.927 | 0.867 | 0.962 |
| IL-6 | IP-10 | TRAIL | 0.952 | 0.93 | 0.888 | 0.791 | 0.965 |
| IP-10 | PCT | TRAIL | 0.951 | 0.893 | 0.927 | 0.862 | 0.944 |
| MX1 | PCT | TRAIL | 0.949 | 1 | 0.909 | 0.889 | 1 |
| CRP | IP-10 | TRAIL | 0.945 | 0.877 | 0.928 | 0.847 | 0.943 |
| CRP | IL-6 | RSAD2 | 0.944 | 0.889 | 1 | 1 | 0.9 |
| CRP | MX1 | RSAD2 | 0.944 | 0.889 | 1 | 1 | 0.9 |
| CRP | MX1 | TRAIL | 0.944 | 0.889 | 1 | 1 | 0.917 |
| IL-6 | RSAD2 | TRAIL | 0.944 | 0.889 | 1 | 1 | 0.9 |
| IL7R | MX1 | TRAIL | 0.944 | 1 | 0.9 | 0.9 | 1 |
| IL7R | PCT | TRAIL | 0.942 | 0.875 | 0.923 | 0.875 | 0.923 |
| CRP | IL7R | RSAD2 | 0.938 | 0.889 | 1 | 1 | 0.889 |
| CRP | PTEN | RSAD2 | 0.938 | 0.875 | 1 | 1 | 0.889 |
| IL-6 | IP-10 | RSAD2 | 0.938 | 0.889 | 1 | 1 | 0.9 |
| IP-10 | MX1 | PCT | 0.938 | 0.875 | 0.909 | 0.875 | 0.909 |
| MX1 | RSAD2 | TRAIL | 0.938 | 1 | 0.889 | 0.9 | 1 |
| CRP | IL-6 | TRAIL | 0.936 | 0.895 | 0.888 | 0.785 | 0.949 |
| IL-6 | MX1 | TRAIL | 0.934 | 0.889 | 1 | 1 | 0.917 |
| IP-10 | MX1 | RSAD2 | 0.932 | 0.889 | 1 | 1 | 0.9 |
| IL-6 | PCT | RSAD2 | 0.931 | 1 | 0.889 | 0.889 | 1 |
| IP-10 | PCT | RSAD2 | 0.931 | 0.875 | 1 | 1 | 0.9 |
| IP-10 | PTEN | RSAD2 | 0.922 | 1 | 0.875 | 0.889 | 1 |

26

(continued)

| Feature #1 | Feature #2 | Feature #3 | AUC | Sensitivity | Specificity | PPV | NPV |
|---|---|---|---|---|---|---|---|
| CRP | IL7R | IP-10 | 0.919 | 0.889 | 0.846 | 0.8 | 0.917 |
| IL7R | RSAD2 | TRAIL | 0.917 | 0.889 | 0.875 | 0.889 | 0.875 |
| CRP | PCT | TRAIL | 0.915 | 0.839 | 0.954 | 0.904 | 0.92 |
| CRP | IL-6 | PCT | 0.903 | 0.839 | 0.835 | 0.723 | 0.91 |
| IL7R | IP-10 | MX1 | 0.889 | 1 | 0.8 | 0.818 | 1 |
| IP-10 | MX1 | TRAIL | 0.879 | 0.778 | 1 | 1 | 0.846 |
| CRP | IP-10 | RSAD2 | 0.877 | 0.778 | 1 | 1 | 0.818 |
| IL-6 | PCT | PTEN | 0.875 | 1 | 0.75 | 0.8 | 1 |
| IL7R | IP-10 | TRAIL | 0.872 | 0.778 | 1 | 1 | 0.867 |
| CRP | PCT | RSAD2 | 0.868 | 0.875 | 0.889 | 0.875 | 0.889 |
| IL7R | IP-10 | RSAD2 | 0.868 | 0.889 | 0.75 | 0.8 | 0.857 |
| IL-6 | PCT | TRAIL | 0.866 | 0.786 | 0.899 | 0.8 | 0.891 |
| IL7R | MX1 | PTEN | 0.865 | 0.87 | 0.9 | 0.952 | 0.75 |
| MX1 | PCT | RSAD2 | 0.861 | 0.875 | 0.778 | 0.778 | 0.875 |
| MX1 | PTEN | TRAIL | 0.859 | 0.875 | 0.875 | 0.875 | 0.875 |
| CRP | IL7R | MX1 | 0.856 | 0.778 | 1 | 1 | 0.833 |
| CRP | IL7R | PCT | 0.851 | 0.875 | 0.846 | 0.778 | 0.917 |
| CRP | IL-6 | PTEN | 0.844 | 0.75 | 1 | 1 | 0.8 |
| PCT | PTEN | RSAD2 | 0.844 | 0.75 | 0.875 | 0.857 | 0.778 |
| IL-6 | IP-10 | MX1 | 0.843 | 0.778 | 0.909 | 0.875 | 0.833 |
| CRP | IP-10 | PTEN | 0.828 | 0.75 | 0.875 | 0.857 | 0.778 |
| PTEN | RSAD2 | TRAIL | 0.828 | 0.75 | 0.875 | 0.857 | 0.778 |
| CRP | IP-10 | MX1 | 0.818 | 0.667 | 1 | 1 | 0.786 |
| IP-10 | MX1 | PTEN | 0.813 | 0.75 | 0.875 | 0.857 | 0.778 |
| MX1 | PTEN | RSAD2 | 0.81 | 0.957 | 0.727 | 0.88 | 0.889 |
| CRP | IL-6 | MX1 | 0.798 | 0.778 | 0.818 | 0.778 | 0.818 |
| IL-6 | IL7R | PCT | 0.798 | 0.75 | 1 | 1 | 0.867 |
| IL7R | PCT | RSAD2 | 0.797 | 0.875 | 0.75 | 0.778 | 0.857 |
| IL-6 | MX1 | PCT | 0.795 | 0.75 | 1 | 1 | 0.846 |
| CRP | MX1 | PTEN | 0.789 | 0.625 | 1 | 1 | 0.727 |
| IL7R | MX1 | PCT | 0.788 | 0.75 | 0.8 | 0.75 | 0.8 |
| IL7R | PTEN | TRAIL | 0.786 | 0.75 | 0.857 | 0.857 | 0.75 |
| PCT | RSAD2 | TRAIL | 0.785 | 0.75 | 0.778 | 0.75 | 0.778 |
| CRP | MX1 | PCT | 0.773 | 0.75 | 1 | 1 | 0.846 |
| CRP | IL7R | PTEN | 0.768 | 0.625 | 0.857 | 0.833 | 0.667 |
| IL-6 | IL7R | MX1 | 0.767 | 0.889 | 0.8 | 0.8 | 0.889 |
| IL-6 | MX1 | PTEN | 0.758 | 0.75 | 0.875 | 0.857 | 0.778 |
| IL7R | MX1 | RSAD2 | 0.75 | 0.844 | 0.692 | 0.871 | 0.643 |

(continued)

| Feature #1 | Feature #2 | Feature #3 | AUC | Sensitivity | Specificity | PPV | NPV |
|---|---|---|---|---|---|---|---|
| PCT | PTEN | TRAIL | 0.75 | 0.875 | 0.625 | 0.7 | 0.833 |
| IL7R | IP-10 | PTEN | 0.732 | 0.75 | 0.857 | 0.857 | 0.75 |
| IL-6 | IL7R | IP-10 | 0.722 | 0.778 | 0.923 | 0.875 | 0.857 |
| IL7R | PTEN | RSAD2 | 0.713 | 0.696 | 0.7 | 0.842 | 0.5 |
| MX1 | PCT | PTEN | 0.703 | 0.75 | 0.75 | 0.75 | 0.75 |
| CRP | PCT | PTEN | 0.688 | 0.625 | 0.75 | 0.714 | 0.667 |
| IL-6 | IP-10 | PTEN | 0.648 | 0.625 | 1 | 1 | 0.727 |
| IL-6 | IL7R | PTEN | 0.643 | 0.75 | 0.714 | 0.75 | 0.714 |
| IL7R | PCT | PTEN | 0.643 | 1 | 0.571 | 0.727 | 1 |

According to a particular embodiment, at least one of the triplet members is PCT, TRAIL, IL-6 or CRP.

Exemplary triplets which include at least one of the above mentioned determinants are set forth in Table 11, herein below.

**Table 11**

| Feature #1 | Feature #2 | Feature #3 | AUC | Sensitivity | Specificity | PPV | NPV |
|---|---|---|---|---|---|---|---|
| CRP | IL7R | TRAIL | 1 | 1 | 1 | 1 | 1 |
| CRP | PTEN | TRAIL | 1 | 1 | 1 | 1 | 1 |
| CRP | RSAD2 | TRAIL | 1 | 1 | 1 | 1 | 1 |
| IL-6 | IL7R | RSAD2 | 1 | 1 | 1 | 1 | 1 |
| IL-6 | MX1 | RSAD2 | 1 | 1 | 1 | 1 | 1 |
| IL-6 | PTEN | RSAD2 | 1 | 1 | 1 | 1 | 1 |
| IL7R | IP-10 | PCT | 0.99 | 1 | 0.923 | 0.889 | 1 |
| IP-10 | RSAD2 | TRAIL | 0.988 | 0.889 | 1 | 1 | 0.9 |
| IL-6 | PTEN | TRAIL | 0.984 | 0.875 | 1 | 1 | 0.889 |
| CRP | IL-6 | IL7R | 0.983 | 1 | 0.923 | 0.9 | 1 |
| IL-6 | IL7R | TRAIL | 0.983 | 0.889 | 1 | 1 | 0.929 |
| CRP | IP-10 | PCT | 0.974 | 0.929 | 0.936 | 0.881 | 0.962 |
| CRP | IL-6 | IP-10 | 0.973 | 0.93 | 0.952 | 0.898 | 0.967 |
| IP-10 | PCT | PTEN | 0.969 | 0.875 | 1 | 1 | 0.889 |
| IP-10 | PTEN | TRAIL | 0.969 | 1 | 0.875 | 0.889 | 1 |
| IL-6 | IP-10 | PCT | 0.966 | 0.929 | 0.927 | 0.867 | 0.962 |
| IL-6 | IP-10 | TRAIL | 0.952 | 0.93 | 0.888 | 0.791 | 0.965 |
| IP-10 | PCT | TRAIL | 0.951 | 0.893 | 0.927 | 0.862 | 0.944 |
| MX1 | PCT | TRAIL | 0.949 | 1 | 0.909 | 0.889 | 1 |
| CRP | IP-10 | TRAIL | 0.945 | 0.877 | 0.928 | 0.847 | 0.943 |
| CRP | IL-6 | RSAD2 | 0.944 | 0.889 | 1 | 1 | 0.9 |
| CRP | MX1 | RSAD2 | 0.944 | 0.889 | 1 | 1 | 0.9 |
| CRP | MX1 | TRAIL | 0.944 | 0.889 | 1 | 1 | 0.917 |

(continued)

| Feature #1 | Feature #2 | Feature #3 | AUC | Sensitivity | Specificity | PPV | NPV |
|------------|------------|------------|-----|-------------|-------------|-----|-----|
| IL-6 | RSAD2 | TRAIL | 0.944 | 0.889 | 1 | 1 | 0.9 |
| IL7R | MX1 | TRAIL | 0.944 | 1 | 0.9 | 0.9 | 1 |
| IL7R | PCT | TRAIL | 0.942 | 0.875 | 0.923 | 0.875 | 0.923 |
| CRP | IL7R | RSAD2 | 0.938 | 0.889 | 1 | 1 | 0.889 |
| CRP | PTEN | RSAD2 | 0.938 | 0.875 | 1 | 1 | 0.889 |
| IL-6 | IP-10 | RSAD2 | 0.938 | 0.889 | 1 | 1 | 0.9 |
| IP-10 | MX1 | PCT | 0.938 | 0.875 | 0.909 | 0.875 | 0.909 |
| MX1 | RSAD2 | TRAIL | 0.938 | 1 | 0.889 | 0.9 | 1 |
| CRP | IL-6 | TRAIL | 0.936 | 0.895 | 0.888 | 0.785 | 0.949 |
| IL-6 | MX1 | TRAIL | 0.934 | 0.889 | 1 | 1 | 0.917 |
| IL-6 | PCT | RSAD2 | 0.931 | 1 | 0.889 | 0.889 | 1 |
| IP-10 | PCT | RSAD2 | 0.931 | 0.875 | 1 | 1 | 0.9 |
| CRP | IL7R | IP-10 | 0.919 | 0.889 | 0.846 | 0.8 | 0.917 |
| IL7R | RSAD2 | TRAIL | 0.917 | 0.889 | 0.875 | 0.889 | 0.875 |
| CRP | PCT | TRAIL | 0.915 | 0.839 | 0.954 | 0.904 | 0.92 |
| CRP | IL-6 | PCT | 0.903 | 0.839 | 0.835 | 0.723 | 0.91 |
| IP-10 | MX1 | TRAIL | 0.879 | 0.778 | 1 | 1 | 0.846 |
| CRP | IP-10 | RSAD2 | 0.877 | 0.778 | 1 | 1 | 0.818 |
| IL-6 | PCT | PTEN | 0.875 | 1 | 0.75 | 0.8 | 1 |
| IL7R | IP-10 | TRAIL | 0.872 | 0.778 | 1 | 1 | 0.867 |
| CRP | PCT | RSAD2 | 0.868 | 0.875 | 0.889 | 0.875 | 0.889 |
| IL-6 | PCT | TRAIL | 0.866 | 0.786 | 0.899 | 0.8 | 0.891 |
| MX1 | PCT | RSAD2 | 0.861 | 0.875 | 0.778 | 0.778 | 0.875 |
| MX1 | PTEN | TRAIL | 0.859 | 0.875 | 0.875 | 0.875 | 0.875 |
| CRP | IL7R | MX1 | 0.856 | 0.778 | 1 | 1 | 0.833 |
| CRP | IL7R | PCT | 0.851 | 0.875 | 0.846 | 0.778 | 0.917 |
| CRP | IL-6 | PTEN | 0.844 | 0.75 | 1 | 1 | 0.8 |
| PCT | PTEN | RSAD2 | 0.844 | 0.75 | 0.875 | 0.857 | 0.778 |
| IL-6 | IP-10 | MX1 | 0.843 | 0.778 | 0.909 | 0.875 | 0.833 |
| CRP | IP-10 | PTEN | 0.828 | 0.75 | 0.875 | 0.857 | 0.778 |
| PTEN | RSAD2 | TRAIL | 0.828 | 0.75 | 0.875 | 0.857 | 0.778 |
| CRP | IP-10 | MX1 | 0.818 | 0.667 | 1 | 1 | 0.786 |
| CRP | IL-6 | MX1 | 0.798 | 0.778 | 0.818 | 0.778 | 0.818 |
| IL-6 | IL7R | PCT | 0.798 | 0.75 | 1 | 1 | 0.867 |
| IL7R | PCT | RSAD2 | 0.797 | 0.875 | 0.75 | 0.778 | 0.857 |
| IL-6 | MX1 | PCT | 0.795 | 0.75 | 1 | 1 | 0.846 |
| CRP | MX1 | PTEN | 0.789 | 0.625 | 1 | 1 | 0.727 |
| IL7R | MX1 | PCT | 0.788 | 0.75 | 0.8 | 0.75 | 0.8 |

(continued)

| Feature #1 | Feature #2 | Feature #3 | AUC | Sensitivity | Specificity | PPV | NPV |
|---|---|---|---|---|---|---|---|
| IL7R | PTEN | TRAIL | 0.786 | 0.75 | 0.857 | 0.857 | 0.75 |
| PCT | RSAD2 | TRAIL | 0.785 | 0.75 | 0.778 | 0.75 | 0.778 |
| CRP | MX1 | PCT | 0.773 | 0.75 | 1 | 1 | 0.846 |
| CRP | IL7R | PTEN | 0.768 | 0.625 | 0.857 | 0.833 | 0.667 |
| IL-6 | IL7R | MX1 | 0.767 | 0.889 | 0.8 | 0.8 | 0.889 |
| IL-6 | MX1 | PTEN | 0.758 | 0.75 | 0.875 | 0.857 | 0.778 |
| PCT | PTEN | TRAIL | 0.75 | 0.875 | 0.625 | 0.7 | 0.833 |
| IL-6 | IL7R | IP-10 | 0.722 | 0.778 | 0.923 | 0.875 | 0.857 |
| MX1 | PCT | PTEN | 0.703 | 0.75 | 0.75 | 0.75 | 0.75 |
| CRP | PCT | PTEN | 0.688 | 0.625 | 0.75 | 0.714 | 0.667 |
| IL-6 | IP-10 | PTEN | 0.648 | 0.625 | 1 | 1 | 0.727 |
| IL-6 | IL7R | PTEN | 0.643 | 0.75 | 0.714 | 0.75 | 0.714 |
| IL7R | PCT | PTEN | 0.643 | 1 | 0.571 | 0.727 | 1 |

RNA markers that can be used to rule in a viral infection of asymptomatic patients. The genes were selected based on their ability to separate day 0-1 viral patients from non-infectious patients or were detected early in a viral challenge study.

*Table* 12

| Gene | RefSeq mRNA | ROC AUC | Sensitivity | Specificity | Fold change |
|---|---|---|---|---|---|
| RSAD2 | NM_080657.5 | 1.00 | 1.00 | 1.00 | 232.8 |
| IFI27 | NM_001130080.3 | 0.99 | 0.93 | 1.00 | 196.1 |
| IFI44L | NM_001375646.1 | 1.00 | 1.00 | 1.00 | 183.8 |
| SERPING1 | NM_000062.3 | 1.00 | 1.00 | 1.00 | 94.4 |
| IFI44 | NM_006417.5 | 1.00 | 1.00 | 1.00 | 90.0 |
| IFIT1 | NM_001270927.2 | 0.98 | 1.00 | 0.92 | 87.0 |
| OAS3 | NM_006187.4 | 1.00 | 1.00 | 1.00 | 86.0 |
| HERCS | NM_016323.4 | 0.98 | 1.00 | 0.92 | 60.9 |
| USP18 | NM_017414.4 | 0.91 | 0.86 | 1.00 | 53.1 |
| CMPK2 | NM_207315.4 | 1.00 | 1.00 | 1.00 | 47.6 |
| CARD17 | NM_001007232.1 | 1.00 | 1.00 | 1.00 | 38.9 |
| EPSTI1 | NM_001002264.4 | 1.00 | 1.00 | 1.00 | 35.6 |
| MX1 | NM_001144925.2 | 0.99 | 1.00 | 0.92 | 32.7 |
| OAS2 | NM_016817.3 | 0.96 | 0.93 | 1.00 | 32.1 |
| IFI6 | NM_022872.3 | 0.99 | 0.93 | 1.00 | 29.3 |
| OASL | NM_003733.3 | 1.00 | 1.00 | 1.00 | 26.8 |
| DDX60 | NM_017631.6 | 1.00 | 1.00 | 1.00 | 25.4 |
| OAS1 | NM_016816.4 | 1.00 | 1.00 | 1.00 | 24.7 |
| IFIT3 | NM_001549.6 | 0.99 | 0.93 | 1.00 | 24.1 |

(continued)

| Gene | RefSeq mRNA | ROC AUC | Sensitivity | Specificity | Fold change |
|---|---|---|---|---|---|
| LY6E | NM_002346.3 | 0.90 | 0.86 | 1.00 | 20.3 |
| IFIH1 | NM_022168.4 | 1.00 | 1.00 | 1.00 | 16.5 |
| PLSCR1 | NM_001363872.1 | 0.98 | 1.00 | 0.92 | 16.4 |
| IFI35 | NM_005533.5 | 1.00 | 1.00 | 1.00 | 16.2 |
| LAP3 | NM_015907.3 | 1.00 | 1.00 | 1.00 | 15.6 |
| IFIT2 | NM_001547.5 | 0.96 | 0.93 | 0.92 | 15.0 |
| XAF1 | NM_017523.5 | 1.00 | 1.00 | 1.00 | 14.6 |
| ANKRD22 | NM_144590.3 | 1.00 | 1.00 | 1.00 | 12.4 |
| RTP4 | NM_022147.3 | 1.00 | 1.00 | 1.00 | 12.2 |
| IFIT5 | NM_012420.3 | 0.99 | 0.93 | 1.00 | 11.9 |
| SAMD9L | NM_152703.5 | 1.00 | 1.00 | 1.00 | 11.2 |
| PARP12 | NM_022750.4 | 1.00 | 1.00 | 1.00 | 11.1 |
| GBP1 | NM_002053.3 | 1.00 | 1.00 | 1.00 | 11.1 |
| TNFAIP6 | NM_007115.4 | 0.96 | 0.79 | 1.00 | 10.9 |
| AIM2 | NM_004833.3 | 1.00 | 1.00 | 1.00 | 10.8 |
| EIF2AK2 | NM_002759.4 | 1.00 | 1.00 | 1.00 | 10.3 |
| ZBP1 | NM_030776.3 | 1.00 | 1.00 | 1.00 | 9.6 |
| ETV7 | NM_016135.4 | 1.00 | 1.00 | 1.00 | 9.6 |
| DDX58 | NM_014314.4 | 0.99 | 0.93 | 1.00 | 8.8 |
| STAT2 | NM_005419.4 | 1.00 | 1.00 | 1.00 | 8.7 |
| GBP1P1 | AK309962.1 | 1.00 | 1.00 | 1.00 | 8.3 |
| ZCCHC2 | XM_011539452.3 | 0.99 | 1.00 | 0.92 | 8.2 |
| PARP14 | XM_011512928.3 | 1.00 | 1.00 | 1.00 | 7.3 |
| DDX60L | XM_005263341.4 | 0.96 | 0.93 | 1.00 | 7.3 |
| SAMD9 | NM_152703.5 | 1.00 | 1.00 | 1.00 | 6.8 |
| PARP9 | NM_031458.3 | 1.00 | 1.00 | 1.00 | 6.7 |
| ISG15 | NM_005101.4 | 1.00 | 1.00 | 1.00 | 6.1 |
| TNFSF10 | NM_003810.4 | 0.95 | 0.79 | 1.00 | 3.3 |
| JUP | NM_001352773.2 | 0.83 | 0.86 | 0.85 | 2.8 |
| OTOF | NM_194248.3 | 1.00 | 1.00 | 1.00 | 1.9 |
| EVI2A | NM_001003927.3 | 0.51 | 0.36 | 1.00 | 1.1 |
| LAX1 | NM_017773.4 | 0.71 | 0.57 | 0.92 | -1.8 |
| EMR3 | XM_007489046.1 | 0.93 | 0.71 | 1.00 | -5.9 |
| CASS4 | NM_001164116.2 | 0.97 | 0.93 | 1.00 | -6.0 |
| FAM101B | NM_182705.2 | 0.89 | 0.86 | 1.00 | -8.4 |
| PHOSPHO1 | NM_001143804.2 | 0.93 | 0.86 | 1.00 | -10.6 |
| PI3 | NM_002638.4 | 0.98 | 0.93 | 1.00 | -36.5 |

RNA pairs that can be used to rule in a viral infection of asymptomatic patients are summarized in Table 24 herein below. The pairs were selected based on their ability to separate day 0-1 viral patients from non-infectious patients or were detected early in a viral challenge study. RNA triplets that can be used to rule in a viral infection of asymptomatic patients are summarized in Table 25 herein below. The pairs were selected based on their ability to separate day 0-1 viral patients from non-infectious patients or were detected early in a viral challenge study.

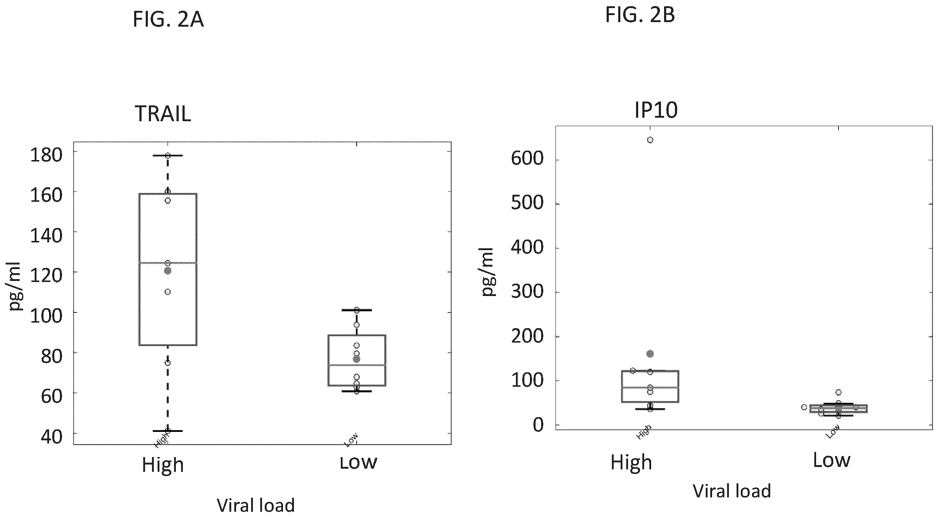

The present inventors have also identified signatures and determinants associated with viral patient's infectivity status. More specifically it was discovered that TRAIL and IP-10 have the potential to indicate patient infectivity status as can be observed by the high correlation of protein levels with viral load (the amount of virus in an organism; Figures 2A-B), a correlation of patient infectivity status.

Thus, according to still another aspect of the present invention, there is provided a method of analyzing the contagiousness of a non-symptomatic subject comprising measuring the level of a determinant selected from the group consisting of TRAIL and IP10 in a body liquid sample of the subject, wherein

(i) when said level of TRAIL is higher than the level of TRAIL in a subject who does not have an infection, it is indicative that the non-symptomatic subject is contagious; and/or
(ii) when said level of IP10 is higher than the level of IP-10 in a subject who does not have an infection, it is indicative that the non-symptomatic subject is contagious.

As used herein, the term "contagiousness" refers to the ability of a subject to infect other subjects that are in close contact. The contagiousness of a particular subject will partly depend on the infectivity of the virus itself, but also the viral load of the subject. It will be appreciated that the subject may be symptomatic or non-symptomatic and level of contagiousness does not necessarily depend on the amount or severity of symptoms a subject is suffering from.

The non-symptomatic subject may be one who is known to have been exposed or is suspected of having been exposed to a subject who has the viral infection.

According to still another aspect of the present invention there is provided a method of analyzing the degree of contagiousness of a test subject comprising measuring the level of a determinant selected from the group consisting of TRAIL and IP10 in a body liquid sample of the subject, wherein the level of TRAIL and/or IP10 is proportional to the degree of contagiousness.

For this aspect, the test subject may be symptomatic or non-symptomatic.

Once a subject has been found to be contagious over a predetermined level, the subject may be treated in a manner in which a subject found to be less contagious is treated - for example the more contagious subject may be isolated, whereas the less contagious subject may not. The more contagious subject may be treated with a particular treatment (e.g. anti-viral agent, mechanical ventilation, invasive monitoring, last-resort drug, sedation, intensive care admission, surgical intervention, hospital admittance), whereas the less contagious subject may not.

Additional protein determinants, pairs of protein determinants or triplets of protein determinants that may be used to determine whether a subject is contagious or not is set forth in any one of Tables 7-11, herein above. According to a particular embodiment, the determinant appears in Table 7. According to another embodiment, the pair of determinants used to determine the level of contagiousness appears in Table 8. According to still another embodiment, the triplet of determinants used to determine the level of contagiousness appears in Table 10.

Additional RNA determinants that may be used to determine whether a subject is contagious or not are set forth in Table 12 herein above.

According to still another aspect there is provided a method of diagnosing a subject that has been infected by a coronavirus, comprising measuring the TRAIL or IP10 protein level in a body liquid sample of the subject, wherein the level is indicative whether the subject has been infected by the coronavirus. The diagnosing may also include classifying the severity of the coronavirus infection.

For any of the aspects disclosed herein, the term "measuring" or "measurement," or alternatively "detecting" or "detection," means assessing the presence, absence, quantity or amount (which can be an effective amount) of the determinant within a clinical or subject-derived sample, including the derivation of qualitative or quantitative concentration levels of such determinants.

A "sample" in the context of the present invention is a biological sample isolated from a subject and can include, by way of example and not limitation, whole blood, serum, plasma, saliva, mucus, breath, urine, CSF, sputum, sweat, stool, hair, seminal fluid, biopsy, rhinorrhea, tissue biopsy, cytological sample, platelets, reticulocytes, leukocytes, epithelial cells, or whole blood cells.

In a particular embodiment, the sample is a blood sample - e.g. serum, plasma, or whole blood. The sample may be a venous sample, peripheral blood mononuclear cell sample or a peripheral blood sample. In one embodiment, the sample comprises white blood cells including for example granulocytes, lymphocytes and/or monocytes. In one embodiment, the sample is depleted of red blood cells.

The sample may be fresh or frozen.

According to a particular embodiment, the test subj ect of any of the aspects disclosed herein does not show signs of having had a heart attack (e.g. has a normal level of creatine kinase, troponin or serum myoglobin, and/or has a normal ECG or EKG).

According to yet another embodiment, the test subject does not have cancer.

Additional information concerning particular determinants is provided herein below.

CRP:, C-reactive protein; additional aliases of CRP include without limitation RP11-419N10.4 and PTX1.

An exemplary amino acid sequence of human CRP is set forth below in SEQ ID NO: 1.

**TRAIL:** The protein encoded by this gene is a cytokine that belongs to the tumor necrosis factor (TNF) ligand family. The present invention contemplates measuring either the soluble and/or the membrane form of this protein. In one embodiment, only the soluble form of this protein is measured. Additional names of the gene include without limitations APO2L, TNF-related apoptosis-inducing ligand, TNFSF10 and CD253. This protein binds to several members of the TNF receptor superfamily such as TNFRSF10A/TRAILR1, TNFRSF10B/TRAILR2, TNFRSF10C/TRAILR3, TNFRSF10D/TRAILR4, and possibly also to TNFRSF11B/OPG.

Exemplary amino acid sequences of TRAIL are set forth in SEQ ID NOs: 2 or 3.

**IP10**: This gene encodes a chemokine of the CXC subfamily and ligand for the receptor CXCR3. Additional names of the gene include without limitations: CXCL10, Gamma-IP10, INP10 and chemokine (C-X-C motif) ligand 10.

An exemplary amino acid sequence of human IP10 is set forth in SEQ ID NO: 4.

**IL1RA:** The protein encoded by this gene is a cytokine receptor that belongs to the interleukin 1 receptor family. Additional names of the gene include without limitations: CD121A, IL-1RT1, p80, CD121a antigen, CD121A, IL1R and IL1ra.

**PCT**: Procalcitonin (PCT) is a peptide precursor of the hormone calcitonin, the latter being involved with calcium homeostasis.

**TREM1:** Triggering receptor expressed on myeloid cells 1; additional aliases of TREM1 are CD354 and TREM-1.

**RSAD2:** Radical S-adenosyl methionine domain containing 2; additional aliases of RSAD2 include without limitation 2510004L01Rik, cig33, cig5 and vig1.

**MX1/MXA:** myxovirus (influenza virus) resistance 1; additional aliases of MX1 include without limitation IFI-78K, IFI78, MX and MxA.

**TRAILR3/ TNFRSF10C:** The protein encoded by this gene is a member of the TNF-receptor superfamily.

Exemplary amino acid sequences of this protein are set forth in SEQ ID NOs: 5 or 6.

**TRAILR4/ TNFRSF10D:** The protein encoded by this gene is a member of the TNF-receptor superfamily.

Exemplary amino acid sequences of this protein are set forth in SEQ ID NOs: 7 or 8.

**TRAIL-R1/ TNFRSF10A:** The protein encoded by this gene is a member of the TNF-receptor superfamily. Examplary amino acid sequences of this protein are set forth in SEQ ID NOs: 9, 10 or 11.

**TRAIL-R2/ TNFRSF10B:** The protein encoded by this gene is a member of the TNF-receptor superfamily, and contains an intracellular death domain. Exemplary amino acid sequences of this protein are set forth in SEQ ID NOs: 12, 13 or 14.

**MMP8:** Matrix metallproteinase 8 (MMP8) is a collagen cleaving enzyme. Exemplary amino acid sequences of MMP8 are set forth in SEQ ID NOs: 16-18.

**Neopterin:** Neopterin is the catabolic product of guanosine triphosphate, a purine nucleotide. Neopterin belongs to the chemical group known as pteridines.

**IL-6:** IL-6 is a Cytokine with a wide variety of biological functions. It is a potent inducer of the acute phase response and plays an essential role in the final differentiation of B-cells. Exemplary amino acid sequences of human IL-6 is set forth in SEQ ID NOs: 19-20.

**GDF15:** Growth Differentiation Factor 15 (GDF15) is expressed in a broad range of cell types, acts as a pleiotropic cytokine and is involved in the stress response program of cells after cellular injury. Additional aliases of GDF15: NSAID-Activated Gene 1 Protein and MIC-1.

**APRIL:** a member of the tumor necrosis factor (TNF) ligand family. This protein and its receptor are both found to be important for B cell development. Additional aliases include TNFSF13 and TALL2.

Methods of measuring the level of protein determinants are well known in the art and include, e.g., immunoassays based on antibodies to proteins, aptamers or molecular imprints.

Protein determinants can be detected in any suitable manner, but are typically detected by contacting a sample from the subject with an antibody, which binds the protein determinant and then detecting the presence or absence of a reaction product. The antibody may be monoclonal, polyclonal, chimeric, or a fragment of the foregoing, and the step of detecting the reaction product may be carried out with any suitable immunoassay.

In one embodiment, the antibody which specifically binds the determinant is attached (either directly or indirectly) to a signal producing label, including but not limited to a radioactive label, an enzymatic label, a hapten, a reporter dye or a fluorescent label.

Immunoassays carried out in accordance with some embodiments of the present invention may be homogeneous assays or heterogeneous assays. In a homogeneous assay the immunological reaction usually involves the specific antibody (e.g., anti- determinant antibody), a labeled analyte, and the sample of interest. The signal arising from the label is modified, directly or indirectly, upon the binding of the antibody to the labeled analyte. Both the immunological reaction and detection of the extent thereof can be carried out in a homogeneous solution. Immunochemical labels, which may be employed, include free radicals, radioisotopes, fluorescent dyes, enzymes, bacteriophages, or coenzymes.

In a heterogeneous assay approach, the reagents are usually the sample, the antibody, and means for producing a detectable signal. Samples as described above may be used. The antibody can be immobilized on a support, such as a bead (such as magnetic bead, protein A and protein G agarose beads), plate, pipette tip or slide, and contacted with the specimen suspected of containing the antigen in a liquid phase.

The support is then separated from the liquid phase and either the support phase or the liquid phase is examined for a detectable signal employing means for producing such signal. The signal is related to the presence of the analyte in the sample. Means for producing a detectable signal include the use of radioactive labels, fluorescent labels, or enzyme labels. For example, if the antigen to be detected contains a second binding site, an antibody which binds to that site can be conjugated to a detectable group and added to the liquid phase reaction solution before the separation step. The presence of the detectable group on the solid support indicates the presence of the antigen in the test sample. Examples of suitable immunoassays are oligonucleotides, immunoblotting, immunofluorescence methods, immunoprecipitation, chemiluminescence methods, electrochemiluminescence (ECL) or enzyme-linked immunoassays.

Those skilled in the art will be familiar with numerous specific immunoassay formats and variations thereof which may be useful for carrying out the method disclosed herein. See generally E. Maggio, Enzyme-Immunoassay, (1980) (CRC Press, Inc., Boca Raton, Fla.); see also U.S. Pat. No. 4,727,022 to Skold et al., titled "Methods for Modulating Ligand-Receptor Interactions and their Application," U.S. Pat. No. 4,659,678 to Forrest et al., titled "Immunoassay of Antigens," U.S. Pat. No. 4,376,110 to David et al., titled "Immunometric Assays Using Monoclonal Antibodies," U.S. Pat. No. 4,275,149 to Litman et al., titled "Macromolecular Environment Control in Specific Receptor Assays," U.S. Pat. No. 4,233,402 to Maggio et al., titled "Reagents and Method Employing Channeling," and U.S. Pat. No. 4,230,767 to Boguslaski et al., titled "Heterogenous Specific Binding Assay Employing a Coenzyme as Label." The determinant can also be detected with antibodies using flow cytometry. Those skilled in the art will be familiar with flow cytometric techniques which may be useful in carrying out the methods disclosed herein (Shapiro 2005). These include, without limitation, Cytokine Bead Array (Becton Dickinson) and Luminex technology.

Antibodies can be conjugated to a solid support suitable for a diagnostic assay (e.g., beads such as magnetic bead, protein A or protein G agarose, microspheres, plates, pipette tips, slides or wells formed from materials such as latex or polystyrene) in accordance with known techniques, such as passive binding. Antibodies as described herein may likewise be conjugated to detectable labels or groups such as radiolabels (e.g., $^{35}S$, $^{125}I$, $^{131}I$), enzyme labels (e.g., horseradish peroxidase, alkaline phosphatase), and fluorescent labels (e.g., fluorescein, Alexa, green fluorescent protein, rhodamine) in accordance with known techniques.

In particular embodiments, the antibodies of the present invention are monoclonal antibodies.

Suitable sources for antibodies for the detection of determinants include commercially available sources such as, for example, Abazyme, Abnova, AssayPro, Affinity Biologicals, AntibodyShop, Aviva bioscience, Biogenesis, Biosense Laboratories, Calbiochem, Cell Sciences, Chemicon International, Chemokine, Clontech, Cytolab, DAKO, Diagnostic BioSystems, eBioscience, Endocrine Technologies, Enzo Biochem, Eurogentec, Fusion Antibodies, Genesis Biotech, GloboZymes, Haematologic Technologies, Immunodetect, Immunodiagnostik, Immunometrics, Immunostar, Immunovision, Biogenex, Invitrogen, Jackson ImmunoResearch Laboratory, KMI Diagnostics, Koma Biotech, LabFrontier Life Science Institute, Lee Laboratories, Lifescreen, Maine Biotechnology Services, Mediclone, MicroPharm Ltd., ModiQuest, Molecular Innovations, Molecular Probes, Neoclone, Neuromics, New England Biolabs, Novocastra, Novus Biologicals, Oncogene Research Products, Orbigen, Oxford Biotechnology, Panvera, PerkinElmer Life Sciences, Pharmingen, Phoenix Pharmaceuticals, Pierce Chemical Company, Polymun Scientific, Polysiences, Inc., Promega Corporation, Proteogenix, Protos Immunoresearch, QED Biosciences, Inc., R&D Systems, Repligen, Research Diagnostics, Roboscreen, Santa Cruz Biotechnology, Seikagaku America, Serological Corporation, Serotec, SigmaAldrich, StemCell Technologies, Synaptic Systems GmbH, Technopharm, Terra Nova Biotechnology, TiterMax, Trillium Diagnostics, Upstate Biotechnology, US Biological, Vector Laboratories, Wako Pure Chemical Industries, and Zeptometrix. However, the skilled artisan can routinely make antibodies, against any of the polypeptide determinants described herein.

The presence of a label can be detected by inspection, or a detector which monitors a particular probe or probe combination is used to detect the detection reagent label. Typical detectors include spectrophotometers, phototubes and photodiodes, microscopes, scintillation counters, cameras, film and the like, as well as combinations thereof. Those skilled in the art will be familiar with numerous suitable detectors that widely available from a variety of commercial sources and may be useful for carrying out the method disclosed herein. Commonly, an optical image of a substrate comprising bound labeling moieties is digitized for subsequent computer analysis. See generally The Immunoassay Handbook [The Immunoassay Handbook. Third Edition. 2005].

Examples of antibodies for measuring TRAIL include without limitation: Mouse anti-Human TRAIL Monoclonal antibody (RIK-2) (12-9927-42) (Invitrogen), Goat IgG anti-Human TRAIL Polyclonal antibody (AF375) (R&D Systems), Mouse anti-Human TRAIL monoclonal antibody [2E5] (ab2219) (Abcam), Mouse anti-Human Monoclonal antibody (Clone # 75402) (MAB687) (R&D Systems),

Examples of antibodies for measuring CRP include without limitation: Rabbit anti-Human C-Reactive Protein/CRP polyclonal antibody (ab31156) (Abcam), Sheep anti-Human C-Reactive Protein/CRP Polyclonal antibody (AF1707) (R&D Systems), rabbit anti-Human C-Reactive Protein/CRP Polyclonal antibody (C3527) (Sigma-Aldrich), Mouse anti-Human C-Reactive Protein/CRP monoclonal antibody (C1688) (MilliporeSigma),

Examples of antibodies for measuring IP-10 include without limitation: Mouse anti-human CXCL10 (IP-10) Monoclonal Antibody (Cat. No. 524401) (BioLegend), Rabbit anti-human CXCL10 (IP-10) polyclonal Antibody (ab9807) (Abcam), Mouse anti-human CXCL10 (IP-10) Monoclonal Antibody (4D5) (MCA1693) (Bio-Rad), Goat anti-human CXCL10 (IP-10) Monoclonal Antibody (PA5-46999) (Invitrogen), Mouse anti-human CXCL10 (IP-10) Monoclonal Antibody (MA5-23819) (Invitrogen),

Examples of antibodies for measuring IL-6 include without limitation: Mouse anti-human IL-6 monoclonal antibody (Clone # 1936) (MAB2061) (R&D Systems), Mouse anti-human IL-6 monoclonal antibody (ab9324) (Abcam), Rat anti-human IL-6 monoclonal antibody (MQ2-39C3) (501204) (BioLegend), Rabbit anti-human IL-6 monoclonal antibody (ab233706) (Abcam),

Examples of antibodies for measuring Procalcitonin (PCT) include without limitation: Mouse Anti-Human Procalcitonin (PCT) monoclonal antibody (DMAB1342MH) (Creative diagnostics), Mouse Anti-Human Procalcitonin (PCT) monoclonal antibody (MAB8350) (R&D Systems), Sheep Anti-Human Procalcitonin (PCT) polyclonal antibody (PA1-75362) (Invitrogen), Mouse Anti-Human Procalcitonin (PCT) monoclonal antibody (6F10) (MA1-20888) (Invitrogen).

Examples of antibodies for measuring IL1R/IL1R1 include without limitation: Goat Anti-Human II,1R1 Polyclonal antibody (PA5-46930) (Invitrogen), Mouse Anti-Human IL1R1 Monoclonal antibody (clone IL1 31-22.2.1) (MA1-10857) (Invitrogen), Goat Anti-Human IL1R1 Polyclonal antibody (AF269) (R&D Systems), Rabbit Anti-Human IL1R1 Polyclonal antibody (ab106278) (Abcam), Mouse Anti-Human IL1R1 monoclonal antibody (sc-393998) (SANTA CRUZ BIOTECHNOLOGY).

Examples of antibodies for measuring Serum Amyloid A1 (SAA/ SAA1) include without limitation: Rabbit Anti-Human Serum Amyloid A1 (SAA/ SAA1) Polyclonal antibody (PA5-112852) (Invitrogen), Mouse Anti-Human Serum Amyloid A1 (SAA/ SAA1) Monoclonal antibody (MA5-11729) (Invitrogen), Mouse Anti-Human Serum Amyloid A1 (SAA/ SAA1) Monoclonal antibody (MBS592153) (MyBioSource), Mouse Anti-Human Serum Amyloid A1 (SAA/ SAA1) Monoclonal antibody (ab687) (Abcam), Mouse Anti-Human Serum Amyloid A1 (SAA/ SAA1) Monoclonal antibody (clone SAA19) (MCA6030GA) (BIO-RAD).

Examples of antibodies for measuring TREM1 include without limitation: Mouse Anti-Human TREM-1 Monoclonal Antibody (R&D Systems), Rabbit Anti-Human TREM1 Polyclonal Antibody (Merck), Rabbit Anti-Human TREM1 Polyclonal Antibody (Invitrogen), Mouse Anti-Human CD354 (TREM-1) Monoclonal Antibody (BioLegend).

Examples of antibodies for measuring TREM2 include without limitation: Rat Anti-Human TREM2 Monoclonal antibody (Clone # 237920) (MAB17291) (R&D Systems), Goat Anti-Human TREM2 Polyclonal antibody (AF1828) (R&D Systems), Mouse Anti-Human TREM2 Monoclonal antibody (clone 2B5) (NBP1-07101) (Novus Biologicals), Rabbit Anti-Human TREM2 Monoclonal antibody (ab209814) (Abeam).

Examples of antibodies for measuring IL8 include without limitation: Mouse Anti-Human IL-8 Monoclonal Antibody (ab18672) (Abcam), Mouse Anti-Human IL-8/CXCL8 Monoclonal Antibody (R&D Systems), Mouse Anti-Human IL-8 (CXCL8) Monoclonal Antibody (Invitrogen).

Examples of antibodies for measuring IL-15 include without limitation: Mouse Anti-Human IL-15 Monoclonal Antibody (MA5-23729) (Invitrogen), Mouse Anti-Human IL-15 Monoclonal Antibody (16-0157-82) (Invitrogen), Rabbit Anti-Human IL-15 Polyclonal Antibody (PA5-102871) (Invitrogen), Mouse Anti-Human IL-15 Monoclonal Antibody (ab55276) (Abcam), Mouse Anti-Human IL-15 Monoclonal Antibody (Clone # 34559) (MAB2471) (R&D Systems).

Examples of antibodies for measuring IL-12 include without limitation: Goat Anti-Human IL-12 Polyclonal Antibody (AF-219-NA) (R&D Systems), Mouse Anti-Human IL-12 Monoclonal Antibody (Clone # 24910) (MAB219) (R&D Systems), Goat Anti-Human IL-12 Polyclonal Antibody (ab9992) (Abeam), Rat Anti-Human II,-12 Monoclonal Antibody (16-8126-85) (Invitrogen).

Examples of antibodies for measuring IL-10 include without limitation: Mouse Anti-Human IL-10 Monoclonal Antibody (Clone # 127107) (MAB2172) (R&D Systems), Rat Anti-Human IL-10 Monoclonal Antibody (clone JES3-9D7) (501403) (BioLegend), Rabbit Anti-Human IL-10 Polyclonal Antibody (ab34843) (Abeam), Rat Anti-Human IL-10 Monoclonal Antibody (clone JES3-12G8) (MA1-82664) (Invitrogen).

Examples of antibodies for measuring MCP-1 include without limitation: Rabbit anti-Human MCP-1 Polyclonal antibody (ab9669) (Abeam), Mouse anti-Human MCP-1 Monoclonal antibody (Clone # 23007) (MAB679) (R&D Systems), Mouse anti-Human MCP-1 Monoclonal antibody (Clone 2D8) (MA5-17040) (Invitrogen), Mouse anti-Human MCP-1 Monoclonal antibody (Clone 5D3-F7) (MCA5981GA) (BIO-RAD).

Examples of antibodies for measuring IL-2R (IL-2RA) include without limitation: Mouse anti-Human IL-2R (IL-2RA) Monoclonal antibody (ab9496) (Abeam), Mouse anti-Human IL-2R (IL-2RA) Monoclonal antibody (Clone # 24212) (MAB1020) (R&D Systems), Mouse anti-Human IL-2R (IL-2RA) Monoclonal antibody (Clone YNRhIL2R) (ANT-104) (ProSpec).

Examples of antibodies for measuring GDF15 include without limitation: Goat anti-Human GDF15 Polyclonal antibody (AF957) (R&D Systems), Mouse anti-Human GDF15 Monoclonal antibody (Clone # 147627) (MAB957) (R&D Systems), Goat anti-Human GDF15 Polyclonal antibody (ab39999) (Abeam), Rabit anti-Human GDF15 Polyclonal antibody (ab106006) (Abeam), Rabit anti-Human GDF15 Polyclonal antibody (HPA011191) (Sigma-Aldrich).

Examples of antibodies for measuring MBL include without limitation: Goat anti-Human MBL Polyclonal antibody (AF2307) (R&D Systems), Mouse anti-Human MBL Monoclonal antibody (3B6) (ab23457) (Abeam), Goat anti-Human MBL Polyclonal antibody (AF2307) (Novus Biologicals).

Examples of antibodies for measuring CD27 include without limitation: Goat anti-Human CD27 Polyclonal antibody (AF382) (R&D Systems), Mouse anti-Human CD27 monoclonal antibody (clone O323) (47-0279-42) (Invitrogen), Rabbit anti-Human CD27 Polyclonal antibody (PA5-83443) (Invitrogen), Mouse anti-Human CD27 monoclonal antibody (clone CLB-27/1) (MHCD2704) (Invitrogen).

Examples of antibodies for measuring MMP2 include without limitation: Rabbit anti-Human MMP2 Polyclonal antibody (ab97779) (Abeam), Mouse anti-Human MMP2 monoclonal antibody (6E3F8) (ab86607) (Abeam), Mouse anti-Human MMP2 monoclonal antibody (Clone # 36006) (MAB902) (R&D Systems), Goat anti-Human MMP2 polyclonal antibody (AF902) (R&D Systems), Rabbit anti-Human MMP2 Polyclonal antibody (AHP2735) (BIO-RAD).

Examples of antibodies for measuring RESISTIN include without limitation: Goat anti-Human RESISTIN Polyclonal antibody (AF1359) (R&D Systems), Mouse anti-Human RESISTIN Monoclonal antibody (Clone # 184305) (MAB13591) (R&D Systems), Rabbit anti-Human RESISTIN Monoclonal antibody (clone EP4738) (ab124681) (Abcam), Mouse anti-Human RESISTIN Monoclonal antibody (sc-376336) (SANTA CRUZ BIOTECHNOLOGY).

Examples of antibodies for measuring RSAD2 include without limitation: Rabbit anti-Human RSAD2 Polyclonal antibody (HPA041160) (Sigma-Aldrich), Mouse anti-Human RSAD2 Monoclonal antibody (sc-390342) (SANTA CRUZ BIOTECHNOLOGY), Mouse anti-Human RSAD2 Monoclonal antibody (OTI4D12) (TA505799) (OriGene), Rabbit anti-Human RSAD2 Polyclonal antibody (LS-C378833) (LSBio), Rabbit anti-Human RSAD2 Polyclonal antibody (TA329507) (OriGene).

Examples of antibodies for measuring MX1 include without limitation: Rabbit anti-Human MX1 Polyclonal antibody (ab95926) (Abeam), Goat anti-Human MX1 Polyclonal antibody (AF7946) (R&D Systems), Mouse anti-Human MX1 monoclonal antibody (sc-271024) (SANTA CRUZ BIOTECHNOLOGY), Rabbit anti-Human MX1 Polyclonal antibody (PA5-56590) (Invitrogen), Mouse anti-Human MX1 Monoclonal antibody (OTI2G12) (MA5-24914) (Invitrogen), Mouse anti-Human MX1 Monoclonal antibody (GT4812) (MA5-31483) (Invitrogen).

Examples of antibodies for measuring TIE2 include without limitation: Mouse anti-Human TIE2 Monoclonal antibody (Cl. 16) (ab24859) (Abeam), Goat anti-Human TIE2 Polyclonal antibody (AF313) (R&D Systems), Mouse anti-Human TIE2 Monoclonal antibody (Clone # 83715) (MAB3131) (R&D Systems), Mouse anti-Human TIE2 Monoclonal antibody (clone 33.1 (Ab33)) (334205) (BioLegend), Rabbit anti-Human TIE2 Polyclonal antibody (SAB4502942) (Sigma-Aldrich).

Examples of antibodies for measuring VCAM-1/CD106 include without limitation: Mouse anti-Human VCAM-1 Monoclonal antibody (Clone # BBIG-V1) (BBA5) (R&D Systems), Rabbit anti-Human VCAM-1 Monoclonal antibody (EPR5047) (ab 134047) (Abeam), Mouse anti-Human VCAM-1 Monoclonal antibody (clone 1.4C3) (MA5-11447) (Invitrogen).

Examples of antibodies for measuring CD14 include without limitation: Mouse anti-Human CD14 Monoclonal antibody (4B4F12) (ab182032) (Abcam), Mouse anti-Human CD14 Monoclonal antibody (M5E2) (301805) (BioLegend), Mouse anti-Human CD14 Monoclonal antibody (Clone # 134620) (MAB3832) (R&D Systems), Mouse anti-Human CD14 Monoclonal antibody (clone TÜK4) (MCA1568) (Bio-Rad).

Examples of antibodies for measuring IGFBP-3 include without limitation: Mouse anti-Human IGFBP-3 Monoclonal antibody (Clone # 84728) (MAB305) (R&D Systems), Goat anti-Human IGFBP-3 Polyclonal antibody (AF675) (R&D Systems), Goat anti-Human IGFBP-3 Polyclonal antibody (ab77635) (Abeam), Rabbit anti-Human IGFBP-3 Polyclonal antibody (PA5-29711) (Invitrogen).

Examples of antibodies for measuring APRIL include without limitation: Mouse anti-Human APRIL/ TNFSF13 Monoclonal antibody (JE49-07) (MA5-34866) (Invitrogen), Mouse anti-Human APRIL/ TNFSF13 Monoclonal antibody (Clone # 670820) (MAB5860) (R&D Systems), Mouse anti-Human APRIL/ TNFSF13 Monoclonal antibody (Clone # 670840) (MAB8843) (R&D Systems), Rabbit anti-Human APRIL/ TNFSF13 Polyclonal antibody (ab3681) (Abeam).

Examples of antibodies for measuring Adiponectin include without limitation: Mouse anti-Human Adiponectin Monoclonal antibody (19F1) (ab22554) (Abcam), Rabbit anti-Human Adiponectin Polyclonal antibody (ab25891) (Abeam), Mouse anti-Human Adiponectin Monoclonal antibody (Clone # 553517) (MAB10652) (R&D Systems), Goat anti-Human Adiponectin Polyclonal antibody (AF1065) (R&D Systems), Rabbit anti-Human Adiponectin Polyclonal antibody (A6354) (Sigma-Aldrich).

Examples of antibodies for measuring Angiogenin include without limitation: Goat anti-Human Angiogenin Polyclonal antibody (AF265) (R&D Systems), Goat anti-Human Angiogenin Polyclonal antibody (AB-265) (R&D Systems), Rabbit anti-Human Angiogenin Polyclonal antibody (ab189207) (Abcam), Mouse anti-Human Angiogenin Monoclonal antibody (clone MANG-1) (0555-5008) (Bio-Rad).

Examples of antibodies for measuring Angiopoietin 2/ANG2 include without limitation: Rabbit anti-Human Angiopoietin 2/ANG2 Polyclonal antibody (ab153934) (Abeam), Goat anti-Human Angiopoietin 2/ANG2 Polyclonal antibody (AF623) (R&D Systems), Mouse anti-Human Angiopoietin 2/ANG2 Monoclonal antibody (Clone # 180102) (MAB0983) (R&D Systems), Mouse anti-Human Angiopoietin 2/ANG2 Monoclonal antibody (Clone # M5203F01) (682702) (BioLegend).

Examples of antibodies for measuring CLUSTERIN include without limitation: Rabbit anti-Human CLUSTERIN Polyclonal antibody (ab69644) (Abcam), Rabbit anti-Human CLUSTERIN Monoclonal antibody [EPR2911] (ab92548) (Abeam), Mouse anti-Human CLUSTERIN Monoclonal antibody (Clone # 350227) (MAB2937) (R&D Systems), Mouse anti-Human CLUSTERIN Monoclonal antibody (Clone # 350270) (MAB29372) (R&D Systems).

Examples of antibodies for measuring CD95 include without limitation: Mouse anti-Human CD95 Monoclonal antibody (clone DX2) (BioLegend), Mouse anti-Human CD95 Monoclonal antibody (clone EOS9.1) (BioLegend), Mouse anti-Human CD95 Monoclonal antibody (clone LOB 3/17) (Bio-Rad).

Examples of antibodies for measuring uPAR include without limitation: Mouse anti-Human uPAR Monoclonal antibody (Clone # 62022) (MAB807) (R&D Systems), Goat anti-Human uPAR Polyclonal antibody (AF807) (R&D Systems), Rabbit anti-Human uPAR Monoclonal antibody (clone 2G10) (MABC88) (Sigma-Aldrich).

Examples of antibodies for measuring IL7R include without limitation: Mouse anti-Human IL7R /CD127 Monoclonal antibody (Clone # 40131) (MAB306) (R&D Systems), Mouse anti-Human IL7R/CD127 Monoclonal antibody (Clone A019D5) (351303) (BioLegend), Mouse anti-Human IL7R/CD127 Monoclonal antibody (eBioRDR5) (48-1278-42) (Invitrogen), Rabbit anti-Human IL7R/CD127 Polyclonal antibody (PA5-97870) (Invitrogen).

Examples of antibodies for measuring PTEN include without limitation: Mouse anti-Human PTEN Monoclonal antibody (Clone # 217702) (MAB847) (R&D Systems), Rabbit anti-Human PTEN Polyclonal antibody (ab31392) (Abeam), Mouse anti-Human PTEN Monoclonal antibody (A2b1) (ab79156) (Abeam), Mouse anti-Human PTEN Monoclonal antibody (clone 6H2.1) (Sigma-Aldrich).

Examples of antibodies for measuring MMP8 include without limitation: Mouse anti-Human MMP8 Monoclonal antibody (Clone # 100608) (MAB9081) (R&D Systems), Mouse anti-Human MMP8 Monoclonal antibody (Clone # 100619) (MAB908) (R&D Systems), Rabbit anti-Human MMP8 Monoclonal antibody (EP1252Y) (ab81286) (Abeam), Rabbit anti-Human MMP8 Polyclonal antibody (PA5-28246) (Invitrogen), Rabbit anti-Human MMP8 Polyclonal antibody (PA5-82805) (Invitrogen), Rabbit anti-Human MMP8 Polyclonal antibody (HPA021221) (Sigma-Aldrich).

Examples of antibodies for measuring Ferritin include without limitation: Mouse anti-Human Ferritin Monoclonal antibody (Clone # 962609) (MAB93541) (R&D Systems), Sheep anti-Human Ferritin Polyclonal antibody (AHP2179G) (Bio-Rad), Mouse anti-Human Ferritin Monoclonal antibody (clone F23 (7A4)) (4420-3010) (Bio-Rad), Goat anti-Human Ferritin Polyclonal antibody (PA5-19058) (Invitrogen), Mouse anti-Human Ferritin Monoclonal antibody (clone 101) (MIF2501) (Invitrogen), Rabbit anti-Human Ferritin Monoclonal antibody (EPR3004Y) (ab75973) (Abeam).

Examples of antibodies for measuring D-Dimer include without limitation: Mouse anti-Human D-Dimer Monoclonal antibody (clone DD1) (MCA2523) (Bio-Rad), Mouse anti-Human D-Dimer Monoclonal antibody (3B6) (ab273889) (Abeam), Rabbit anti-Human D-Dimer Monoclonal antibody (Clone # 2609D) (MAB104712) (R&D Systems), Mouse anti-Human D-Dimer Monoclonal antibody (clone DD2) (NB110-8376) (Novus).

Soluble TRAIL and membrane TRAIL can be distinguished by using different measuring techniques and samples. For example, Soluble TRAIL can be measured without limitation in cell free samples such as serum or plasma, using without limitation lateral flow immunoassay (LFIA), as further described herein below. Membrane TRAIL can be measured in samples that contain cells using cell based assays including without limitation flow cytometry, ELISA, and other immunoassays.

*Lateral Flow Immunoassays (LFIA):* This is a technology which allows rapid measurement of analytes at the point of care (POC) and its underlying principles are described below. According to one embodiment, LFIA is used in the context of a hand-held device.

The technology is based on a series of capillary beds, such as pieces of porous paper or sintered polymer. Each of these elements has the capacity to transport fluid (e.g., urine) spontaneously. The first element (the sample pad) acts as a sponge and holds an excess of sample fluid. Once soaked, the fluid migrates to the second element (conjugate pad) in which the manufacturer has stored the so-called conjugate, a dried format of bio-active particles (see below) in a salt-sugar matrix that contains everything to guarantee an optimized chemical reaction between the target molecule (e.g., an antigen) and its chemical partner (e.g., antibody) that has been immobilized on the particle's surface. While the sample fluid dissolves the salt-sugar matrix, it also dissolves the particles and in one combined transport action the sample and conjugate mix while flowing through the porous structure. In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex.

After a while, when more and more fluid has passed the stripes, particles accumulate and the stripe-area changes color. Typically there are at least two stripes: one (the control) that captures any particle and thereby shows that reaction conditions and technology worked fine, the second contains a specific capture molecule and only captures those particles onto which an analyte molecule has been immobilized. After passing these reaction zones the fluid enters the final porous material, the wick, that simply acts as a waste container. Lateral Flow Tests can operate as either competitive or sandwich assays.

Different formats may be adopted in LFIA. Strips used for LFIA contain four main components. A brief description of each is given before describing format types.

*Sample application pad:* It is made of cellulose and/or glass fiber and sample is applied on this pad to start assay. Its function is to transport the sample to other components of lateral flow test strip (LFTS). Sample pad should be capable of transportation of the sample in a smooth, continuous and homogenous manner. Sample application pads are sometimes designed to pretreat the sample before its transportation. This pretreatment may include separation of sample components, removal of interferences, adjustment of pH, etc.

*Conjugate pad:* It is the place where labeled biorecognition molecules are dispensed. Material of conjugate pad should immediately release labeled conjugate upon contact with moving liquid sample. Labeled conjugate should stay stable over entire life span of lateral flow strip. Any variations in dispensing, drying or release of conjugate can change results of assay significantly. Poor preparation of labeled conjugate can adversely affect sensitivity of assay. Glass fiber, cellulose, polyesters and some other materials are used to make conjugate pad for LFIA. Nature of conjugate pad material has an effect on release of labeled conjugate and sensitivity of assay.

*Nitrocellulose membrane:* It is highly critical in determining sensitivity of LFIA. Nitrocellulose membranes are available in different grades. Test and control lines are drawn over this piece of membrane. So an ideal membrane should provide support and good binding to capture probes (antibodies, aptamers etc.). Nonspecific adsorption over test and control lines may affect results of assay significantly, thus a good membrane will be characterized by lesser non-specific adsorption in the regions of test and control lines. Wicking rate of nitrocellulose membrane can influence assay sensitivity. These membranes are easy to use, inexpensive, and offer high affinity for proteins and other biomolecules. Proper dispensing of bioreagents, drying and blocking play a role in improving sensitivity of assay.

*Adsorbent pad:* It works as sink at the end of the strip. It also helps in maintaining flow rate of the liquid over the membrane and stops back flow of the sample. Adsorbent capacity to hold liquid can play an important role in results of assay.

All these components are fixed or mounted over a backing card. Materials for backing card are highly flexible because they have nothing to do with LFIA except providing a platform for proper assembling of all the components. Thus backing card serves as a support and it makes easy to handle the strip.

Major steps in LFIA are (i) preparation of antibody against target analyte (ii) preparation of label (iii) labeling of biorecognition molecules (iv) assembling of all components onto a backing card after dispensing of reagents at their proper pads (v) application of sample and obtaining results.

*Sandwich format:* In a typical format, label (Enzymes or nanoparticles or fluorescence dyes) coated antibody or aptamer is immobilized at conjugate pad. This is a temporary adsorption which can be flushed away by flow of any buffer solution. A primary antibody or aptamer against target analyte is immobilized over test line. A secondary antibody or probe against labeled conjugate antibody/aptamer is immobilized at control zone.

Sample containing the analyte is applied to the sample application pad and it subsequently migrates to the other parts of strip. At conjugate pad, target analyte is captured by the immobilized labeled antibody or aptamer conjugate and results in the formation of labeled antibody conjugate/analyte complex. This complex now reaches at nitrocellulose membrane and moves under capillary action. At test line, label antibody conjugate/analyte complex is captured by another antibody which is primary to the analyte. Analyte becomes sandwiched between labeled and primary antibodies forming labeled antibody conjugate/analyte/primary antibody complex. Excess labeled antibody conjugate will be captured at control zone by secondary antibody. Buffer or excess solution goes to absorption pad. Intensity of color at test line corresponds to the amount of target analyte and is measured with an optical strip reader or visually inspected. Appearance of color at control line ensures that a strip is functioning properly.

*Competitive format:* Such a format suits best for low molecular weight compounds which cannot bind two antibodies simultaneously. Absence of color at test line is an indication for the presence of analyte while appearance of color both at test and control lines indicates a negative result. Competitive format has two layouts. In the first layout, solution containing target analyte is applied onto the sample application pad and prefixed labeled biomolecule (antibody/aptamer) conjugate gets hydrated and starts flowing with moving liquid. Test line contains pre-immobilized antigen (same analyte to be detected) which binds specifically to label conjugate. Control line contains pre-immobilized secondary antibody which has the ability to bind with labeled antibody conjugate. When liquid sample reaches at the test line, pre-immobilized antigen will bind to the labeled conjugate in case target analyte in sample solution is absent or present in such a low quantity that some sites of labeled antibody conjugate were vacant. Antigen in the sample solution and the one which is immobilized at test line of strip compete to bind with labeled conjugate. In another layout, labeled analyte conjugate is dispensed at conjugate pad while a primary antibody to analyte is dispensed at test line. After application of analyte solution a competition takes place between analyte and labeled analyte to bind with primary antibody at test line.

*Multiplex detection format:* Multiplex detection format is used for detection of more than one target species and assay is performed over the strip containing test lines equal to number of target species to be analyzed. It is highly desirable to analyze multiple analytes simultaneously under same set of conditions. Multiplex detection format is very useful in clinical diagnosis where multiple analytes which are inter-dependent in deciding about the stage of a disease are to be detected. Lateral flow strips for this purpose can be built in various ways i.e. by increasing length and test lines on conventional strip, making other structures like stars or T-shapes. Shape of strip for LFIA will be dictated by number of target analytes. Miniaturized versions of LFIA based on microarrays for multiplex detection of DNA sequences have been reported to have several advantages such as less consumption of test reagents, requirement of lesser sample volume and better sensitivity.

*Labels:* Any material that is used as a label should be detectable at very low concentrations and it should retain its properties upon conjugation with biorecognition molecules. This conjugation is also expected not to change features of biorecognition probes. Ease in conjugation with biomolecules and stability over longer period of time are desirable features for a good label. Concentrations of labels down to $10^{-9}$ M are optically detectable. After the completion of assay, some labels generate direct signal (as color from gold colloidal) while others require additional steps to produce analytical signal (as enzymes produce detectable product upon reaction with suitable substrate). Hence the labels which give direct signal are preferable in LFA because of less time consumption and reduced procedure.

*Gold nanoparticles:* Colloidal gold nanoparticles are the most commonly used labels in LFA. Colloidal gold is inert and gives very perfect spherical particles. These particles have very high affinity toward biomolecules and can be easily functionalized. Optical properties of gold nanoparticles are dependent on size and shape. Size of particles can be tuned by use of suitable chemical additives. Their unique features include environment friendly preparation, high affinity toward proteins and biomolecules, enhanced stability, exceptionally higher values for charge transfer and good optical signaling. Optical signal of gold nanoparticles in colorimetric LFA can be amplified by deposition of silver, gold nanoparticles and enzymes.

*Magnetic particles and aggregates:* Colored magnetic particles produce color at the test line which is measured by an optical strip reader but magnetic signals coming from magnetic particles can also be used as detection signals and recorded by a magnetic assay reader. Magnetic signals are stable for longer time compared to optical signals and they enhance sensitivity of LFA by 10 to 1000 folds.

*Fluorescent and luminescent materials:* Fluorescent molecules are widely used in LFA as labels and the amount of fluorescence is used to quantitate the concentration of analyte in the sample. Detection of proteins is accomplished by using organic fluorophores such as rhodamine as labels in LFA.

Current developments in nanomaterial have headed to manufacture of quantum dots which display very unique electrical and optical properties. These semiconducting particles are not only water soluble but can also be easily combined with biomolecules because of closeness in dimensions. Owing to their unique optical properties, quantum dots have come up as a substitute to organic fluorescent dyes. Like gold nanoparticles QDs show size dependent optical properties and a broad spectrum of wavelengths can be monitored. Single light source is sufficient to excite quantum dots of all different sizes. QDs have high photo stability and absorption coefficients.

Upconverting phosphors (UCP) are characterized by their excitation in infra-red region and emission in high energy visible region. Compared to other fluorescent materials, they have a unique advantage of not showing any auto fluorescence. Because of their excitation in IR regions, they do not photo degrade biomolecules. A major advantage lies in their production from easily available bulk materials. Although difference in batch to batch preparation of UCP reporters can affect sensitivity of analysis in LFA, it was observed that they can enhance sensitivity of analytical signal by 10 to 100 folds compared to gold nanoparticles or colored latex beads, when analysis is carried out under same set of biological conditions.

*Enzymes:* Enzymes are also employed as labels in LFA. But they increase one step in LFA which is application of suitable substrate after complete assay. This substrate will produce color at test and control lines as a result of enzymatic reaction. In case of enzymes, selection of suitable enzyme substrate combination is one necessary requirement in order to get a colored product for strip reader or electroactive product for electrochemical detection. In other words, sensitivity of detection is dependent on enzyme substrate combination.

*Colloidal carbon:* Colloidal carbon is comparatively inexpensive label and its production can be easily scaled up. Because of their black color, carbon NPs can be easily detected with high sensitivity. Colloidal carbon can be functionalized with a large variety of biomolecules for detection of low and high molecular weight analytes.

*Detection systems:* In case of gold nanoparticles or other color producing labels, qualitative or semi-quantitative analysis can be done by visual inspection of colors at test and control lines. The major advantage of visual inspection is rapid qualitative answer in "Yes" or "NO". Such quick replies about presence of an analyte in clinical analysis have very high importance. Such tests help doctors to make an immediate decision near the patients in hospitals in situations where test results from central labs cannot be waited for because of huge time consumption. But for quantification, optical strip readers are employed for measurement of the intensity of colors produced at test and control lines of strip. This is achieved by inserting the strips into a strip reader and intensities are recorded simultaneously by imaging softwares. Optical images of the strips can also be recorded with a camera and then processed by using a suitable software. Procedure includes proper placement of strip under the camera and a controlled amount of light is thrown on the areas to be observed. Such systems use monochromatic light and wavelength of light can be adjusted to get a good contrast among test and control lines and background. In order to provide good quantitative and reproducible results, detection system should be sensitive to different intensities of colors. Optical standards can be used to calibrate an optical reader device. Automated systems have advantages over manual imaging and processing in terms of time consumption, interpretation of results and adjustment of variables.

In case of fluorescent labels, a fluorescence strip reader is used to record fluorescence intensity of test and control lines. Fluorescence brightness of test line increased with an increase in nitrated seruloplasmin concentration in human serum when it was detected with a fluorescence strip reader. A photoelectric sensor was also used for detection in LFIA where colloidal gold is exposed to light emitting diode and resulting photoelectrons are recorded. Chemiluminescence which results from reaction of enzyme and substrate is measured as a response to amount of target analyte. Magnetic strip readers and electrochemical detectors are also reported as detection systems in LFTS but they are not very common. Selection of detector is mainly determined by the label employed in analysis.

Methods of quantitating levels of RNA markers are known in the art and are summarized infra:

**Northern Blot analysis:** This method involves the detection of a particular RNA in a mixture of RNAs. An RNA sample is denatured by treatment with an agent (e.g., formaldehyde) that prevents hydrogen bonding between base pairs, ensuring that all the RNA molecules have an unfolded, linear conformation. The individual RNA molecules are then separated according to size by gel electrophoresis and transferred to a nitrocellulose or a nylon-based membrane to which the denatured RNAs adhere. The membrane is then exposed to labeled DNA probes. Probes may be labeled using radio-isotopes or enzyme linked nucleotides. Detection may be using autoradiography, colorimetric reaction or chemiluminescence. This method allows both quantitation of an amount of particular RNA molecules and determination of its identity by a relative position on the membrane which is indicative of a migration distance in the gel during electrophoresis.

**RT-PCR analysis:** This method uses PCR amplification of relatively rare RNAs molecules. First, RNA molecules are purified from the cells and converted into complementary DNA (cDNA) using a reverse transcriptase enzyme (such as an MMLV-RT) and primers such as, oligo dT, random hexamers or gene specific primers. Then by applying gene specific primers and Taq DNA polymerase, a PCR amplification reaction is carried out in a PCR machine. Those of skills in the art are capable of selecting the length and sequence of the gene specific primers and the PCR conditions (*i.e.,* annealing temperatures, number of cycles and the like) which are suitable for detecting specific RNA molecules. It will be appreciated that a semi-quantitative RT-PCR reaction can be employed by adjusting the number of PCR cycles and comparing the amplification product to known controls.

**RNA in situ hybridization stain:** In this method DNA or RNA probes are attached to the RNA molecules present in the cells. Generally, the cells are first fixed to microscopic slides to preserve the cellular structure and to prevent the RNA molecules from being degraded and then are subjected to hybridization buffer containing the labeled probe. The hybridization buffer includes reagents such as formamide and salts (e.g., sodium chloride and sodium citrate) which enable specific hybridization of the DNA or RNA probes with their target mRNA molecules *in situ* while avoiding non-specific binding of probe. Those of skills in the art are capable of adjusting the hybridization conditions (*i.e.,* temperature, concentration of salts and formamide and the like) to specific probes and types of cells. Following hybridization, any unbound probe is washed off and the bound probe is detected using known methods. For example, if a radio-labeled probe is used, then the slide is subjected to a photographic emulsion which reveals signals generated using radio-labeled probes; if the probe was labeled with an enzyme then the enzyme-specific substrate is added for the formation of a colorimetric reaction; if the probe is labeled using a fluorescent label, then the bound probe is revealed using a fluorescent microscope; if the probe is labeled using a tag (e.g., digoxigenin, biotin, and the like) then the bound probe can be detected following interaction with a tag-specific antibody which can be detected using known methods.

**In situ RT-PCR stain:** This method is described in Nuovo GJ, et al. [Intracellular localization of polymerase chain reaction (PCR)-amplified hepatitis C cDNA. Am J Surg Pathol. 1993, 17: 683-90] and Komminoth P, et al. [Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. Pathol Res Pract. 1994, 190: 1017-25]. Briefly, the RT-PCR reaction is performed on fixed cells by incorporating labeled nucleotides to the PCR reaction. The reaction is carried on using a specific *in situ* RT-PCR apparatus such as the laser-capture microdissection PixCell I LCM system available from Arcturus Engineering (Mountainview, CA).

### DNA microarrays/DNA chips:

The expression of thousands of genes may be analyzed simultaneously using DNA microarrays, allowing analysis of the complete transcriptional program of an organism during specific developmental processes or physiological responses. DNA microarrays consist of thousands of individual gene sequences attached to closely packed areas on the surface of a support such as a glass microscope slide. Various methods have been developed for preparing DNA microarrays. In one method, an approximately 1 kilobase segment of the coding region of each gene for analysis is individually PCR amplified. A robotic apparatus is employed to apply each amplified DNA sample to closely spaced zones on the surface of a glass microscope slide, which is subsequently processed by thermal and chemical treatment to bind the DNA sequences to the surface of the support and denature them. Typically, such arrays are about 2 x 2 cm and contain about individual nucleic acids 6000 spots. In a variant of the technique, multiple DNA oligonucleotides, usually 20 nucleotides in length, are synthesized from an initial nucleotide that is covalently bound to the surface of a support, such that tens of thousands of identical oligonucleotides are synthesized in a small square zone on the surface of the support. Multiple oligonucleotide sequences from a single gene are synthesized in neighboring regions of the slide for analysis of expression of that gene. Hence, thousands of genes can be represented on one glass slide. Such arrays of synthetic oligonucleotides may be referred to in the art as "DNA chips", as opposed to "DNA microarrays", as described above [Lodish et al. (eds.). Chapter 7.8: DNA Microarrays: Analyzing Genome-Wide Expression. In: Molecular Cell Biology, 4th ed., W. H. Freeman, New York. (2000)].

*Oligonucleotide microarray* - In this method oligonucleotide probes capable of specifically hybridizing with the polynucleotides of some embodiments of the invention are attached to a solid surface (e.g., a glass wafer). Each oligonucleotide probe is of approximately 20-25 nucleic acids in length. To detect the expression pattern of the polynucleotides of some embodiments of the invention in a specific cell sample (e.g., blood cells), RNA is extracted from the cell sample using methods known in the art (using e.g., a TRIZOL solution, Gibco BRL, USA). Hybridization can take place using either labeled oligonucleotide probes (e.g., 5'-biotinylated probes) or labeled fragments of complementary DNA (cDNA) or RNA (cRNA). Briefly, double stranded cDNA is prepared from the RNA using reverse transcriptase (RT) (e.g., Superscript II RT), DNA ligase and DNA polymerase I, all according to manufacturer's instructions (Invitrogen Life Technologies, Frederick, MD, USA). To prepare labeled cRNA, the double stranded cDNA is subjected to an *in vitro* transcription reaction in the presence of biotinylated nucleotides using e.g., the BioArray High Yield RNA Transcript Labeling Kit (Enzo, Diagnostics, Affymetix Santa Clara CA). For efficient hybridization the labeled cRNA can be fragmented by incubating the RNA in 40 mM Tris Acetate (pH 8.1), 100 mM potassium acetate and 30 mM magnesium acetate for 35 minutes at 94 °C. Following hybridization, the microarray is washed and the hybridization signal is scanned using a confocal laser fluorescence scanner which measures fluorescence intensity emitted by the labeled cRNA bound to the probe arrays.

For example, in the Affymetrix microarray (Affymetrix®, Santa Clara, CA) each gene on the array is represented by a series of different oligonucleotide probes, of which, each probe pair consists of a perfect match oligonucleotide and a mismatch oligonucleotide. While the perfect match probe has a sequence exactly complimentary to the particular gene, thus enabling the measurement of the level of expression of the particular gene, the mismatch probe differs from the perfect match probe by a single base substitution at the center base position. The hybridization signal is scanned using the Agilent scanner, and the Microarray Suite software subtracts the non-specific signal resulting from the mismatch probe from the signal resulting from the perfect match probe.

*RNA sequencing:* Methods for RNA sequence determination are generally known to the person skilled in the art. Preferred sequencing methods are next generation sequencing methods or parallel high throughput sequencing methods. An example of an envisaged sequence method is pyrosequencing, in particular 454 pyrosequencing, e.g. based on the Roche 454 Genome Sequencer. This method amplifies DNA inside water droplets in an oil solution with each droplet containing a single DNA template attached to a single primer-coated bead that then forms a clonal colony. Pyrosequencing uses luciferase to generate light for detection of the individual nucleotides added to the nascent DNA, and the combined data are used to generate sequence read-outs. Yet another envisaged example is Illumina or Solexa sequencing, e.g. by using the Illumina Genome Analyzer technology, which is based on reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle. Yet another example is the use of Applied Biosystems' SOLiD technology, which employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing. A further method is based on Helicos' Heliscope technology, wherein fragments are captured by polyT oligomers tethered to an array. At each sequencing cycle, polymerase and single fluorescently labeled nucleotides are added and the array is imaged. The fluorescent tag is subsequently removed and the cycle is repeated. Further examples of sequencing techniques encompassed within the methods of the present invention are sequencing by hybridization, sequencing by use of nanopores, microscopy-based sequencing techniques, microfluidic Sanger sequencing, or microchip-based sequencing methods. The present invention also envisages further developments of these techniques, e.g. further improvements of the accuracy of the sequence determination, or the time needed for the determination of the genomic sequence of an organism etc.

According to one embodiment, the sequencing method comprises deep sequencing.

As used herein, the term "deep sequencing" refers to a sequencing method wherein the target sequence is read multiple times in the single test. A single deep sequencing run is composed of a multitude of sequencing reactions run on the same target sequence and each, generating independent sequence readout.

It will be appreciated that in order to analyze the amount of an RNA, oligonucleotides may be used that are capable of hybridizing thereto or to cDNA generated therefrom. According to one embodiment a single oligonucleotide is used to determine the presence of a particular determinant, at least two oligonucleotides are used to determine the presence of a particular determinant, at least five oligonucleotides are used to determine the presence of a particular determinant, at least four oligonucleotides are used to determine the presence of a particular determinant, at least five or more oligonucleotides are used to determine the presence of a particular determinant.

Oligonucleotides that are capable of specifically hybridizing to the determinants described herein may comprise a detectable moiety (e.g. a fluorescent moiety, a phosphorescent moiety) and/or a quencher moiety.

According to embodiments of the present invention, the fluorescent moiety is selected from the group consisting of an Atto dye, fluorescein, fluorescein chlorotriazinyl, rhodamine green, rhodamine red, tetramethylrhodamine, FITC, Oregon green, Alexa Fluor, FAM, JOE, ROX, HEX, Texas Red, TET, TRITC, TAMRA, cyanine-based dye and thiadicarbocyanine dye.

According to embodiments of the present invention, the quencher moiety is selected from the group consisting of Dabcyl, TAMRA, Eclipse, DDQ, QSY, Blackberry Quencher, Black Hole Quencher, Qxl, Iowa black FQ, Iowa black RQ, and IRDye QC-1.

When more than one oligonucleotide is used, the sequence of the oligonucleotides may be selected such that they hybridize to the same exon of the RNA determinant or different exons of the RNA determinant. In one embodiment, at least one of the oligonucleotides hybridizes to the 3' exon of the RNA determinant. In another embodiment, at least one of the oligonucleotides hybridizes to the 5' exon of the RNA determinant.

Classification of subjects into subgroups according to any of the aspects of the present invention is preferably done with an acceptable level of clinical or diagnostic accuracy. An "acceptable degree of diagnostic accuracy", is herein defined as a test or assay (such as the test used in some aspects of the invention) in which the AUC (area under the ROC curve for the test or assay) is at least 0.60, desirably at least 0.65, more desirably at least 0.70, preferably at least 0.75, more preferably at least 0.80, and most preferably at least 0.85.

By a "very high degree of diagnostic accuracy", it is meant a test or assay in which the AUC (area under the ROC curve for the test or assay) is at least 0.75, 0.80, desirably at least 0.85, more desirably at least 0.875, preferably at least 0.90, more preferably at least 0.925, and most preferably at least 0.95.

Alternatively, the methods diagnose viral infections with at least 75% total accuracy, more preferably 80%, 85%, 90%, 95%, 97%, 98%, 99% or greater total accuracy.

Alternatively, the methods diagnose viral infections with an MCC larger than 0.2, 0.3, 0.4, 0.5, 0.6, 0.7, 0.8, 0.9 or 1.0.

For any of the aspects described herein above, preferably no more than 50 protein markers are analyzed in a single test/analysis. In another embodiment, no more than 40 protein markers are analyzed in a single test/analysis. In another embodiment, no more than 30 protein markers are analyzed in a single test/analysis. In another embodiment, no more than 20 protein markers are analyzed in a single test/analysis. In another embodiment, no more than 10 protein markers are analyzed in a single test/analysis. In another embodiment, no more than 9 protein markers are analyzed in a single test/analysis. In another embodiment, no more than 8 protein markers are analyzed in a single test/analysis. In another embodiment, no more than 7 protein markers are analyzed in a single test/analysis. In another embodiment, no more than 6 protein markers are analyzed in a single test/analysis. In another embodiment, no more than 5 protein markers are analyzed in a single test/analysis. In another embodiment, no more than 4 protein markers are analyzed in a single test/analysis. In another embodiment, no more than 3 protein markers are analyzed in a single test/analysis. In another embodiment, no more than 2 protein markers are analyzed in a single test/analysis. In another embodiment, no more than 1 protein marker is analyzed in a single test/analysis.

For any of the aspects described herein above, preferably no more than 50 RNA markers are analyzed in a single test/analysis. In another embodiment, no more than 40 RNA markers are analyzed in a single test/analysis. In another embodiment, no more than 30 RNA markers are analyzed in a single test/analysis. In another embodiment, no more than 20 RNA markers are analyzed in a single test/analysis. In another embodiment, no more than 10 RNA markers are analyzed in a single test/analysis. In another embodiment, no more than 9 RNA markers are analyzed in a single test/analysis. In another embodiment, no more than 8 RNA markers are analyzed in a single test/analysis. In another embodiment, no more than 7 RNA markers are analyzed in a single test/analysis. In another embodiment, no more than 6 RNA markers are analyzed in a single test/analysis. In another embodiment, no more than 5 RNA markers are analyzed in a single test/analysis. In another embodiment, no more than 4 RNA markers are analyzed in a single test/analysis. In another embodiment, no more than 3 RNA markers are analyzed in a single test/analysis. In another embodiment, no more than 2 RNA markers are analyzed in a single test/analysis. In another embodiment, no more than 1 RNA marker is analyzed in a single test/analysis.

Some aspects of the present invention also comprise a kit with a detection reagent that binds to one or more determinant (e.g. protein or RNA). Also provided by the invention is an array of detection reagents, e.g., antibodies that can bind to one or more protein determinants or oligonucleotides that can bind to one or more RNA determinants). In one embodiment, the kit contains antibodies and/or oligonucleotides that specifically bind to at least two, three, four, five, six, seven, eight, nine or ten determinants which appear in any of Tables 1 and 7-12.

According to an exemplary embodiment, the kit (or array) does not specifically detect more than 2 determinants, does not specifically detect more than 3 determinants, does not specifically detect more than 4 determinants, does not specifically detect more than 5 determinants, does not specifically detect more than 6 determinants does not specifically detect more than 7 determinants, does not specifically detect more than 8 determinants, does not specifically detect more than 9 determinants, does not specifically detect more than 10 determinants.

Preferably, the kit does not contain components which specifically bind to more than 15, 20 25, 30, 35, 40, 45, 50, 100 different or 200 different determinants.

Preferably, the concentration of the determinants is measured within about 24 hours after sample is obtained. Alternatively, the concentration of the polypeptide-determinant is measured in a sample that was stored at 12 $^0$C or lower, when storage begins less than 24 hours after the sample is obtained.

In some embodiments the sample could have been stored in either room temperature, 4$^0$C, -20$^0$C or -80$^0$C before measurement is performed.

In some embodiments the sample could have been stored for 1, 2, 3, 4, 5, 10, 12, 15, 20 or 24 hours before measurement is performed.

In some embodiments the sample may be stored for less than 5 minutes, 10 minutes, 20 minutes, 30 minutes, 45 minutes or 60 minutes before measurement is performed.

In some embodiments, the sample is collected in a serum separator tube (SST). Following collection, the sample may be left at room temperature for at least 5, 10, 12, 15, 20, 25 30 minutes to allow blood clotting and then centrifuged for about 5-30 minutes (e.g. at least 5, 10, 12, 15, 20, 25, or 30 minutes) at 1200 x g or at about 3000 RPM.

Furthermore, traditional risk factors and additional clinical parameters may be used to diagnose the viral disease of the non-symptomatic subject, to classify contagiousness or to rule in a coronaviral infection or to determine the severity of the virus.

"Traditional laboratory risk factors" encompass biomarkers isolated or derived from subject samples and which are currently evaluated in the clinical laboratory and used in traditional global risk assessment algorithms, such as absolute neutrophil count (abbreviated ANC), absolute lymphocyte count (abbreviated ALC), white blood count (abbreviated WBC), neutrophil % (defined as the fraction of white blood cells that are neutrophils and abbreviated Neu (%)), lymphocyte % (defined as the fraction of white blood cells that are lymphocytes and abbreviated Lym (%)), monocyte % (defined as the fraction of white blood cells that are monocytes and abbreviated Mon (%)),Sodium (abbreviated Na), Potassium (abbreviated K), Bilirubin (abbreviated Bili).

"Clinical parameters" encompass all non-sample or non-analyte biomarkers of subject health status or other characteristics, such as, without limitation, age (Age), ethnicity (RACE), gender (Sex), core body temperature (abbreviated "temperature"), maximal core body temperature since initial appearance of symptoms (abbreviated "maximal temperature"), time from initial appearance of symptoms (abbreviated "time from symptoms") or family history (abbreviated FamHX).

Combining the levels of particular markers is typically effected using algorithms or formulas as described herein below.

A "formula," "algorithm," or "model" is any mathematical equation, algorithmic, analytical or programmed process, or statistical technique that takes one or more continuous or categorical inputs (herein called "parameters") and calculates an output value, sometimes referred to as an "index" or "index value". Non-limiting examples of "formulas" include sums, ratios, and regression operators, such as coefficients or exponents, biomarker value transformations and normalizations (including, without limitation, those normalization schemes based on clinical-determinants, such as gender, age, or ethnicity), rules and guidelines, statistical classification models, and neural networks trained on historical populations.

Of particular use in combining determinants are linear and non-linear equations and statistical classification analyses to determine the relationship between levels of determinants detected in a subject sample and the subject's risk assessment. In panel and combination construction, of particular interest are structural and syntactic statistical classification algorithms, and methods of index construction, utilizing pattern recognition features, including established techniques such as cross-correlation, Principal Components Analysis (PCA), factor rotation, Logistic Regression (LogReg), Linear Discriminant Analysis (LDA), Eigengene Linear Discriminant Analysis (ELDA), Support Vector Machines (SVM), Random Forest (RF), Recursive Partitioning Tree (RPART), as well as other related decision tree classification techniques, Shrunken Centroids (SC), StepAIC, Kth-Nearest Neighbor, Boosting, Decision Trees, Neural Networks, Bayesian Networks, and Hidden Markov Models, among others. Other techniques may be used in survival and time to event hazard analysis, including Cox, Weibull, Kaplan-Meier and Greenwood models well known to those of skill in the art. Many of these techniques are useful either combined with a determinant selection technique, such as forward selection, backwards selection, or stepwise selection, complete enumeration of all potential panels of a given size, genetic algorithms, or they may themselves include biomarker selection methodologies in their own technique. These may be coupled with information criteria, such as Akaike's Information Criterion (AIC) or Bayes Information Criterion (BIC), in order to quantify the tradeoff between additional biomarkers and model improvement, and to aid in minimizing overfit. The resulting predictive models may be validated in other studies, or cross-validated in the study they were originally trained in, using such techniques as Bootstrap, Leave-One-Out (LOO) and 10-Fold cross-validation (10-Fold CV).

Any formula may be used to combine TRAIL levels with the additional determinant into indices useful in the practice of the invention. As indicated above, and without limitation, such indices may indicate, among the various other indications, the probability, likelihood, absolute or relative risk, time to or rate of conversion from one to another disease states, or make predictions of future biomarker measurements of infection. This may be for a specific time period or horizon, or for remaining lifetime risk, or simply be provided as an index relative to another reference subject population.

Although various preferred formulas are described here, several other model and formula types beyond those mentioned herein and in the definitions above are well known to one skilled in the art. The actual model type or formula used may itself be selected from the field of potential models based on the performance and diagnostic accuracy characteristics of its results in a training population.

Preferred formulas include the broad class of statistical classification algorithms, and in particular the use of discriminant analysis. The goal of discriminant analysis is to predict class membership from a previously identified set of features. In the case of linear discriminant analysis (LDA), the linear combination of features is identified that maximizes the separation among groups by some criteria. Features can be identified for LDA using an eigengene based approach with different thresholds (ELDA) or a stepping algorithm based on a multivariate analysis of variance (MANOVA). Forward, backward, and stepwise algorithms can be performed that minimize the probability of no separation based on the Hotelling-Lawley statistic.

Eigengene-based Linear Discriminant Analysis (ELDA) is a feature selection technique developed by Shen et al. (2006). The formula selects features (e.g. biomarkers) in a multivariate framework using a modified eigen analysis to identify features associated with the most important eigenvectors. "Important" is defined as those eigenvectors that explain the most variance in the differences among samples that are trying to be classified relative to some threshold.

A support vector machine (SVM) is a classification formula that attempts to find a hyperplane that separates two classes. This hyperplane contains support vectors, data points that are exactly the margin distance away from the hyperplane. In the likely event that no separating hyperplane exists in the current dimensions of the data, the dimensionality is expanded greatly by projecting the data into larger dimensions by taking non-linear functions of the original variables (Venables and Ripley, 2002). Although not required, filtering of features for SVM often improves prediction. Features (e.g., biomarkers) can be identified for a support vector machine using a non-parametric Kruskal-Wallis (KW) test to select the best univariate features. A random forest (RF, Breiman, 2001) or recursive partitioning (RPART, Breiman et al., 1984) can also be used separately or in combination to identify biomarker combinations that are most important. Both KW and RF require that a number of features be selected from the total. RPART creates a single classification tree using a subset of available biomarkers.

Other formula may be used in order to pre-process the results of individual determinant measurement into more valuable forms of information, prior to their presentation to the predictive formula. Most notably, normalization of biomarker results, using either common mathematical transformations such as logarithmic or logistic functions, as normal or other distribution positions, in reference to a population's mean values, etc. are all well known to those skilled in the art. Of particular interest are a set of normalizations based on clinical-determinants such as age, time from symptoms, gender, race, or sex, where specific formula are used solely on subjects within a class or continuously combining a clinical-determinants as an input. In other cases, analyte-based biomarkers can be combined into calculated variables which are subsequently presented to a formula.

In addition to the individual parameter values of one subject potentially being normalized, an overall predictive formula for all subjects, or any known class of subjects, may itself be recalibrated or otherwise adjusted based on adjustment for a population's expected prevalence and mean biomarker parameter values, according to the technique outlined in D'Agostino et al, (2001) JAMA 286:180-187, or other similar normalization and recalibration techniques. Such epidemiological adjustment statistics may be captured, confirmed, improved and updated continuously through a registry of past data presented to the model, which may be machine readable or otherwise, or occasionally through the retrospective query of stored samples or reference to historical studies of such parameters and statistics. Additional examples that may be the subject of formula recalibration or other adjustments include statistics used in studies by Pepe, M. S. et al, 2004 on the limitations of odds ratios; Cook, N. R., 2007 relating to ROC curves. Finally, the numeric result of a classifier formula itself may be transformed post-processing by its reference to an actual clinical population and study results and observed endpoints, in order to calibrate to absolute risk and provide confidence intervals for varying numeric results of the classifier or risk formula.

Some determinants may exhibit trends that depends on the patient age (e.g. the population baseline may rise or fall as a function of age). One can use an Age dependent normalization or stratification scheme to adjust for age related differences. Performing age dependent normalization or stratification can be used to improve the accuracy of determinants for differentiating between different types of infections. For example, one skilled in the art can generate a function that fits the population mean levels of each determinant as function of age and use it to normalize the determinant of individual subjects levels across different ages. Another example is to stratify subjects according to their age and determine age specific thresholds or index values for each age group independently.

As well as integrating age in order to optimize the above, the present inventors further contemplate integrating patient comorbidities (background states) in order to optimize the above, Integrating patient specific clinical syndrome in order to optimize the above and/or integrating additional clinical data to optimize the above.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

In the context of the present invention, the following abbreviations may be used: ANC = Absolute neutrophil count; ANN = Artificial neural networks; AUC = Area under the receiver operating curve; BP = Bordetella pertussis; CHF = Congestive heart failure; CI = Confidence interval; CID = Congenital immune deficiency; CLL = Chronic lymphocytic leukemia; CMV = Cytomegalovirus; CNS = Central nervous system; COPD = Chronic obstructive pulmonary disease; CP = Chlamydophila pneumonia; CRP = C-reactive protein; CSF = Cerebrospinal fluid; CV = Coefficient of variation; DOR = Diagnostic odds ratio; EBV = Epstein bar virus; eCRF = Electronic case report form; ED = Emergency department, ELISA = Enzyme-linked immunosorbent assay; FDR = False discovery rate; FMF = Familial Mediterranean fever; G-CSF = Granulocyte colony-stimulating factor; GM-CSF = Granulocyte-macrophage colony-stimulating factor; HBV = Hepatitis B virus; HCV = Hepatitis C virus; HI = Haemophilus influenza; HIV = Human immunodeficiency virus; IDE = Infectious disease experts; IL = Interleukin; IRB = institutional review board; IVIG = Intravenous immunoglobulin; KNN = K-nearest neighbors; LP = Legionella pneumophila; LR+ = Positive likelihood ratio; LR- = Negative likelihood ratio; LRTI = Lower respiratory tract infections; mAb = Monoclonal antibodies; MDD = Minimum detectable dose; MDS = Myelodysplastic syndrome; MP = Mycoplasma pneumonia; MPD = Myeloproliferative disease; NPV = Negative predictive value; PCT = Procalcitonin; PED = Pediatric emergency department; PPV = Positive predictive value; QA = Quality assurance; RSV = Respiratory syncytial virus; RV = Rhinovirus; SIRS = systemic inflammatory syndrome; SP = Streptococcus pneumonia; STARD = Standards for Reporting of Diagnostic Accuracy; SVM = Support vector machine; TNF = Tumor necrosis factor; URTI = Upper respiratory tract infection; UTI = Urinary tract infection; WBC = White blood cell;

WS = Wilcoxon rank-sum.

In the context of the present invention, the following statistical terms may be used:

"TP" is true positive, means positive test result that accurately reflects the tested-for activity. For example in the context of the present invention a TP, is for example but not limited to, truly classifying a bacterial infection as such.

"TN" is true negative, means negative test result that accurately reflects the tested-for activity. For example in the context of the present invention a TN, is for example but not limited to, truly classifying a viral infection as such.

"FN" is false negative, means a result that appears negative but fails to reveal a situation. For example in the context of the present invention a FN, is for example but not limited to, falsely classifying a bacterial infection as a viral infection.

"FP" is false positive, means test result that is erroneously classified in a positive category. For example in the context of the present invention a FP, is for example but not limited to, falsely classifying a viral infection as a bacterial infection.

"Sensitivity" is calculated by TP/(TP+FN) or the true positive fraction of disease subjects.

"Specificity" is calculated by TN/(TN+FP) or the true negative fraction of non-disease or normal subjects.

"Total accuracy" is calculated by (TN + TP)/(TN + FP +TP + FN).

"Positive predictive value" or "PPV" is calculated by TP/(TP+FP) or the true positive fraction of all positive test results. It is inherently impacted by the prevalence of the disease and pre-test probability of the population intended to be tested.

"Negative predictive value" or "NPV" is calculated by TN/(TN + FN) or the true negative fraction of all negative test results. It also is inherently impacted by the prevalence of the disease and pre-test probability of the population intended to be tested. See, e.g., O'Marcaigh AS, Jacobson RM, "Estimating The Predictive Value Of A Diagnostic Test, How To Prevent Misleading Or Confusing Results," Clin. Ped. 1993, 32(8): 485-491, which discusses specificity, sensitivity, and positive and negative predictive values of a test, e.g., a clinical diagnostic test.

"MCC" (Mathews Correlation coefficient) is calculated as follows: MCC = (TP * TN - FP * FN) / {(TP + FN) * (TP + FP) * (TN + FP) * (TN + FN)}^0.5

where TP, FP, TN, FN are true-positives, false-positives, true-negatives, and false-negatives, respectively. Note that MCC values range between -1 to +1, indicating completely wrong and perfect classification, respectively. An MCC of 0 indicates random classification. MCC has been shown to be a useful for combining sensitivity and specificity into a single metric (Baldi, Brunak et al. 2000). It is also useful for measuring and optimizing classification accuracy in cases of unbalanced class sizes (Baldi, Brunak et al. 2000).

"Accuracy" refers to the degree of conformity of a measured or calculated quantity (a test reported value) to its actual (or true) value. Clinical accuracy relates to the proportion of true outcomes (true positives (TP) or true negatives (TN) versus misclassified outcomes (false positives (FP) or false negatives (FN)), and may be stated as a sensitivity, specificity, positive predictive values (PPV) or negative predictive values (NPV), Mathews correlation coefficient (MCC), or as a likelihood, odds ratio, Receiver Operating Characteristic (ROC) curve, Area Under the Curve (AUC) among other measures.

"Analytical accuracy" refers to the reproducibility and predictability of the measurement process itself, and may be summarized in such measurements as coefficients of variation (CV), Pearson correlation, and tests of concordance and calibration of the same samples or controls with different times, users, equipment and/or reagents. These and other considerations in evaluating new biomarkers are also summarized in Vasan, 2006.

"Performance" is a term that relates to the overall usefulness and quality of a diagnostic or prognostic test, including, among others, clinical and analytical accuracy, other analytical and process characteristics, such as use characteristics (e.g., stability, ease of use), health economic value, and relative costs of components of the test. Any of these factors may be the source of superior performance and thus usefulness of the test, and may be measured by appropriate "performance metrics," such as AUC and MCC, time to result, shelf life, etc. as relevant.

By "statistically significant", it is meant that the alteration is greater than what might be expected to happen by chance alone (which could be a "false positive"). Statistical significance can be determined by any method known in the art. Commonly used measures of significance include the p-value, which presents the probability of obtaining a result at least as extreme as a given data point, assuming the data point was the result of chance alone. A result is often considered highly significant at a p-value of 0.05 or less.

The term "determinant" as used herein refers to a disease associated parameter or biomarker.

According to yet another aspect of the present invention there is provided a method of analyzing biological data, the biological data containing expression values of the TRAIL protein, the IP10 protein, and the CRP protein in a body liquid sample of the subject, the method comprising:

by a hardware processor, receiving the expression values, calculating a distance between a segment of a curved line and an axis defined by a direction, determining a score classifying a severity of a coronaviral infection of the subject based on said distance, and generating an output indicative of said score;

wherein said distance is calculated at a point over said curved line defined by a coordinate $\delta$ along said direction, said coordinate $\delta$ being defined based on the expression values,

wherein at least 90% of said segment is between a lower bound line $f(\delta)-\varepsilon_0$ and an upper bound line $f(\delta)+\varepsilon_1$, wherein each of said $\varepsilon_0$ and said $\varepsilon_1$ is less than 0.5, and wherein said $f(\delta)$ comprises a multiplication between a saturation function of said $\delta$ coordinate, and a saturation function of the expression value of the CRP protein.

It is to be understood that, unless otherwise defined, the operations described hereinbelow can be executed either contemporaneously or sequentially in many combinations or orders of execution. Specifically, the ordering of the flowchart diagrams is not to be considered as limiting. For example, two or more operations, appearing in the following description or in the flowchart diagrams in a particular order, can be executed in a different order (e.g., a reverse order) or substantially contemporaneously. Additionally, several operations described below are optional and may not be executed.

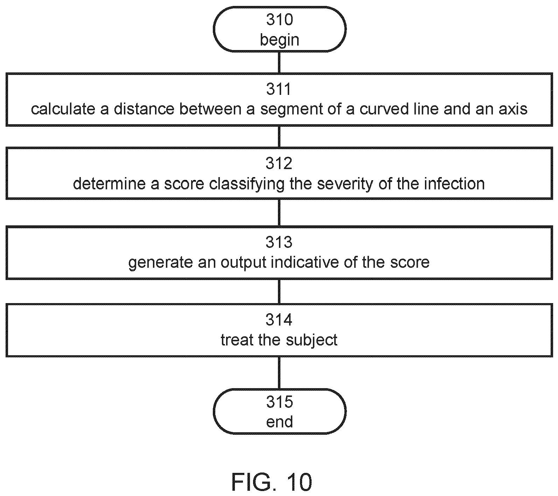

FIG. 10 is a flowchart diagram of a method suitable for analyzing biological data obtained from a subject, according to various exemplary embodiments of the present invention.

Any of the methods described herein can be embodied in many forms. For example, it can be embodied in on a tangible medium such as a computer for performing the method operations. It can be embodied on a computer readable medium, comprising computer readable instructions for carrying out the method operations. It can also be embodied in electronic device having digital computer capabilities arranged to run the computer program on the tangible medium or execute the instruction on a computer readable medium.

Computer programs implementing the method of the present embodiments can commonly be distributed to users on a distribution medium such as, but not limited to, CD-ROMs or flash memory media. From the distribution medium, the computer programs can be copied to a hard disk or a similar intermediate storage medium. In some embodiments of the present invention, computer programs implementing the method of the present embodiments can be distributed to users by allowing the user to download the programs from a remote location, via a communication network, e.g., the internet. The computer programs can be run by loading the computer instructions either from their distribution medium or their intermediate storage medium into the execution memory of the computer, configuring the computer to act in accordance with the method of this invention. All these operations are well-known to those skilled in the art of computer systems.

The computational operations of the method of the present embodiments can be executed by a computer, either remote from the subject or near the subject. When the computer is remote from the subject, it can receive the data over a network, such as a telephone network or the Internet. To this end, a local computer can be used to transmit the data to the remote computer. This configuration allows performing the analysis while the subject is at a different location (e.g., at home), and also allows performing simultaneous analyses for multiple subjects in multiple different locations.

The computational operations of the method can also be executed by a cloud computing resource of a cloud computing facility. The cloud computing resource can include a computing server and optionally also a storage server, and can be operated by a cloud computing client as known in the art.

The method according to some embodiments may be used to estimate the severity of a coronaviral infection.

The biological data analyzed by the method contain expression values of a plurality of proteins in a body sample of a subject. In some embodiments the biological data comprises expression values of three or more proteins. In some embodiments the biological data comprises expression values of at least CRP, TRAIL and IP-10.

According to a particular embodiment, the levels of secreted (i.e. soluble) proteins (e.g., TRAIL, CRP and IP-10) are analyzed by the method.

Referring to FIG. 10, the method begins at **310** and continues to **311** at which a distance $d$ between a segment $S_{ROI}$ of a curved line $S$ and a non-curved axis $\pi$ is calculated.

The axis $\pi$ is defined by a direction. The distance between the segment of line S and axis $\pi$ is calculated at a point P over the axis $\pi$. P is defined by a coordinate denoted $\delta$.

It is recognized that in the context of lines, coordinates are numbers that determine positions on axes that are defined by directions. Usually, the terms "coordinate", "axis", and "direction" are interchanged in the literature, but they actually represent different (but related) mathematical objects. For example, in a Cartesian coordinate system, the letter $x$, is used to denote (*i*) the horizontal axis, (*ii*) the rightward direction on this axis, and (*iii*) a point on this axis.

A formulation for obtaining the value of the coordinate $\delta$ is provided below. The formulation when considered generally (namely as a function and not as a value returned by the function) defines the direction along which the axis $\pi$ extends. Below, the direction along which $\pi$ extends is are denoted using the same Greek letters as the coordinate $\delta$, except that the direction is denoted by an underlined Greek letter to indicate that it is a vector quantity. In this notation, the axis $\pi$ extends along direction $\underline{\delta}$.

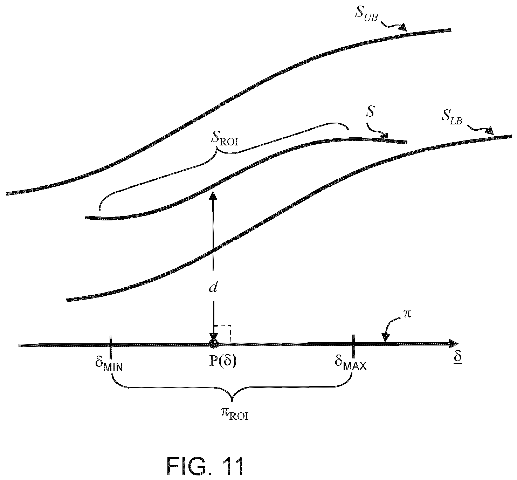

FIG. 11 illustrates the axis $\pi$ along direction $\underline{\delta}$. Also shown is a point P at coordinate $\delta$. On axis $\pi$, along direction $\underline{\delta}$, there is a region-of-interest $\pi_{ROI}$ which is a linear segment of axis $\pi$ spanning from a minimal coordinate $\delta_{MIN}$ to a

maximal coordinate $\delta_{MAX}$ along direction $\underline{\delta}$. The point P is within the region-of-interest $\pi_{ROI}$.

The distance *d,* calculated at **311,** is measured from $S$ to the point P, perpendicularly to $\pi$. The segment $S_{ROI}$ of $S$ is above the region-of-interest $\pi_{ROI}$. In other words, $\pi_{ROI}$ is the projection of $S_{ROI}$ on $\pi$. $S_{ROI}$ is preferably a curved segment of the curve S.

The coordinate is defined by a combination of expression values of the proteins. For example, $\delta$ can be a combinations of the expression values, according to the following equation:

$$\delta = C_0 + C_1 D_1 + C_2 D_2 + ... + \phi$$

where $C_0$, $C_1$, $C_2,...$ are constant and predetermined coefficients, each of the variables $D_1$, $D_2$, ... is an expression value of one of the proteins (*e.g.,* $D_1$ can be the expression value of the TRAIL protein, $D_2$ can be the expression value of the IP-10 protein, and $D_3$ can be the expression value of the CRP protein), and $\phi$ is a function that is nonlinear with respect to at least one of the expression values. $C_0$ is referred to as an offset coefficient.

In some embodiments of the present invention, prior to the calculation of the coordinate $\delta$, each of the expression values is compared to one or more respective thresholds. In these embodiments, the coordinate $\delta$ is defined at least based on the result of this comparison. For example, each of the expression values can be compared to an upper threshold and a lower threshold, wherein when the respective expression value is within a range defined by the lower and upper thresholds, the respective expression value is used for calculating the coordinate $\delta$, when the respective expression value is outside this range but less than the lower threshold, the lower threshold is used for calculating the coordinate $\delta$ instead of the respective expression value, and when the respective expression value is outside this range but more than the upper threshold, the upper threshold is used for calculating the coordinate $\delta$ instead of the respective expression value.

The lower threshold for the TRAIL protein is denoted $TRAIL_{min}$, and the upper threshold for the TRAIL protein is denoted $TRAIL_{max}$. Typical values for $TRAIL_{min}$ are from about 0 to about 20 pg/ml, *e.g.,* about 15 pg/ml, and typical values for $TRAIL_{max}$ are from about 250 to about 350 pg/ml, *e.g.,* about 300 pg/ml.

The lower threshold for the IP-10 protein is denoted $IP10_{min}$, and the upper threshold for the IP-10 protein is denoted $IP10_{max}$. Typical values for $IP10_{min}$ are from about 0 to about 150 pg/ml, *e.g.,* about 100 pg/ml, and typical values for $IP10_{max}$ are from about 5000 to about 7000 pg/ml, *e.g.,* about 6000 pg/ml.

The lower threshold for the CRP protein is denoted $CRP_{min}$, and the upper threshold for the CRP protein is denoted $CRP_{max}$. Typical values for $CRP_{min}$ are from about 0 to about 1.5 mg/L, *e.g.,* about 1 mg/L, and typical values for $CRP_{max}$ are from about 200 to about 300 mg/L, *e.g.,* about 250 mg/L.

The present embodiments also contemplate an operation in which each of the expression values is compared a confidence range of values, wherein when the respective expression value is outside the confidence range, the method issues an error or warning message that the respective expression value is outside the confidence range. A typical confidence range for the TRAIL protein is from about -5 to about 1,000 pg/ml, a typical confidence range for the IP-10 protein is from about -50 to about 30,000 pg/ml, and a typical confidence range for the CRP protein is from about -10 to about 1,000 mg/L. Other confidence ranges are also contemplated.

The function $\phi$ is optional and may be set to zero (or, equivalently, not be used in the calculation of the coordinate $\delta$). When $\phi=0$ the coordinate $\delta$ is a linear combination of the proteins. The nonlinear function $\phi$ can optionally and preferably be expressed as a sum of powers of the expression values, for example, according to the following equation:

$$\phi = \Sigma_i q_i X_i^{\gamma i}$$

where i is a summation index, $q_i$ is a set of coefficients, $X_i \in \{D_1, D_2, ...\}$, and $\gamma_i$ are numerical exponents. Note that the number of terms in $\phi$ does not necessarily equals the number of the proteins, and that two or more terms in the sum may correspond to the same protein, albeit with a different numerical exponent.

A representative example of the offset coefficient $C_0$, suitable for the present embodiments, is from about 0.9-$\kappa$ to about 0.9+$\kappa$, where $\kappa$ is defined as $(\log(p_{max}/(1-p_{max})) - \log(p_{min}/(1-p_{min})))$, where $p_{max}$ is a predetermined parameter which is more than 0.9 and less than 1 (e.g., 0.99), and $p_{min}$ is a predetermined parameter from about 0.01 to about 0.5. For example, when $p_{max}$ is about 0.99 and $p_{min}$ is about 0.01, $C_0$ is from about -8 to about 10, when $p_{max}$ is about 0.99 and $p_{min}$ is about 0.2, $C_0$ is from about -5 to about 6.9, and when $p_{max}$ is about 0.99 and $p_{min}$ is about 0.5, $C_0$ is from about -3.67 to about 5.5. In some embodiments of the present invention $C_0$ is about 0.93.

Herein, "log" means a natural logarithm.

A representative example of the coefficient $C_1$, suitable in embodiments in which $C_1$ is a coefficient of the expression value of the TRAIL protein, is from about -0.04-$\kappa$/$TRAIL_{min}$ ml/pg to about -0.04+$\kappa$/$TRAIL_{min}$ ml/pg, where $\kappa$ and $TRAIL_{min}$ are as indicated above. For example, when $p_{max}$ is about 0.99, $p_{min}$ is about 0.01 and $TRAIL_{min}$ is about

15 pg/ml, $C_1$ is from about -0.65 ml/pg to about 0.57 ml/pg, when $p_{max}$ is about 0.99, $p_{min}$ is about 0.2 and $TRAIL_{min}$ is about 15 ml/ml, $C_1$ is from about -0.44 ml/pg to about 0.36 ml/pg, and when $p_{max}$ is about0.99, $p_{min}$ is about 0.5 and $TRAIL_{min}$ is about 15 ml/ml, $C_1$ is from about -0.35 ml/pg to about 0.27 ml/pg. In some embodiments of the present invention $C_1$ is about -0.041 ml/pg.

A representative example of the coefficient $C_2$, suitable in embodiments in which $C_2$ is a coefficient of the expression value of the IP-10 protein, is from about $0.0006-\kappa/IP10_{min}$ ml/pg to about $0.0006+\kappa/IP10_{min}$ ml/pg, where $\kappa$ and $IP10_{min}$ are as indicated above. For example, when $p_{max}$ is about 0.99, $p_{min}$ is about 0.01 and $IP10_{min}$ is about 100 pg/ml, $C_2$ is from about -0.1 ml/pg to about 0.1 ml/pg, when $p_{max}$ is about 0.99, $p_{min}$ is about 0.2 and $IP10_{min}$ is about 100 pg/ml, $C_2$ is from about -0.06 ml/pg to about 0.06 ml/pg, and when $p_{max}$ is about 0.99, $p_{min}$ is about 0.5 and $IP10_{min}$ is about 100 pg/ml, Cz is from about-0.04 ml/pg to about 0.05 ml/pg. In some embodiments of the present invention $C_2$ is about 0.0006 ml/pg.

A representative example of the coefficient $C_3$, suitable in embodiments in which $C_3$ is a coefficient of the expression value of the CRP protein, is from about $0.003-\kappa/CRP_{min}$ L/mg to about $0.003+\kappa/CRP_{min}$ L/mg, where $p_{max}$, $p_{min}$ and $CRP_{min}$ are as indicated above. For example, when $p_{max}$ is about 0.99, $p_{min}$ is about 0.01 and $CRP_{min}$ is about 1 mg/L, $C_3$ is from about -9.19 L/mg to about 9.19 L/mg, when $p_{max}$ is about 0.99, $p_{min}$ is about 0.2 and $CRP_{min}$ is about 1 mg/L, $C_3$ is from about -5.98 L/mg to about 5.98 L/mg, and when $p_{max}$ is about 0.99, $p_{min}$ is about 0.5 and $CRP_{min}$ is about 1 mg/L, $C_3$ is from about -4.6 L/mg to about 4.6 L/mg. In some embodiments of the present invention $C_3$ is about 0.0025 L/mg.

The boundaries $\delta_{MIN}$, and $\delta_{MAX}$ of $\pi_{ROI}$ preferably correspond to the physiologically possible ranges of the expression values of the proteins, according to a protocol used for measuring those expression values. For example, in measurements performed by the Inventors using an ELISA protocol, typical physiologically possible ranges are from 0 to about 400 ug/ml for CRP, from 0 to about 3000pg/ml for IP-10, and from 0 to about 700 pg/ml for TRAIL. Some subjects may exhibit concentrations that lie outside these ranges. It is appreciated that when the expression values of TRAIL, CRP and IP-10 are measured by other techniques or protocols the values of the boundaries $\delta_{MIN}$, and $\delta_{MAX}$ of $\pi_{ROI}$, and optionally also the coefficients $C_0$, $C_1$, $C_2$, $C_3$, may be different that the above exemplified values.

At least a major part of the segment $S_{ROI}$ of curved line S is between two curved lines referred to below as a lower bound curved line $S_{LB}$ and an upper bound curved line $S_{UB}$.

As used herein "major part of the segment $S_{ROI}$" refers to a part of a smoothed version $S_{ROI}$ whose length is 60% or 70% or 80% or 90% or 95% or 99% of a smoothed version of the length of $S_{ROI}$.

As used herein, "a smooth version of the segment $S_{ROI}$" refers to the segment $S_{ROI}$, excluding regions of $S_{ROI}$ at the vicinity of points at which the Gaussian curvature is above a curvature threshold, which is X times the median curvature of $S_{ROI}$, where X is 1.5 or 2 or 4 or 8.

The following procedure can be employed for the purpose of determining whether the major part of the segment $S_{ROI}$ is between $S_{LB}$ and $S_{UB}$. Firstly, a smoothed version of the segment $S_{ROI}$ is obtained. Secondly, the length Ai of the smoothed version of the segment $S_{ROI}$ is calculated. Thirdly, the length $A_2$ of the part of the smoothed version of the segment $S_{ROI}$ that is between $S_{LB}$ and $S_{UB}$ is calculated. Fourthly, the percentage of $A_2$ relative to Ai is calculated.

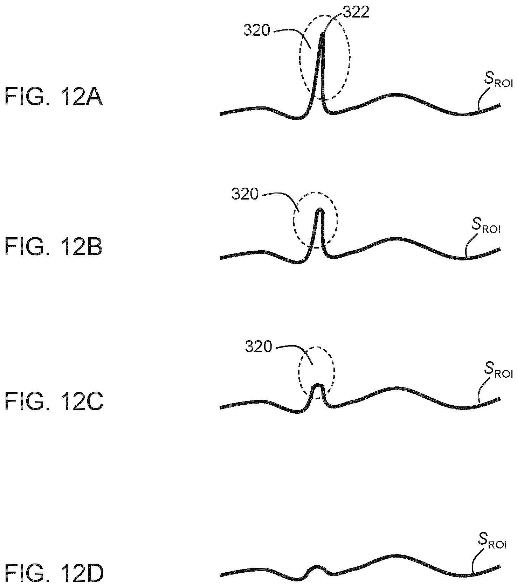

FIGs. 12A-D illustrates a procedure for obtaining the smooth version of $S_{ROI}$. The Gaussian curvature is calculated for a sufficient number of sampled points on $S_{ROI}$. For example, when the line is represented as a set of points, the Gaussian curvature can be calculated for the points in the set. The median of the Gaussian curvature is then obtained, and the curvature threshold is calculated by multiplying the obtained median by the factor X. FIG. 12A illustrates $S_{ROI}$ before the smoothing operation. Marked is a region **320** having one or more points **322** at which the Gaussian curvature is above the curvature threshold. The point or points at which the Gaussian curvature is maximal within region **320** is removed and region **320** is smoothly interpolated, e.g., via polynomial interpolation (FIG. 12B). The removal and interpolation is repeated iteratively (FIG. 12C) until the segment $S_{ROI}$ does not contain regions at which the Gaussian curvature is above the curvature threshold (FIG. 12D).

The lower and upper bound curved lines $S_{LB}$ and $S_{UB}$ can be written in the form:

$$S_{LB} = f(\delta)-\varepsilon_0,$$

$$S_{UB} = f(\delta)+\varepsilon_1$$

where $f(\delta)$ is a classification function of the coordinate $\delta$ (along the direction $\underline{\delta}$) which represents the severity of a coronaviral infection of the subject. In some embodiments of the invention $f(\delta)$ comprises a multiplication between a saturation function $p$ of the coordinate $\delta$, and a saturation function w of the expression value of the CRP protein.

A saturation function, as used herein, is a monotonically increasing function which exhibits a plateau for sufficiently large value of its argument. The plateau is preceded by a segment at which the second derivative of the saturation function changes its sign. At the plateau, the first derivative of the saturation function monotonically decreases for any value of the argument above a predetermined value.

In some embodiments of the present invention the saturation function $p$ of the $\delta$ coordinate is linearly proportional to $1/(1+Exp(-\delta))$. In some embodiments of the present invention $p = 1/(1+Exp(-\delta))$. In these embodiments, setting the coefficients $C_0$, $C_1$, etc., based on the predetermined parameters $p_{min}$ and $p_{max}$ as further detailed hereinabove, ensures that that a change of the coefficient value to the upper limit of the respective range or to the lower limit of the respective range translates to a change in $p$ from $p_{min}$ to $p_{max}$.

In some embodiments of the present invention the saturation function $w$ of the expression value of the CRP protein is linearly proportional to $1/(1+(CRP/CRP_0)^{-h})$, where $CRP_0$ and $h$ are predetermined shift and width parameters. Representative examples of a value for the shift parameter $CRP_0$ is from about 1 mg/L to about 1000 mg/L, or from about 100 mg/L to about 500 mg/L, or from about 200 mg/L to about 300 mg/L, *e.g.,* about 260 mg/L. Representative examples of a value for the width parameter $h$ is from about 2 to about 100, or from about 2 to about 50, or from about 2 to about 10, *e.g.,* about 6. In some embodiments of the present invention $w=1/(1+(CRP/CRP_0)^{-h})$.

The classification function optionally and preferably also includes at least one term that does not depend on $w$ and/or at least one term that does not depend on $p$. In experiments performed by the Inventors it was found that when the classification function is in the form $f(\delta)= p(1-w)+w$, a more accurate classification of the severity of the coronaviral infection is obtained.

In any of the above embodiments each of the parameters $\varepsilon_0$ and $\varepsilon_1$ is less than 0.5 or less than 0.4 or less than 0.3 or less than 0.2 or less than 0.1 or less than 0.05.

Referring again to FIG. 10, the method proceeds to **312** at which a score classifying the severity of a coronaviral infection of the subject is determined based on the distance $d$. For example, the score can be the calculated distance or some proxy thereof, *e.g.,* a value that is proportional to $d$. In some embodiments, the score is defined as Round(100.d), where Round is a function that returns the nearest integer of its argument (*e.g.*, Round(40.1)=40, and Round(40.51)=41).

The method optionally and preferably continues to **313** at which an output indicative of the score is generated. The output can be presented as text, and/or graphically and/or using a color index.

The output can in some embodiments of the present invention be a description of the score, rather than the score itself. As a representative example, which is not to be considered as limiting, when the score ranges from 0 to 100, the method can output an indication that there is a very low likelihood for severe outcome for scores between 0 and about 20, an indication that there is a low likelihood for severe outcome for scores between about 20 and about 40, an indication that there is a moderate likelihood for severe outcome for scores between about 40 and about 80, and an indication that there is a high likelihood for severe outcome for scores between about 80 and about 100.

In some embodiments of the present invention, the subject is treated (**314**) for the coronaviral infection based on the output generated at **313**. For example, when the score is above a predetermined severity threshold, the subject can be treated by , *e.g.*, mechanical ventilation, life support, catheterization, hemofiltration, invasive monitoring, sedation, intensive care admission, surgical intervention, drug of last resort, and the like.

The method ends at **315**.

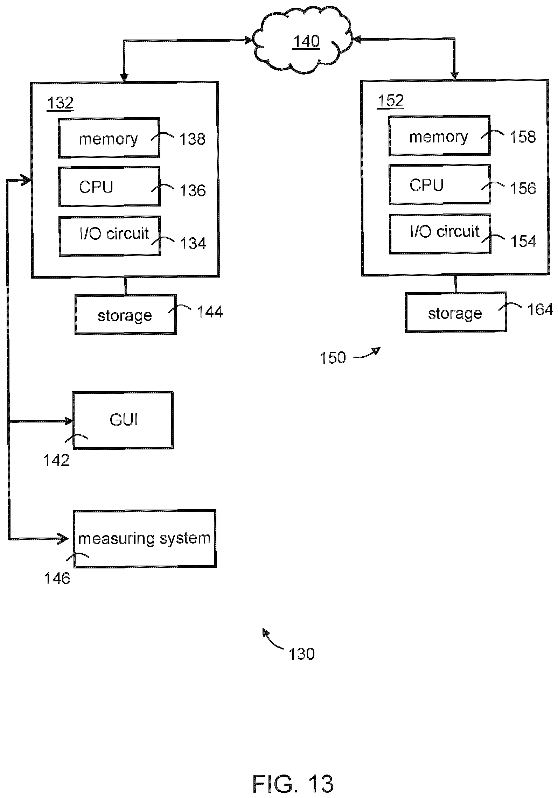

FIG. 13 is a schematic illustration of a client computer **130** having a hardware processor **132,** which typically comprises an input/output (I/O) circuit **134,** a hardware central processing unit (CPU) **136** (*e.g.*, a hardware microprocessor), and a hardware memory **138** which typically includes both volatile memory and non-volatile memory. CPU **136** is in communication with I/O circuit **134** and memory **138**. Client computer **130** preferably comprises a graphical user interface (GUI) **142** in communication with processor **132**. I/O circuit **134** preferably communicates information in appropriately structured form to and from GUI **142**. Also shown is a server computer **150** which can similarly include a hardware processor **152**, an I/O circuit **154**, a hardware CPU **156**, a hardware memory **158**. I/O circuits **134** and **154** of client **130** and server **150** computers can operate as transceivers that communicate information with each other via a wired or wireless communication. For example, client **130** and server **150** computers can communicate via a network **140,** such as a local area network (LAN), a wide area network (WAN) or the Internet. Server computer **150** can be in some embodiments be a part of a cloud computing resource of a cloud computing facility in communication with client computer **130** over the network **140**. Further shown, is a measuring system **146** that is associated with client computer **130,** and that measures the amount of the proteins in the body liquid sample of the subject blood, for example, e.g., using one or more of the techniques described herein.

GUI **142** and processor **132** can be integrated together within the same housing or they can be separate units communicating with each other. Similarly, system **146** and processor **132** can be integrated together within the same housing or they can be separate units communicating with each other.

GUI **142** can optionally and preferably be part of a system including a dedicated CPU and I/O circuits (not shown) to allow GUI **142** to communicate with processor **132**. Processor **132** issues to GUI **142** graphical and textual output generated by CPU **136**. Processor **132** also receives from GUI **142** signals pertaining to control commands generated by GUI **142** in response to user input. GUI **142** can be of any type known in the art, such as, but not limited to, a keyboard and a display, a touch screen, and the like. In some embodiments, GUI **142** is a GUI of a mobile device such as a smartphone, a tablet, a smartwatch and the like. When GUI **142** is a GUI of a mobile device, the CPU circuit of the mobile device can serve as processor **132** and can execute the code instructions pertaining to the method described herein.

Client **130** and server **150** computers can further comprise one or more computer-readable storage media **144, 164,** respectively. Media **144** and **164** are preferably non-transitory storage media storing computer code instructions for executing the method as further detailed herein, and processors **132** and **152** execute these code instructions. The code instructions can be run by loading the respective code instructions into the respective execution memories **138** and **158** of the respective processors **132** and **152**.

Each of storage media **144** and **164** can store program instructions which, when read by the respective processor, cause the processor to receive the expression values of the proteins and to execute the method as described herein. In some embodiments of the present invention, the expression values of the proteins are generated as digital data by system **146** and are transmitted to processor **132** by means of I/O circuit **134**. Processor **132** receives the expression values, calculates the distance between the segment of a curved line and the axis, as further detailed hereinabove, determines the score as further detailed hereinabove and displays the score, for example, on GUI **142**. Alternatively, processor **132** can transmit the expression values over network **140** to server computer **150**. Computer **150** receives the expression values, calculates the distance between the segment of a curved line and the axis, and determines the score as further detailed hereinabove, and transmits the score back to computer **130** over network **140**. Computer **130** receives the score and displays it on GUI **142**.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

**EXAMPLE 1**

*Diagnosing non-symptomatic subjects.*

In the presented analysis, serial measurements of TRAIL, CRP and IP-10 were performed on a non-symptomatic individual who had been in close contact with a virally infected patient. On day 3 the patient developed upper respiratory tract symptoms. TRAIL, CRP and IP-10 levels were shown to increase from basal level prior to symptoms onset. More specifically, a 25% and 50% increase in TRAIL and IP-10 levels, respectively, were found to accurately detect the non-symptomatic individual prior to symptoms onset (Figures 1A-C).

**EXAMPLE 2**

The ability of TRAIL, CRP and IP10 to distinguish between non-infectious subjects from virally infected subjects within 1 day from symptom onset was analyzed. Protein levels were significantly differentially expressed across different viruses (adenovirus; CRP p-value=0.002, IP-10 p-value<0.001, TRAIL p-value<0.001, bocavirus; CRP p-value=0.01, IP-10 p-value=0.007, TRAIL p-value<0.001, coronavirus; CRP p-value=0.01, IP-10 p-value<0.001, TRAIL p-value<0.001, enterovirus; CRP p-value<0.001, IP-10 p-value<0.001, TRAIL p-value<0.001, respiratory syncytial virus; CRP p-value=0.007, IP-10 p-value<0.001, TRAIL p-value<0.001, metapneumovirus; CRP p-value=0.02, IP-10 p-value<0.001, TRAIL p-value<0.001, parainfluenza virus; CRP p-value=0.007, IP-10 p-value<0.001, TRAIL p-value<0.001).

Data derived from the comparison of viral patients tested at time from symptoms <=1 (day 0 or day 1) to non-infectious patients allowed for the estimation of cutoffs for predicting whether a non-symptomatic subject has a virus. The cutoffs are provided in Table 13, herein below.

*Table 13*

| Biomarker | Cutoff |
|---|---|
| TRAIL (pg/ml) | 100 |
| CRP (mg/L) | 5 |
| IP-10 (pg/ml) | 200 |

**EXAMPLE 3**

*Analyzing contagiousness of non-symptomatic subjects.*

In the presented cohort, 81 non-symptomatic individuals were analyzed. Using multiplex PCR applied to nasal swabs, 21 were found to be positive for rhinovirus. TRAIL and IP-10 levels were correlated with viral loads (Figures 2A-B).

Furthermore, TRAIL, IP-10 and CRP were shown to accurately differentiate low vs high viral loads and thus could be used to inform a non-symptomatic individual contagiousness status (Table 14).

**Table 14.** TRAIL, IP-10 and CRP single/triplet's accuracy for differentiating non-symptomatic individuals with high and low viral loads.

| Feature #1 | Feature #2 | Feature #3 | AUC |
|---|---|---|---|
| CRP | | | 0.598 |
| IP-10 | | | 0.893 |
| TRAIL | | | 0.786 |
| CRP | IP-10 | TRAIL | 0.911 |

Pairs of determinants were also tested. CRP + IP10, CRP + TRAIL and IP10 + TRAIL all gave AUCs above 0.85, with the highest being for CRP +IP10.

**EXAMPLE 4**

***Correlation of IP10 levels with patient status***

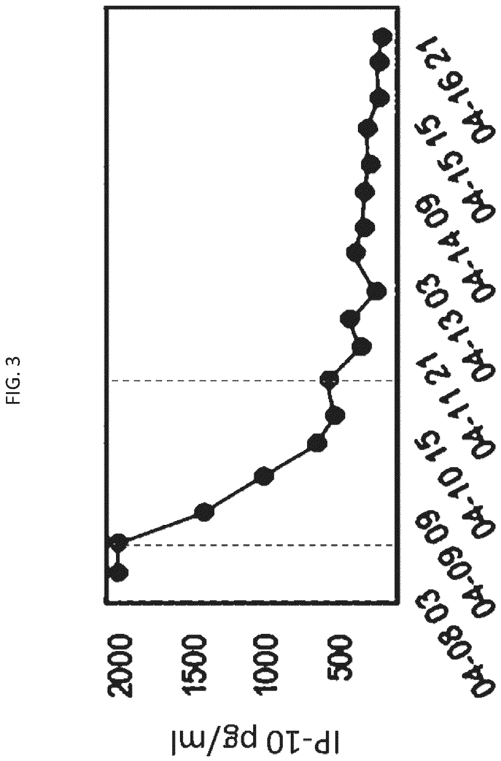

The level of IP10 was measured for a SARS-COV-2 positive male patient having the following comorbidities: Hypothyroidism, Hypercholesterol, Osteoporosis. The patient was diagnosed with Covid 19 on 31/3/2020. The patient was treated with steroid on 9th of April, and the dose was lowered on 11th April.
The results are illustrated in Figure 3.

**EXAMPLE 5**

***Study Design***

The prospective study included a cohort of SARS-CoV-2 positive patients recruited at a COVID-19 dedicated medical center (n = 52). An additional retrospective study was conducted to enable comparison of IP-10 levels across other acute respiratory infections (n = 182) and healthy subjects (n = 98).

***Patient population***

COVID-19 patients

The prospective study included 52 SARS-CoV-2 positive patients recruited at a COVID-19 dedicated medical centre. ARDS and its severity were defined according to the Berlin definition.24

***Patients with other respiratory viruses***

A retrospective analysis was performed on a cohort of adult patients (n = 182) pooled from 2 studies, Curiosity[14] (NCT01917461) and Observer (NCT03011515) who fulfilled all of the following inclusion criteria: meet inclusion/exclusion criteria of original study; detection of at least one of the following viruses (human rhinovirus [HRV], respiratory syncytial virus [RSV], Influenza A [Flu A], Influenza B [Flu B], human coronavirus [HCoV]); and adjudication as viral infection by an expert panel (no bacterial detection).

***Healthy subjects***

A retrospective analysis was performed on a cohort of adult patients (n = 98) from Curiosity[14] (NCT01917461) and Observer (NCT03011515) who were afebrile with no apparent infectious disease.

***Clinical decision support protocol***

An overview of the clinical decision support protocol is provided in Figure 4. In brief, to manage oxygenation, PaO2/FiO2 (P/F, ratio of arterial oxygen partial pressure to fractional inspired oxygen) was monitored twice daily and positive end-expiratory pressure (PEEP) titration was made based on cardiac output measurements when indicated. All patients received high dose N-acetylcysteine and bromohexin inhalations in order to induce mucolysis and prevent sticky secretion and atelectasis. Intra-tracheal injected surfactant was an option as a compassionate therapy for refractory hypoxemic ventilated patients.
Echocardiography and bedside Doppler ultrasound were ordered when pulmonary emboli or deep vein thrombosis were clinically suspected and when D-dimer levels significantly increased. Troponin levels were measured daily and electrocardiogram measurements were performed routinely to detect QT prolongation. All ICU patients received 40 mg twice daily of enoxaparin. D-dimers were measured daily and the enoxaparin dose was adjusted up to 60 mg twice daily when D-dimer levels increased significantly. In case of proven thrombosis (as visualized by imaging), full anti-coagulation therapy was recommended. When minor bleeding occurred, sub-cutaneous heparin was administered.
All ICU patients considered as hyperinflammatory were administered a total tocilizumab dosing of 800 mg, given in 2 separate doses of 400 mg, 12 hours apart. The following parameters were considered as reflective of a hyperinflammatory state: C-reactive protein (CRP) > 90 mg/l, ferritin >500 ng/dl, and IP-10 >1,000 pg/ml. All hyperinflammatory ICU patients were administered methylprednisolone or equivalent dosing of another corticosteroid; basic dosing was 1.5 mg/kg divided into 3 doses. Patients on hemodialysis who were hyper-inflamed had access to cytokine removal hemodialysis (Theranova dialyzer).
The anti-viral therapy was based on combinations of hydroxychloroquine, azithromycin, zinc, and lopinavir/ritonavir at the discretion of the infectious disease consultant and was discussed daily. Patients who did not show clinical improvement were given convalescent plasma divided into two doses as a compassionate therapy. Compassionate Remdesivir was given to only one patient for a total of 7 days (200 mg loading dose followed by 100 mg maintenance dose from day 2 onwards) and was stopped as a result of elevated liver function tests.

### Laboratory procedures

Blood was collected serially from 52 patients as part of routine blood draw into serum separating tubes (SST). Serum was separated within 2 hours of collection and the fresh serum samples were measured on the MeMed Key™ (MeMed, Israel) rapid immunoassay platform that provides measurements of 3 immune system proteins (IP-10, CRP, and TNF-related apoptosis inducing ligand [TRAIL]), in 15 minutes, based on MeMed BV™ (MeMed, Israel) testing cartridges. Any unused serum was frozen at -20°C for up to one week and then transferred for storage at -80°C.

Frozen serum was used to determine retrospectively blood concentrations of IL-6. Measurements were performed using 'Quantikine human IL-6' (R&D systems, MN, USA) ELISA assay kits. Due to high IL-6 concentrations, each serum sample was diluted 1:4 and 1:40 with RD6F calibrator diluent supplied in the kits. Results were averaged when the difference in concentration between the 2 dilutions was <20%, otherwise the dilution in the linear range of the calibration curve was taken. Measurements were performed using an Absorbance Microplate Reader Sunrise and Magellan program (Tecan life science Grödig, Austria).

SARS-CoV-2 positivity was determined by RT-PCR (Seegene Inc, South Korea).

### Statistical analysis

Continuous variables are reported as median (interquartile range). Values for 2 related variables were compared using Mann-Whitney-U test (for continuous values) and Fisher's Exact test (for discrete values). Steroid dosage was converted to methylprednisolone equivalent dosage, using MedCalc's Steroid Conversion Calculator.25. The analysis was performed using Python 3.7.6.

## RESULTS

### Patient population

Overall, 502 serial blood draws were sampled from 52 SARS-CoV-2 patients that were admitted to the COVID-19 dedicated medical centre between 7th April 2020 and 10th May 2020 and included in the cohort study, with 12 admitted to the ICU. The median age of the 52 patients was 69, 69% were male, 23% were ventilated, 4 died. There was low in-ICU mortality (1 death, 8%) and non-pulmonary organ failure (0%); the 90-day mortality for ICU patients was 25% (3 deaths). The most common comorbidities were diabetes (42%) and hypertension (46%) (Table 15). Among the 12 ICU patients, 10 needed invasive mechanical ventilation and exhibited a pulmonary status compatible with severe ARDS (Table 16). Of these, 7 were weaned during their ICU stay and transferred to a rehabilitation center, 1 was transferred to a respiratory rehabilitation center (Patient #5) and 2 (Patients #3 and #9) were transferred to the internal medicine department, where patient #3 died. 8 patients developed secondary bacterial infection (Table 16).

## Table 15

| | All (n=52) | Non-ICU (n=40) | ICU (n=12) | p-value |
|---|---|---|---|---|
| General | | | | |
| Age | 68.6 (16.7) | 66.4 (20.0) | 70.2 (13.2) | 0.313 |
| Female | 16 (31.0%) | 15 (38.0%) | 1 (8.0%) | 0.078 |
| Smoking Status | | | | |
| Smoker | 3 (6.0%) | 3 (8.0%) | 0 (0.0%) | 1 |
| Past Smoker | 3 (6.0%) | 3 (8.0%) | 0 (0.0%) | 1 |
| Other | | | | |
| Death | 4 (8.0%) | 1 (2.0%) | 3 (25.0%) | 0.034 |
| Discharged | 51 (98.0%) | 39 (98.0%) | 12 (100.0%) | 1 |
| Highest Temp | 37.4 (1.2) | 37.3 (0.9) | 38.3 (0.9) | <0.001 |
| Duration of Hospital Stay | 11.0 (13.5) | 9.0 (7.5) | 26.5 (12.2) | <0.001 |
| Time between PCR Positive and BV Test (days) | 4.5 (6.0) | 3.0 (5.0) | 8.0 (8.8) | 0.003 |
| ICU | 12 (23.0%) | 0 (0.0%) | 12 (100.0%) | <0.001 |
| Ventilated | 12 (23.0%) | 2 (5.0%) | 10 (83.0%) | <0.001 |
| Time between Symptoms and BV Test (days) | 7.0 (10.2) | 6.0 (10.0) | 14.0 (7.0) | 0.063 |
| Time between Symptoms and ICU Admission | 8.0 (4.0) | | 8.0 (4.0) | |
| Time between Symptoms and PCR Positive | 2.5 (5.0) | 2.0 (4.5) | 4.0 (5.2) | 0.343 |
| Time on Ventilation (days) | 0.0 (0.0) | 0.0 (0.0) | 15.0 (11.0) | <0.001 |
| Time between PCR Positive and ICU Admission | 4.5 (2.2) | | 4.5 (2.2) | |
| Time between PCR Positive and PCR Negative | 19.0 (17.2) | 8.5 (10.0) | 26.5 (10.0) | 0.015 |
| Co-infection | | | | |
| Bacterial Infection | 10 (19.0%) | 2 (5.0%) | 8 (67.0%) | <0.001 |

| | All (n=52) | Non-ICU (n=40) | ICU (n=12) | p-value |
|---|---|---|---|---|
| Co-infection | 10 (19.0%) | 2 (5.0%) | 8 (67.0%) | <0.001 |
| Fungal Infection | 4 (8.0%) | 0 (0.0%) | 4 (33.0%) | 0.002 |
| Conditions | | | | |
| Diabetes | 22 (42.0%) | 16 (40.0%) | 6 (50.0%) | 0.74 |
| Cardiovascular Disease | 9 (17.0%) | 7 (18.0%) | 2 (17.0%) | 1 |
| Cerebrovascular disease | 3 (6.0%) | 3 (8.0%) | 0 (0.0%) | 1 |
| Chronic Lung Disease | 7 (13.0%) | 6 (15.0%) | 1 (8.0%) | 1 |
| Chronic renal disease | 6 (12.0%) | 4 (10.0%) | 2 (17.0%) | 0.612 |
| Dyslipidemia | 9 (17.0%) | 6 (15.0%) | 3 (25.0%) | 0.415 |
| Hypertension | 24 (46.0%) | 18 (45.0%) | 6 (50.0%) | 1 |
| Hypothyroidism | 6 (12.0%) | 2 (5.0%) | 4 (33.0%) | 0.021 |
| Malignancy | 5 (10.0%) | 2 (5.0%) | 3 (25.0%) | 0.074 |
| Obesity (BMI>30 kg/m2) | 9 (17.0%) | 8 (20.0%) | 1 (8.0%) | 0.666 |
| Immunodeficiency | 1 (2.0%) | 1 (2.0%) | 0 (0.0%) | 1 |
| Symptoms | | | | |
| Sore throat | 1 (2.0%) | 1 (2.0%) | 0 (0.0%) | 1 |
| Vomiting | 2 (4.0%) | 1 (2.0%) | 1 (8.0%) | 0.412 |
| Urinary Complaints | 1 (2.0%) | 1 (2.0%) | 0 (0.0%) | 1 |
| Sputum production | 1 (2.0%) | 1 (2.0%) | 0 (0.0%) | 1 |
| Weakness | 12 (23.0%) | 11 (28.0%) | 1 (8.0%) | 0.253 |
| Rhinorrhea | 1 (2.0%) | 1 (2.0%) | 0 (0.0%) | 1 |
| Lethargy | 2 (4.0%) | 2 (5.0%) | 0 (0.0%) | 1 |
| Agitation | 1 (2.0%) | 1 (2.0%) | 0 (0.0%) | 1 |
| Chest pain | 2 (4.0%) | 2 (5.0%) | 0 (0.0%) | 1 |
| Confusion | 2 (4.0%) | 2 (5.0%) | 0 (0.0%) | 1 |
| Chills | 1 (2.0%) | 0 (0.0%) | 1 (8.0%) | 0.231 |
| Diarrhea | 6 (12.0%) | 6 (15.0%) | 0 (0.0%) | 0.316 |
| Dyspnea | 23 (44.0%) | 17 (42.0%) | 6 (50.0%) | 0.746 |
| Fatigue | 3 (6.0%) | 2 (5.0%) | 1 (8.0%) | 0.553 |
| Fever | 29 (56.0%) | 18 (45.0%) | 11 (92.0%) | 0.007 |
| Headache | 5 (10.0%) | 4 (10.0%) | 1 (8.0%) | 1 |
| Loss of Taste and Smell | 5 (10.0%) | 5 (12.0%) | 0 (0.0%) | 0.578 |
| Myalgia | 4 (8.0%) | 4 (10.0%) | 0 (0.0%) | 0.562 |
| Nausea | 3 (6.0%) | 3 (8.0%) | 0 (0.0%) | 1 |
| Cough | 26 (50.0%) | 17 (42.0%) | 9 (75.0%) | 0.097 |
| Markers | | | | |
| AST (GOT) (u/l) max | 37.0 (38.8) | 28.4 (25.5) | 86.0 (91.2) | 0 |
| ALT (GPT) (u/l) max | 34.2 (70.1) | 28.6 (27.0) | 120.1 (147.1) | 0 |
| Albumin (g/dl) max | 3.8 (0.5) | 3.8 (0.5) | 3.6 (0.3) | 0.113 |
| INR max | 1.1 (0.2) | 1.1 (0.2) | 1.2 (0.2) | 0.037 |
| Glucose (mg/dl) max | 153.5 (129.6) | 126.4 (85.1) | 247.0 (76.9) | <0.001 |
| LDH (u/l) max | 610.0 (347.5) | 500.0 (244.5) | 912.5 (263.8) | <0.001 |
| Lymph.abs (K/micl) max | 1.3 (0.8) | 1.2 (0.6) | 2.0 (3.9) | 0.03 |
| Lymph.abs (K/micl) min | 0.8 (0.7) | 0.8 (0.7) | 0.4 (0.4) | 0.003 |
| Neu.abs (K/micl) max | 5.6 (5.6) | 5.0 (3.0) | 13.8 (6.2) | <0.001 |
| Neu.abs (K/micl) min | 3.1 (1.9) | 3.1 (2.4) | 3.0 (1.1) | 0.214 |
| PLT (10^3) max | 375.0 (193.8) | 319.0 (219.2) | 408.5 (71.5) | 0.062 |
| PLT (10^3) min | 186.5 (111.8) | 228.0 (123.5) | 139.5 (49.8) | 0.001 |
| PaO2/FiO2 max | 284.0 (163.5) | 166.0 (0.0) | 313.5 (153.0) | <0.001 |
| Total protein (g/dl) max | 7.0 (0.7) | 7.0 (0.6) | 7.0 (0.8) | 0.136 |
| PaO2/FiO2 min | 83.0 (56.5) | 141.0 (0.0) | 80.5 (42.0) | <0.001 |
| Troponin max | 19.5 (44.5) | 11.5 (18.5) | 69.5 (55.5) | <0.001 |
| Urea max | 46.3 (40.6) | 37.2 (27.5) | 105.0 (66.9) | <0.001 |
| Ferritin min | 283.0 (365.4) | 277.2 (421.7) | 330.2 (216.8) | 0.368 |
| Ferritin max | 595.0 | 462.0 (459.2) | 1691.5 (916.8) | <0.001 |
| D-Dimer max | 1319.0 | 1140.0 | 7235.5 | <0.001 |
| Creatinine (mg/dl) max | 1.0 (0.4) | 0.9 (0.4) | 1.0 (0.5) | 0.086 |
| Bili total (mg/dl) max | 0.5 (0.5) | 0.5 (0.4) | 0.9 (0.5) | 0.001 |
| CRP min (mg/l) | 19.6 (56.2) | 29.9 (67.4) | 2.2 (3.8) | <0.001 |
| Median CRP (mg/l) | 70.9 (85.4) | 77.8 (70.1) | 36.0 (166.8) | 0.5 |

|  | All (n=52) | Non-ICU (n=40) | ICU (n=12) | p-value |
|---|---|---|---|---|
| CRP max (mg/l) | 97.9 (122.8) | 95.0 (92.1) | 158.4 (172.6) | 0.04 |
| Scores |  |  |  |  |
| COVID-19 Severity | 26 (50.0%) | 14 (35.0%) | 12 (100.0%) | <0.001 |
| qSOFA (on admission) | 0.0 (1.0) | 0.0 (1.0) | 1.0 (1.2) | 0.003 |
| Treatments |  |  |  |  |
| Solumedrol | 12 (23.0%) | 1 (2.0%) | 11 (92.0%) | <0.001 |
| Hydrocortisone | 10 (19.0%) | 4 (10.0%) | 6 (50.0%) | 0.006 |
| Systemic Steroids (IV/PO) | 22 (42.0%) | 10 (25.0%) | 12 (100.0%) | <0.001 |
| Renal Replacement Therapy | 2 (4.0%) | 1 (2.0%) | 1 (8.0%) | 0.412 |
| Remdesivir | 1 (2.0%) | 0 (0.0%) | 1 (8.0%) | 0.231 |
| Prednisone | 6 (12.0%) | 6 (15.0%) | 0 (0.0%) | 0.316 |
| Nitric Oxide | 5 (10.0%) | 0 (0.0%) | 5 (42.0%) | <0.001 |
| ECMO | 1 (2.0%) | 0 (0.0%) | 1 (8.0%) | 0.231 |
| Vasopressors | 11 (21.0%) | 2 (5.0%) | 9 (75.0%) | <0.001 |
| Convalescent Plasma | 7 (13.0%) | 3 (8.0%) | 4 (33.0%) | 0.041 |
| Azithromycin | 35 (67.0%) | 24 (60.0%) | 11 (92.0%) | 0.076 |
| Antiviral Lopinavir/Ritonavir | 7 (13.0%) | 1 (2.0%) | 6 (50.0%) | <0.001 |
| Antiviral Hydroxychloroquine plus Azithromycin | 33 (63.0%) | 22 (55.0%) | 11 (92.0%) | 0.037 |
| Antiviral Hydroxychloroquine | 36 (69.0%) | 24 (60.0%) | 12 (100.0%) | 0.01 |
| Antibiotics | 30 (58.0%) | 19 (48.0%) | 11 (92.0%) | 0.008 |
| Tocilizumab | 13 (25.0%) | 3 (8.0%) | 10 (83.0%) | <0.001 |
| Days under Systemic Steroids | 0.0 (6.2) | 0.0 (0.2) | 7.5 (7.5) | <0.001 |

BMI, Body mass index; AST, Aspartate transaminase; AL, Alanine transaminase; LDH, Lactate dehydrogenase; Lymph. Abs, Absolute lymphocytes; Neu. Abs, Absolute neutrophils; PLT, platelets; PaO2/FiO2, Ratio of arterial oxygen partial pressure to fractional inspired oxygen; ECMO, Extracorporeal membrane oxygenation; qSOFA, quick sequential organ failure assessment; TRAIL, TNF-related apoptosis inducing ligand; IP-10, interferon-γ induced protein 10 (also known as CXCL-10); CRP, C-reactive protein.

*Table 16*

| | | ICU patient # | 1 | 2 | 3 | 4 | 5 | 6 | 7 | 8 | 9 | 11 | 18 | 38 |
|---|---|---|---|---|---|---|---|---|---|---|---|---|---|---|
| ICU patient status | | Age | 48 | 69 | 76 | 63 | 61 | 46 | 71 | 76 | 68 | 76 | 73 | 84 |
| | | Sex | Ma | Ma | Ma | Ma | Ma | Ma | Ma | Ma | Ma | Fem | Ma | Ma |
| | | Hypertension | no | no | yes | no | yes | no | no | no | yes | yes | yes | yes |
| | | Diabetes | no | no | yes | no | yes | yes | no | no | yes | yes | no | yes |
| | | Days in ICU | 21 | 27 | 17 | 16 | 19 | 18 | 38 | 35 | 14 | 21 | 3 | 4 |
| | | Secondary bacterial | no | yes | yes | yes | no | yes | yes | yes | yes | yes | no | no |
| | | Mortality | no | no | yes | no | no | no | no | yes | no | yes | no | no |
| Improve 02 delivery | Guidance tools | Echocardiography | yes | no | yes | no | no | yes | yes | yes | no | yes | no | no |
| | | D-Dimer > 10,000 | yes | no | yes | no | no | no | yes | yes | yes | yes | no | no |
| | | US/CT | no | no | no | no | no | no | yes | no | no | no | yes | no |
| | | Troponin > 14 ng/l | yes | yes | yes | yes | yes | yes | yes | yes | yes | yes | yes | yes |
| | Intervention | Nitric Oxide | yes | no | yes | no | no | yes | no | yes | no | yes | no | no |
| | | Prone | yes | yes | yes | no | no | yes | yes | yes | no | yes | no | no |
| | | Invasive mechanical | yes | yes | yes | yes | yes | yes | yes | yes | yes | yes | no | no |
| | | Fluids | yes | yes | no | yes | yes | yes | yes | yes | yes | yes | yes | yes |
| | | Vasopressor therapy | no | yes | yes | yes | yes | yes | yes | yes | yes | yes | no | no |
| | | ECMO | yes | no | no | no | no | no | no | no | no | no | no | no |
| | | Enoxaparin | yes | yes | yes | yes | yes | yes | yes | yes | yes | yes | yes | yes |
| | | Heparin | yes | yes | no | yes | no | yes | yes | yes | yes | yes | no | no |
| | | TPA | no | no | yes | no | no | no | no | no | no | no | no | no |
| | | Ferritin > 500 | yes | yes | yes | yes | yes | yes | yes | yes | yes | yes | yes | yes |

| Control inflammation | Guidance tools | CRP > 90 mg/l* | no | yes | yes | no | no | no | yes | yes | yes | yes | yes | yes |
| | | IP-10 > 1,000 | no | yes | no | yes | yes | no | no | yes | yes | yes | yes | yes |
| | Intervention | Tocilizumab | no | yes | yes | yes | yes | yes | yes | yes | yes | yes | no | yes |
| | | Corticosteroids | yes | yes | yes | yes | yes | yes | yes | yes | yes | yes | yes | yes |
| Viral clearance | Guidance tools | PCR (viral load)** | yes | yes | no | no | yes | yes | yes | yes | yes | no | yes | no |
| | | TRAIL level < 25 | no | yes | yes | yes | no | no | no | yes | no | yes | no | yes |
| | Intervention | Zinc | no | no | yes | no | no | yes | yes | no | yes | no | no | no |
| | | Lopinavir/Ritonavir | no | yes | no | yes | yes | no | no | yes | no | yes | yes | no |
| | | Remdesivir | no | no | no | no | no | no | no | no | no | yes | no | no |
| | | Hydroxychloroquine | yes | yes | yes | yes | yes | yes | yes | yes | yes | yes | yes | yes |
| | | Azithromycin | no | yes | yes | yes | yes | yes | yes | yes | yes | yes | yes | yes |
| | | Convalescent Plasma | no | yes | no | no | no | no | no | yes | no | yes | no | yes |

ICU, Intensive care unit; US, Ultrasound; CT, Computed tomography; ECMO, Extracorporeal membrane oxygenation; TPA, Tissue plasminogen activator; PCR, polymerase chain reaction.

### IP-10 levels indicate hyperinflammation and associate with mortality

A dynamic clinical decision support protocol that includes IP-10 was employed for managing the SARS-CoV-2 patients admitted to the COVID-19 dedicated medical center (Figure 4). Based on an evolving understanding of the complex host-pathogen interaction during disease progression, the goals of this protocol were to improve oxygen delivery and control inflammation without compromising viral clearance.

To assess IP-10 levels in SARS-CoV-2 infection as compared to other viral infections and healthy subjects, a retrospective analysis was performed that included cohorts from previous studies.

The data shows that IP-10 levels are significantly higher in viral infections associated with pulmonary pathology. IP-10 levels in the non-ICU (n = 40) and ICU (n = 12) COVID-19 patients and IP-10 levels in healthy subjects (n = 98) and virally infected patients recruited in Curiosity14 (NCT01917461) and Observer (NCT03011515) studies (n = 182) with PCR-confirmed viral infections including human rhinovirus (HRV), respiratory syncytial virus (RSV), influenza (Flu), and human coronavirus (HCoV). For the COVID-19 patients, IP-10 was measured using MeMed Key™ at multiple time points and the first IP-10 measurement is shown.

Focusing on the prospective cohort of 52 SARS-CoV-2 positive patients, IP-10 >1,000 pg/ml correlated with increased COVID-19 severity score5 (p<0.01) and ICU admission (p<0.05; Table 17).

*Table 17*

| | 0<IP-10<=1000 | IP-10>1000 | p-value |
|---|---|---|---|
| **General** | | | |
| Age | 68.8 (21.5) | 68.5 (14.2) | 0.388 |
| Female | 12 (36.0%) | 4 (21.0%) | 0.353 |
| **Smoking Status** | | | |
| Smoker | 1 (3.0%) | 2 (11.0%) | 0.546 |
| Past Smoker | 2 (6.0%) | 1 (5.0%) | 1 |

|  | 0<IP-10<=1000 | IP-10>1000 | p-value |
|---|---|---|---|
| **Other** | | | |
| Death | 2 (6.0%) | 2 (11.0%) | 0.617 |
| Discharged | 32 (97.0%) | 19 (100.0%) | 1 |
| Highest Temp | 37.2 (1.0) | 38.1 (1.3) | 0.001 |
| Duration of Hospital Stay | 9.0 (13.8) | 12.0 (15.0) | 0.052 |
| Time between PCR Positive and BV Test | 5.0 (6.0) | 3.0 (5.0) | 0.051 |
| ICU | 4 (12.0%) | 8 (42.0%) | 0.019 |
| Ventilated | 6 (18.0%) | 6 (32.0%) | 0.317 |
| Time between Symptoms and BV Test | 7.0 (12.0) | 7.0 (8.0) | 0.199 |
| Time between Symptoms and ICU | 8.0 (2.8) | 8.5 (5.5) | 0.244 |
| Time between Symptoms and PCR Positive | 2.0 (3.0) | 4.0 (4.5) | 0.255 |
| Time on Ventilation (days) | 0.0 (0.0) | 0.0 (12.5) | 0.174 |
| Time between PCR Positive and ICU | 4.0 (2.2) | 4.5 (1.8) | 0.265 |
| Time between PCR Positive and PCR | 18.0 (11.8) | 25.0 (13.2) | 0.112 |
| **Co-infection** | | | |
| Bacterial Infection | 4 (12.0%) | 6 (32.0%) | 0.142 |
| Co-infection | 4 (12.0%) | 6 (32.0%) | 0.142 |
| Fungal Infection | 2 (6.0%) | 2 (11.0%) | 0.617 |
| **Conditions** | | | |
| Diabetes | 13 (39.0%) | 9 (47.0%) | 0.771 |
| Cardiovascular Disease | 7 (21.0%) | 2 (11.0%) | 0.458 |
| Cerebrovascular disease | 3 (9.0%) | 0 (0.0%) | 0.291 |
| Chronic Lung Disease | 3 (9.0%) | 4 (21.0%) | 0.4 |
| Chronic renal disease | 4 (12.0%) | 2 (11.0%) | 1 |
| Dyslipidemia | 4 (12.0%) | 5 (26.0%) | 0.26 |
| Hypertension | 11 (33.0%) | 13 (68.0%) | 0.021 |
| Hypothyroidism | 2 (6.0%) | 4 (21.0%) | 0.175 |
| Malignancy | 1 (3.0%) | 4 (21.0%) | 0.054 |
| Obesity (BMI>30 kg/m2) | 7 (21.0%) | 2 (11.0%) | 0.458 |
| Immunodeficiency | 1 (3.0%) | 0 (0.0%) | 1 |
| **Symptoms** | | | |
| Sore throat | 1 (3.0%) | 0 (0.0%) | 1 |
| Vomiting | 0 (0.0%) | 2 (11.0%) | 0.129 |
| Urinary Complaints | 1 (3.0%) | 0 (0.0%) | 1 |
| Sputum production | 1 (3.0%) | 0 (0.0%) | 1 |
| Weakness | 8 (24.0%) | 4 (21.0%) | 1 |
| Rhinorrhea | 1 (3.0%) | 0 (0.0%) | 1 |
| Lethargy | 2 (6.0%) | 0 (0.0%) | 0.527 |
| Agitation | 0 (0.0%) | 1 (5.0%) | 0.365 |
| Chest pain | 2 (6.0%) | 0 (0.0%) | 0.527 |
| Confusion | 2 (6.0%) | 0 (0.0%) | 0.527 |
| Chills | 1 (3.0%) | 0 (0.0%) | 1 |
| Diarrhea | 4 (12.0%) | 2 (11.0%) | 1 |
| Dyspnea | 12 (36.0%) | 11 (58.0%) | 0.157 |
| Fatigue | 1 (3.0%) | 2 (11.0%) | 0.546 |
| Fever | 16 (48.0%) | 13 (68.0%) | 0.247 |
| Headache | 4 (12.0%) | 1 (5.0%) | 0.641 |
| Loss of Taste and Smell | 4 (12.0%) | 1 (5.0%) | 0.641 |
| Myalgia | 4 (12.0%) | 0 (0.0%) | 0.284 |
| Nausea | 2 (6.0%) | 1 (5.0%) | 1 |
| Cough | 15 (45.0%) | 11 (58.0%) | 0.565 |
| **Markers** | | | |
| AST (GOT) (u/l) max | 26.6 (35.2) | 55.7 (37.9) | 0.005 |
| ALT (GPT) (u/l) max | 28.6 (46.1) | 63.4 (85.1) | 0.017 |
| Albumin (g/dl) max | 3.8 (0.6) | 3.7 (0.3) | 0.082 |
| INR max | 1.1 (0.2) | 1.1 (0.3) | 0.343 |
| Glucose (mg/dl) max | 131.2 (110.8) | 184.4 (150.3) | 0.017 |
| LDH (u/l) max | 491.0 (312.8) | 689.0 (266.0) | 0.011 |
| Lymph.abs (K/micl) max | 1.3 (0.8) | 1.3 (0.9) | 0.402 |

| | 0<IP-10<=1000 | IP-10>1000 | p-value |
|---|---|---|---|
| Lymph.abs (K/micl) min | 0.9 (0.7) | 0.5 (0.4) | 0.004 |
| Neu.abs (K/micl) max | 5.1 (3.8) | 8.0 (8.5) | 0.014 |
| Neu.abs (K/micl) min | 3.7 (2.5) | 2.8 (1.5) | 0.045 |
| PLT (10^3) max | 374.0 (202.0) | 375.0 (158.5) | 0.421 |
| PLT (10^3) min | 229.0 (129.0) | 162.0 (55.0) | 0.013 |
| PaO2/FiO2 max | 238.0 (84.0) | 344.5 (136.5) | 0.388 |
| Total protein (g/dl) max | 7.0 (0.7) | 7.0 (0.7) | 0.406 |
| PaO2/FiO2 min | 83.0 (63.0) | 89.5 (46.5) | 0.44 |
| Troponin max | 12.0 (33.0) | 25.0 (40.0) | 0.059 |
| Urea max | 34.6 (38.0) | 58.0 (57.9) | 0.002 |
| Ferritin min | 281.1 (338.8) | 313.3 (365.0) | 0.202 |
| Ferritin max | 449.4 (697.1) | 901.0 (1147.6) | 0.005 |
| D-Dimer max | 1173.5 (2575.2) | 2175.0 (6061.5) | 0.055 |
| Creatinine (mg/dl) max | 0.9 (0.4) | 1.0 (0.4) | 0.008 |
| Bili total (mg/dl) max | 0.4 (0.4) | 0.8 (0.6) | 0.018 |
| CRP min (mg/l) | 20.3 (54.2) | 19.0 (52.2) | 0.458 |
| Median CRP (mg/l) | 38.4 (70.4) | 107.4 (88.8) | 0 |
| CRP max (mg/l) | 66.1 (82.3) | 169.5 (109.0) | 0 |
| **Scores** | | | |
| COVID-19 Severity | 11 (33.0%) | 15 (79.0%) | 0.003 |
| qSOFA (on admission) | 0.0 (1.0) | 1.0 (1.0) | 0.064 |
| **Treatments** | | | |
| Solumedrol | 4 (12.0%) | 8 (42.0%) | 0.019 |
| Hydrocortisone | 5 (15.0%) | 5 (26.0%) | 0.467 |
| Systemic Steroids (IV/PO) | 10 (30.0%) | 12 (63.0%) | 0.04 |
| Renal Replacement Therapy | 1 (3.0%) | 1 (5.0%) | 1 |
| Remdesivir | 0 (0.0%) | 1 (5.0%) | 0.365 |
| Prednisone | 3 (9.0%) | 3 (16.0%) | 0.656 |
| Nitric Oxide | 2 (6.0%) | 3 (16.0%) | 0.342 |
| ECMO | 1 (3.0%) | 0 (0.0%) | 1 |
| Vasopressors | 5 (15.0%) | 6 (32.0%) | 0.181 |
| Convalescent Plasma | 1 (3.0%) | 6 (32.0%) | 0.007 |
| Azithromycin | 16 (48.0%) | 19 (100.0%) | >0.001 |
| Antiviral Lopinavir/Ritonavir | 0 (0.0%) | 7 (37.0%) | >0.001 |
| Antiviral Hydroxychloroquine plus | 14 (42.0%) | 19 (100.0%) | >0.001 |
| Antiviral Hydroxychloroquine | 17 (52.0%) | 19 (100.0%) | >0.001 |
| Antibiotics | 15 (45.0%) | 15 (79.0%) | 0.023 |
| Tocilizumab | 5 (15.0%) | 8 (42.0%) | 0.047 |
| Days under Systemic Steroids | 0.0 (3.0) | 5.0 (10.5) | 0.007 |
| **First BV test** | | | |
| TRAIL (pg/ml) | 58.2 (22.5) | 50.7 (52.7) | 0.131 |
| CRP (mg/l) | 38.4 (70.4) | 107.4 (88.8) | >0.001 |
| IP-10 (pg/ml) | 333.2 (419.5) | 1984.5 (1525.6) | >0.001 |

BMI, Body mass index; AST, Aspartate transaminase; AL, Alanine transaminase; LDH, Lactate dehydrogenase; Lymph. Abs, Absolute lymphocytes; Neu. Abs, Absolute neutrophils; PLT, platelets; PaO2/FiO2, Ratio of arterial oxygen partial pressure to fractional inspired oxygen; ECMO, Extracorporeal membrane oxygenation; qSOFA, quick sequential organ failure assessment; TRAIL, TNF-related apoptosis inducing ligand; IP-10, interferon-$\gamma$ induced protein 10 (also known as CXCL-10); CRP, C-reactive protein.

Among the 12 ICU patients, 11 were considered hyperinflammatory, and 10 received tocilizumab and methylprednisolone; patient #18 was admitted to the ICU after pneumothorax surgery and was not administered tocilizumab. For COVID-19 patients (n = 52) and the sub-group of ICU patients (n = 12), the number of days with IP-10 > 1,000 pg/ml was associated with mortality (Tables 18 and 19).

**Table 18**

| | Death | Days of IP-10>=1000pg/ml |
|---|---|---|
| 1 - 3140- EY | FALSE | 0 |

(continued)

|  | Death | Days of IP-10>=1000pg/ml |
|---|---|---|
| 2 - 5011- IG | FALSE | 2 |
| 4 - 5449- BTR | FALSE | 2 |
| 5 - 5557- MP | FALSE | 1 |
| 6 - 2726- SEBB | FALSE | 0 |
| 7 - 4267- HH | FALSE | 0 |
| 8 - 1493- MR | TRUE | 6 |
| 9 - 5065- YK | FALSE | 3 |
| 10 - 8752- YHT | FALSE | 10 |
| 11 - 4108- SG | TRUE | 9 |
| 12 - 3216- SS | FALSE | 0 |
| 13 - 1434- HD | FALSE | 0 |
| 14 - 5051- MM | FALSE | 1 |
| 15 - 3050- BP | FALSE | 0 |
| 16 - 1202- HT | FALSE | 0 |
| 17 - 2000- MBG | FALSE | 0 |
| 18 - 6494- NBT | FALSE | 1 |
| 19 - 3091- RM | FALSE | 0 |
| 20 - 5921- RA | FALSE | 1 |
| 21 - 5239- YAF | FALSE | 3 |
| 22 - 5397- SOC | FALSE | 0 |
| 23 - 3092- MSA | FALSE | 0 |
| 24 - 5388- GZM | FALSE | 0 |
| 25 - 4604- STFR | FALSE | 0 |
| 26 - 2964- ARH | FALSE | 0 |
| 27 - 5304- NAG | FALSE | 1 |
| 28 - 3422- SHY | FALSE | 3 |
| 29 - 6044- ZAPY | FALSE | 0 |
| 30 - 5488- DAZ | FALSE | 2 |
| 31 - 5422- DSM | FALSE | 0 |
| 32 - 3167- TAM | TRUE | 0 |
| 33 - 5002- BAL | FALSE | 0 |
| 34 - 7461- TS | FALSE | 0 |
| 35 - 1159- LM | FALSE | 0 |
| 36 - 2771- AS | FALSE | 0 |
| 37 - 1434- BDN | FALSE | 0 |
| 38 - 1075 - AMH | FALSE | 1 |
| 39 - 5416- FY | FALSE | 0 |
| 40 - 5587-GAR | FALSE | 7 |

(continued)

| | Death | Days of IP-10>=1000pg/ml |
|---|---|---|
| 41 - 9601-RY | FALSE | 0 |
| 42 - 3692-AMA | FALSE | 1 |
| 43 - 3185-LMH | FALSE | 0 |
| 44 - 3426- AM | FALSE | 3 |
| 45 - 4154- RA | FALSE | 2 |
| 46 - 56824- YH | FALSE | 0 |
| 47 - 3804- KM | FALSE | 0 |
| 48 - 56825-SH | FALSE | 0 |
| 49 - 5930-GMY | FALSE | 0 |
| 50 - 5643-BHN | FALSE | 0 |
| 51 - 3068-YS | FALSE | 0 |
| 52 - 8222-TE | FALSE | 0 |

*Table 19*

| | Death | Days of IP-10>=1000pg/ml |
|---|---|---|
| 1 - 3140- EY | FALSE | 0 |
| 2 - 5011- IG | FALSE | 2 |
| 4 - 5449- BTR | FALSE | 2 |
| 5 - 5557- MP | FALSE | 1 |
| 6 - 2726- SEBB | FALSE | 0 |
| 7 - 4267- HH | FALSE | 0 |
| 8 - 1493- MR | TRUE | 6 |
| 9 - 5065- YK | FALSE | 3 |
| 11 - 4108- SG | TRUE | 9 |
| 18 - 6494- NBT | FALSE | 1 |
| 38 - 1075 - AMH | FALSE | 1 |

Tables 18 and 19 provide data that shows that mortality correlates with the number of days IP-10 >1000 pg/ml in SARS-CoV-2 positive patients (n = 52 - Table 18) and among the subset of ICU patients (n = 12, Table 19). IP-10 was measured using MeMed Key™ at multiple time points during the SARS-CoV-2 positive patient's hospitalization. At least one IP-10 measurement ≥ 1,000 pg/ml in a given day was sufficient to classify as a day > 1,000 pg/ml.

In contrast, the number of days with CRP > 90 mg/l or ferritin > 500 mg/dl was not associated with mortality (Table 20).

*Table 20*

| | Days of CRP>=90mg/l | Days of Ferritin>=500ng/dl | Days of IL-6>=80pg/ml | Days of IL-6>=100pg/ml | Deat h |
|---|---|---|---|---|---|
| 1 - 3140- EY | 0 | 20 | 4 | 4 | FAL SE |

(continued)

| | Days of CRP>=90mg/l | Days of Ferritin>=500ng/dl | Days of IL-6>=80pg/ml | Days of IL-6>=100pg/ml | Deat h |
|---|---|---|---|---|---|
| 11 - 4108- SG | 4 | 6 | 8 | 8 | TRU E |
| 18 - 6494- NBT | 6 | 12 | 0 | 0 | FAL SE |
| 2 - 5011- IG | 1 | 30 | 8 | 8 | FAL SE |
| 3 - 4139- ST | 2 | 17 | 8 | 8 | TRU E |
| 38 - 1075 - AMH | 2 | 2 | 2 | 2 | FAL SE |
| 4 - 5449- BTR | 0 | 18 | 2 | 2 | FAL SE |
| 5 - 5557- MP | 0 | 21 | 2 | 2 | FAL SE |
| 6 - 2726- SEBB | 0 | 1 | 2 | 2 | FAL SE |
| 7 - 4267- HH | 13 | 39 | 12 | 12 | FAL SE |
| 8 - 1493- MR | 5 | 6 | 10 | 10 | TRU E |
| 9 - 5065- YK | 4 | 19 | 15 | 15 | FAL SE |

Overall, 3 ICU patients died; patient #3 died of candidemia. Focusing on the remaining 2 ICU patients who died, IP-10 > 1,000 pg/ml was exhibited for an average of 7.5 days (standard deviation [SD], 2.1 days) in contrast to the 9 surviving patients who exhibited IP-10 > 1,000 pg/ml for an average of 1 day (SD, 1.1 day; p=0.04).

The data in Table 20 shows that that mortality is not correlated with the number of days CRP > 90 mg/l (upper panel) or number of days ferritin > 500 ng/dl (lower panel) in the ICU patients (n = 12). In addition, the data in Table 20 shows that mortality is not correlated with the number of days IL-6 > 80 pg/ml (upper panel) or number of days IL-6 > 100 pg/ml (lower panel) in the ICU patients (n = 12)

CRP and ferritin were measured at multiple time points during the 12 patient's stay in the ICU. At least one CRP > 90 mg/l or ferritin > 500 ng/dl on a given day was sufficient for the analysis. IL-6 was measured at multiple time points during the 12 patient's stay in the ICU. At least one IL-6 > 80 pg/ml (upper panel) or IL-6 > 100 pg/ml in a given day was sufficient for the analysis.

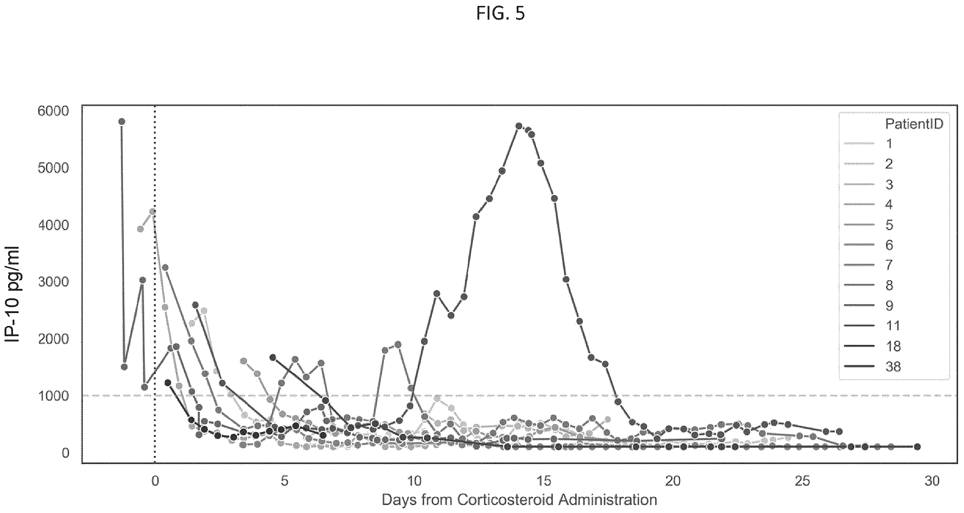

All ICU patients exhibited reduced IP-10 levels within 3 days of their first corticosteroid treatment administered in the ICU (Figure 5). In the case where patients were given additional corticosteroids a decrease in IP-10 levels was observed (Figures 6A-C); patients #8 and #11 who exhibited multiple days of highly elevated IP-10 died. Retrospective examination of IL-6 levels in the ICU patients revealed a weak correlation with IP-10 levels (Pearson correlation coefficient, 0.26) and no association with mortality, both for number of days with IL-6 > 80 pg/ml or IL-6 > 100 pg/ml (Table 20).

***TRAIL levels flag viral persistence***

The MeMed Key™ platform was used to provide measurements of the immune protein TRAIL. Applying the formal criterion of two negative SARS-CoV-2 PCR tests as confirmation of viral clearance, eight out of the 12 ICU patients were clear of the virus within the study period. TRAIL ≤ 25 pg/ml was associated with longer duration of SARS-CoV-2 positivity (p = 0.012). The number of days TRAIL ≤ 25 pg/ml was found to correlate with the number of virus positive days (Pearson correlation coefficient, 0.7).

## EXAMPLE 6

A multinational clinical study for the purpose of developing a novel signature (comprising TRAIL, IP-10 and CRP proteins) that aids in stratifying the likelihood of severe COVID-19 infection was set up. The cohort included SARS-CoV-2 positive adult patients, sampled within 7 days of emergency department (ED) arrival. No exclusion criteria were applied. A patient was defined as severe if he/she met within 14 days from blood draw one or more of the following severe outcomes: mortality or ICU-level care (i.e., ICU admission, high flow nasal cannula, continuous positive airway pressure, bi-level airway pressure, invasive mechanical ventilation).

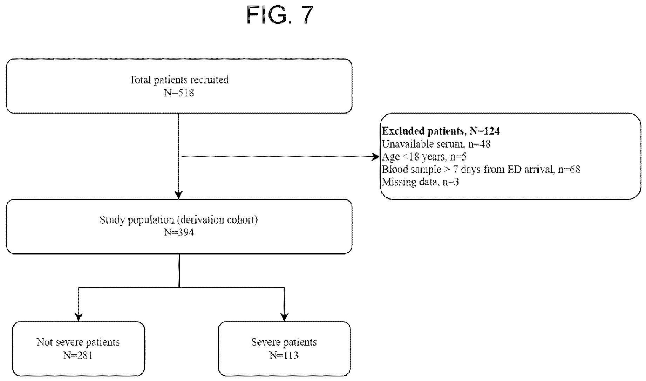

A total of 518 US, Israeli, and German SARS-CoV-2 positive patients were recruited between March 20 and Nov 20, of which 124 were excluded due to unavailability of serum (n=48), age <18 years (n=5), sampling >7 days from ED arrival (n=68) and missing data (n=3). The remaining 394 patients were eligible for inclusion in the derivation cohort, 281 of which were severe and 113 non-severe (Figure 7).

Table 21 shows the demographics of the derivation cohort across severe and non-severe patients. Severe patients exhibited a significantly longer length of stay (Median; 11 vs 5 days; p < 0.01), were more likely to be male (%; 70.8 vs 54.4; p < 0.01) and older (Median; 65 vs 60 years; p < 0.01), in line with previous reports. Significance of difference between proportions was calculated using Fisher's exact test. Significance of difference in numeric variables was calculated using the Mann-Whitney U test.

*Table 21*

|  | Statistics | Severe (n=113) | Non-Severe (n=281) | Derivation cohort (n=394) |
|---|---|---|---|---|
| Age (years) | Median (IQR) | 65.0 (21.5) | 60.0 (27.0) | 61.3 (25.8) |
| Male sex | n (%) | 80 (70.8%) | 153 (54.4%) | 233 (59.1%) |
| Time from ED arrival to blood draw (days) | Median (IQR) | 2.0 (2.0) | 1.0 (2.0) | 1.0 (2.0) |
| Severe outcome |  |  |  |  |

| | | | | |
|---|---|---|---|---|
| ICU admission | n (%) | 51 (45.1%) | 0 (0.0%) | 51 (12.9%) |
| High flow nasal cannula, bilevel airway pressure, or continuous positive airway pressure | n (%) | 65 (57.5%) | 0 (0.0%) | 65 (16.5%) |
| Invasive mechanical ventilation | n (%) | 49 (43.4%) | 0 (0.0%) | 49 (12.4%) |
| Mortality | n (%) | 30 (26.5%) | 0 (0.0%) | 30 (7.6%) |
| Length of hospital stay (days) * | Median (IQR) | 11.0 (13.8) | 5.0 (8.0) | 6.5 (10.0) |
| Coexisting conditions | | | | |
| Hypertension | n (%) | 47 (41.6%) | 100 (35.6%) | 147 (37.3%) |
| Diabetes | n (%) | 36 (31.9%) | 68 (24.2%) | 104 (26.4%) |
| Chronic heart failure | n (%) | 6 (5.3%) | 10 (3.6%) | 16 (4.1%) |
| Active malignancy | n (%) | 8 (7.1%) | 7 (2.5%) | 15 (3.8%) |

## RESULTS

The levels of CRP and IP-10 were found to be significantly higher in patients with severe as compared to non-severe COVID-19 infection: median (and interquartile range) levels of CRP were 167 (178) mg/L vs. 42 (84) mg/L, (p<0.01); and IP-10 levels were 1632 (2250) pg/ml vs. 420 (681) pg/ml, (p<0.01). TRAIL exhibits the opposite pattern, with concentration significantly lower in patients with a severe infection as compared to a non-severe infection: 31 (18) pg/ml vs. 61 (51) pg/ml, (p<0.01).

To integrate the protein levels into a single score the present inventors examined multiple computational models. Among the several top performing models that differentiated between severe and non-severe COVID-19 infection, they chose regularized logistic regression y. Only original features without any polynomial combination were selected in order to reduce the chance of overfitting. The features' discriminatory potential was evaluated by 10-fold cross-validation yielding an AUC of 0.86 (95% CI, 0.81 - 0.9). Adding age or sex to the host-protein features, did not improve results. The final COVID-19 Severity model was trained on the entire derivation cohort and showed comparable results to the 10-fold cross validation indicating that the model was not overfitted.

A pragmatic assessment of performance that helps to translate a risk stratification model into a clinically intuitive and easy-to-use tool is to assign patients into bins with increasing signature scores and evaluate if there is a meaningful increase in the relative likelihood of severe outcome across the bins. To that end the present inventors assigned each patient to a specific bin based on their COVID-19 Severit score, and within the bin according to their severity outcome. As can be seen in Table 22, the relative likelihood of COVID-19 severe outcome significantly increases across the four bins.

### Table 22. Score Distribution Across Bins in the Derivation Cohort

| Score bins | %Patients | n total | $n_{Severe}$ | $n_{NonSevere}$ | % Severe | % NonSevere | *LR (95% CI) |
|---|---|---|---|---|---|---|---|
| 80 < score ≤ 100 | 19.0 | 75 | 54 | 21 | 72.0 | 28.0 | 6.4 (4.1-10.1) |
| 40 ≤ score ≤ 80 | 29.4 | 116 | 46 | 70 | 39.7 | 60.3 | 1.6 (1.2-2.2) |
| 20 < score < 40 | 20.1 | 79 | 10 | 69 | 12.7 | 87.3 | 0.4 (0.2-0.7) |
| 0 ≤ score ≤ 20 | 31.5 | 124 | 3 | 121 | 2.4 | 97.6 | 0.1 (0.0-0.2) |
| Total | 100.0 | 394 | 113 | 281 | | | |

*The Likelihood Ratio (LR) for a score bin was calculated as the fraction of severe patients in the bin out of all severe patients, divided by the fraction of non-severe patients in the bin out of all non-severe patients.

The Cochran–Armitage exact statistical test was used to calculate the significance of the trend of severe and non-severe patient distribution across the score bins, resulting in p-value < 0.01.

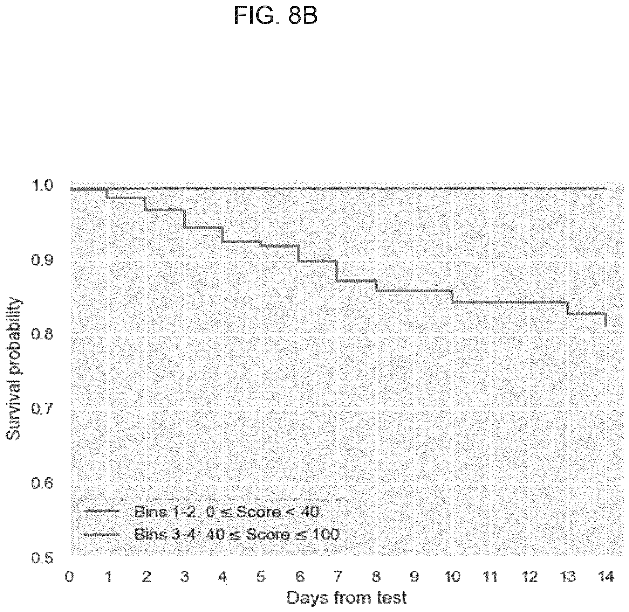

In addition, the present inventors assessed the probability for 14-day mortality with a Kaplan-Meyer survival estimator across the COVID-19 Severity score bins (Figure 8A). In total 30 patients died. Survival distribution was significantly different comparing bins 1-2 to bins 3-4 (Figure 8B; p-value < 0.01).

Several parameters (e.g., age) and biomarkers (e.g., IL-6) have been reported as candidate tools for aiding the clinician in discriminating between severe and non-severe COVID-19 infection. The present inventors compared the performance of the COVID-19 Severity™ algorithm to that of various biomarkers and parameters using AUROC analysis. In the analysis presented in Figure 9, the AUROC was calculated for a given parameter or biomarker based on the subset of patients with the available measurement. Additionally, a head-to-head comparison is presented in Table 23, where AUROC was calculated for all biomarkers based only on the subset of patients that had measurements for both comparator biomarker and the COVID-19 Severity algorithm at time of blood draw. COVID-19 Severity was found to compare favorably to the parameter and biomarkers tested.

**Table 23 Performance of COVID-19 Severity signature Compared to Other Biomarkers**

|  | Comparator AUROC (95% CI) | COVID-19 Severity™ AUROC (95% CI) in matching population | p-value* |
|---|---|---|---|
| Age | 0.58 (0.52, 0.65) | 0.86 (0.81, 0.91) | <0.01 |
| IL6 | 0.77 (0.67, 0.87) | 0.89 (0.81, 0.97) | 0.03 |
| PCT | 0.77 (0.66, 0.88) | 0.90 (0.82, 0.98) | 0.04 |
| CRP | 0.78 (0.73, 0.83) | 0.86 (0.81, 0.91) | <0.01 |
| Ferritin | 0.65 (0.52, 0.78) | 0.80 (0.69, 0.91) | 0.05 |
| Lactate | 0.52 (0.31, 0.74) | 0.84 (0.68, 1.00) | 0.01 |

**EXAMPLE 7**

Particular RNA pairs that can be used to rule in a viral infection of asymptomatic patients are summarized in Table 24 herein below. The genes were selected based on their ability to separate day 0-1 viral patients (time from symptoms) from non-infectious patients or were detected early in a viral challenge study.

**Table 24**

| Gene 1 | Gene 2 | ROC AUC | Sensitivity | Specificity | Fold change (absolute value) |
|---|---|---|---|---|---|
| IFI27 | IFI44L | 1.00 | 1.00 | 1.00 | 183.8 |
| IFI44L | RSAD2 | 1.00 | 1.00 | 1.00 | 183.8 |
| IFI44L | SERPING1 | 1.00 | 1.00 | 1.00 | 94.4 |
| IFI44 | IFI44L | 1.00 | 1.00 | 1.00 | 90.0 |
| IFI44L | IFIT1 | 1.00 | 1.00 | 1.00 | 87.0 |
| IFI44L | OAS3 | 1.00 | 1.00 | 1.00 | 86.0 |
| HERC5 | IFI44L | 1.00 | 1.00 | 1.00 | 60.9 |
| IFI44L | USP18 | 1.00 | 1.00 | 1.00 | 53.1 |
| CMPK2 | IFI44L | 1.00 | 1.00 | 1.00 | 47.6 |
| CARD17 | IFI44L | 1.00 | 1.00 | 1.00 | 38.9 |
| IFI44L | PI3 | 1.00 | 1.00 | 1.00 | 36.5 |
| EPSTI1 | IFI44L | 1.00 | 1.00 | 1.00 | 35.6 |
| IFI44L | MX1 | 1.00 | 1.00 | 1.00 | 32.7 |
| IFI44L | OAS2 | 1.00 | 1.00 | 1.00 | 32.1 |
| IFI44L | IFI6 | 1.00 | 1.00 | 1.00 | 29.3 |
| IFI44L | OASL | 1.00 | 1.00 | 1.00 | 26.8 |
| DDX60 | IFI44L | 1.00 | 1.00 | 1.00 | 25.4 |

(continued)

| Gene 1 | Gene 2 | ROC AUC | Sensitivity | Specificity | Fold change (absolute value) |
|---|---|---|---|---|---|
| IFI44L | OAS1 | 1.00 | 1.00 | 1.00 | 24.7 |
| IFI44L | IFIT3 | 1.00 | 1.00 | 1.00 | 24.1 |
| IFI44L | LY6E | 1.00 | 1.00 | 1.00 | 20.3 |
| IFI44L | IFIH1 | 1.00 | 1.00 | 1.00 | 16.5 |
| IFI44L | PLSCR1 | 1.00 | 1.00 | 1.00 | 16.4 |
| IFI35 | IFI44L | 1.00 | 1.00 | 1.00 | 16.2 |
| IFI44L | LAP3 | 1.00 | 1.00 | 1.00 | 15.6 |
| IFI44L | IFIT2 | 1.00 | 1.00 | 1.00 | 15.0 |
| IFI44L | XAF1 | 1.00 | 1.00 | 1.00 | 14.6 |
| ANKRD22 | IFI44L | 1.00 | 1.00 | 1.00 | 12.4 |
| IFI44L | RTP4 | 1.00 | 1.00 | 1.00 | 12.2 |
| IFI44L | IFIT5 | 1.00 | 1.00 | 1.00 | 11.9 |
| IFI44L | SAMD9L | 1.00 | 1.00 | 1.00 | 11.2 |
| IFI44L | PARP12 | 1.00 | 1.00 | 1.00 | 11.1 |
| GBP1 | IFI44L | 1.00 | 1.00 | 1.00 | 11.1 |
| IFI44L | TNFAIP6 | 1.00 | 1.00 | 1.00 | 10.9 |
| AIM2 | IFI44L | 1.00 | 1.00 | 1.00 | 10.8 |
| IFI44L | PHOSPHO1 | 1.00 | 1.00 | 1.00 | 10.6 |
| EIF2AK2 | IFI44L | 1.00 | 1.00 | 1.00 | 10.3 |
| IFI44L | ZBP1 | 1.00 | 1.00 | 1.00 | 9.6 |
| ETV7 | IFI44L | 1.00 | 1.00 | 1.00 | 9.6 |
| DDX58 | IFI44L | 1.00 | 1.00 | 1.00 | 8.8 |
| IFI44L | STAT2 | 1.00 | 1.00 | 1.00 | 8.7 |
| FAM101B | IFI44L | 1.00 | 1.00 | 1.00 | 8.4 |
| GBP1P1 | IFI44L | 1.00 | 1.00 | 1.00 | 8.3 |
| IFI44L | ZCCHC2 | 1.00 | 1.00 | 1.00 | 8.2 |
| IFI44L | PARP14 | 1.00 | 1.00 | 1.00 | 7.3 |
| DDX60L | IFI44L | 1.00 | 1.00 | 1.00 | 7.3 |
| IFI44L | SAMD9 | 1.00 | 1.00 | 1.00 | 6.8 |
| IFI44L | PARP9 | 1.00 | 1.00 | 1.00 | 6.7 |
| IFI44L | ISG15 | 1.00 | 1.00 | 1.00 | 6.1 |
| CASS4 | IFI44L | 1.00 | 1.00 | 1.00 | 6.0 |
| EMR3 | IFI44L | 1.00 | 1.00 | 1.00 | 5.9 |
| IFI44L | TNFSF10 | 1.00 | 1.00 | 1.00 | 3.3 |
| IFI44L | JUP | 1.00 | 1.00 | 1.00 | 2.8 |
| IFI27 | JUP | 0.93 | 0.86 | 1.00 | 2.8 |
| IFI44L | OTOF | 1.00 | 1.00 | 1.00 | 1.9 |
| IFI44L | LAX1 | 1.00 | 1.00 | 1.00 | 1.8 |

(continued)

| Gene 1 | Gene 2 | ROC AUC | Sensitivity | Specificity | Fold change (absolute value) |
|---|---|---|---|---|---|
| IFI27 | LAX1 | 0.99 | 0.93 | 1.00 | 1.8 |
| JUP | LAX1 | 0.89 | 0.93 | 0.77 | 1.8 |
| EVI2A | IFI44L | 1.00 | 1.00 | 1.00 | 1.1 |

Particular RNA triplets that can be used to rule in a viral infection of asymptomatic patients are summarized in Table 25, herein below. The genes were selected based on their ability to separate day 0-1 viral patients (time from symptoms) from non-infectious patients or were detected early in a viral challenge study.

**Table 25**

| Gene 1 | Gene 2 | Gene 3 | ROC AUC | Sensitivity | Specificity | Fold change (absolute value) |
|---|---|---|---|---|---|---|
| CARD1 7 | IFI44L | PI3 | 1.00 | 1.00 | 1.00 | 36.5 |
| CMPK2 | IFI44L | PI3 | 1.00 | 1.00 | 1.00 | 36.5 |
| HERC5 | IFI44L | PI3 | 1.00 | 1.00 | 1.00 | 36.5 |
| IFI27 | IFI44L | PI3 | 1.00 | 1.00 | 1.00 | 36.5 |
| IFI44 | IFI44L | PI3 | 1.00 | 1.00 | 1.00 | 36.5 |
| IFI44L | IFIT1 | PI3 | 1.00 | 1.00 | 1.00 | 36.5 |
| IFI44L | OAS3 | PI3 | 1.00 | 1.00 | 1.00 | 36.5 |
| IFI44L | PI3 | RSAD2 | 1.00 | 1.00 | 1.00 | 36.5 |
| IFI44L | PI3 | SERPIN G1 | 1.00 | 1.00 | 1.00 | 36.5 |
| IFI44L | PI3 | USP18 | 1.00 | 1.00 | 1.00 | 36.5 |
| EPSTI1 | IFI44L | PI3 | 1.00 | 1.00 | 1.00 | 35.6 |
| IFI44L | MX1 | PI3 | 1.00 | 1.00 | 1.00 | 32.7 |
| IFI44L | OAS2 | PI3 | 1.00 | 1.00 | 1.00 | 32.1 |
| IFI44L | IFI6 | PI3 | 1.00 | 1.00 | 1.00 | 29.3 |
| IFI44L | OASL | PI3 | 1.00 | 1.00 | 1.00 | 26.8 |
| DDX60 | IFI44L | PI3 | 1.00 | 1.00 | 1.00 | 25.4 |
| IFI44L | IFIT3 | PI3 | 1.00 | 1.00 | 1.00 | 24.1 |
| IFI44L | LY6E | PI3 | 1.00 | 1.00 | 1.00 | 20.3 |
| IFI44L | IFIT2 | PI3 | 1.00 | 1.00 | 1.00 | 15.0 |
| IFI44L | PI3 | XAF1 | 1.00 | 1.00 | 1.00 | 14.6 |
| IFI44L | IFIT5 | PI3 | 1.00 | 1.00 | 1.00 | 11.9 |
| IFI44L | PI3 | TNFAIP 6 | 1.00 | 1.00 | 1.00 | 10.9 |
| IFI44L | PHOSPH O1 | PI3 | 1.00 | 1.00 | 1.00 | 10.6 |
| DDX58 | IFI44L | PI3 | 1.00 | 1.00 | 1.00 | 8.8 |
| FAM10 1B | IFI44L | PI3 | 1.00 | 1.00 | 1.00 | 8.4 |
| IFI44L | ISG15 | PI3 | 1.00 | 1.00 | 1.00 | 6.1 |
| CASS4 | IFI44L | PI3 | 1.00 | 1.00 | 1.00 | 6.0 |

(continued)

| Gene 1 | Gene 2 | Gene 3 | ROC AUC | Sensitivity | Specificity | Fold change (absolute value) |
|--------|--------|--------|---------|-------------|-------------|------------------------------|
| EMR3 | IFI44L | PI3 | 1.00 | 1.00 | 1.00 | 5.9 |
| IFI44L | PI3 | TNFSF1 0 | 1.00 | 1.00 | 1.00 | 3.3 |
| IFI44L | JUP | PI3 | 1.00 | 1.00 | 1.00 | 2.8 |
| IFI44L | OTOF | PI3 | 1.00 | 1.00 | 1.00 | 1.9 |
| IFI44L | LAX1 | PI3 | 1.00 | 1.00 | 1.00 | 1.8 |
| IFI27 | JUP | LAX1 | 0.99 | 0.93 | 1.00 | 1.8 |
| EVI2A | IFI44L | PI3 | 1.00 | 1.00 | 1.00 | 1.1 |

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting. In addition, any priority documents of this application are hereby incorporated herein by reference in their entirety.

CLAUSES:

1. A method of classifying the severity of a coronaviral infection of a subject, comprising measuring the TRAIL and/or IP10 protein level in a body liquid sample of the subject, wherein said level is indicative of the severity of the coronaviral infection.

2. The method of clause 1, wherein said subject does not present with symptoms of an infectious disease.

3. The method of clause 1, wherein said subject presents with symptoms of an infectious disease.

4. The method of clause 3, wherein said symptoms comprise fever.

5. The method of clause 1, further comprising measuring the level of at least one determinant set forth in Table 1.

6. The method of clause 5, wherein said at least one determinant is selected from the group consisting of PCT, IL-6 and CRP.

7. The method of clause 1, further comprising measuring the level of at least one determinant selected from the group consisting of ferritin, d-dimer and lactate.

8. The method of any one of clauses 1-7, further comprising measuring the level of CRP.

9. The method of clause 1, further comprising treating the subject who has been classified as severe with a management selected from the group consisting of oxygen therapy, non-invasive ventilation, mechanical ventilation, invasive monitoring, last-resort drug, sedation, intensive care admission, surgical intervention, hospital admittance, anti-viral drug, anti-viral regimen, anti-bacterial drug, anti-fungal drug, immune-globulin treatment isolation, glucocorticoid therapy, extracorporeal membrane oxygenation and kidney replacement therapy.

10. The method of clause 1, wherein the coronavirus of said coronaviral infection is severe acute respiratory syndrome coronavirus (SARS-CoV-2) and Middle East respiratory syndrome coronavirus (MERS-CoV).

11. A method of managing treatment of a disease which brings about a dysregulation of an immune and/or inflammatory response of a subject, said subject already being medicated with a first treatment to treat said disease comprising:

(a) measuring an amount of IP10 and/or TRAIL in a sample of the subject; and
(b) altering the treatment or treatment course when the level of IP10 is above a predetermined threshold and/or when the level of TRAIL is below a predetermined threshold in said sample.

12. The method of clause 11, wherein said disease is a viral disease.

13. The method of clause 12, wherein said viral disease is a respiratory viral disease.

14. The method of clause 13, wherein said respiratory viral disease is COVID-19.

15. The method of clause 13, wherein said respiratory viral disease comprises acute respiratory distress syndrome (ARDS).

16. The method of clause 13, wherein the virus of said respiratory viral disease is selected from the group consisting of RSV, Flu A, Flu B, HCoV and SARS-Cov-2.

17. The method of clause 11, wherein said predetermined threshold for IP10 is above 1000 pg/ml.

18. The method of clause 17, further comprising determining the length of time for which the level of IP10 is above said predetermined threshold, wherein said length of time is indicative that the treatment or treatment course should be altered.

19. The method of clause 11, wherein said predetermined threshold for TRAIL is below 25 pg/ml.

20. The method of clause 19, further comprising determining the length of time for which the level of TRAIL is below a predetermined threshold, wherein said length of time is indicative that the treatment or treatment course should be altered.

21. The method of clause 11, wherein said subject is hospitalized.

22. The method of clause 11, wherein said subject is non-hospitalized.

23. The method of clause 11, wherein said subject is in intensive care.

24. The method of clause 11, further comprising re-measuring the amount of IP10 and/or TRAIL following said altering.

25. The method of clause 24, wherein said re-measuring is effected no more than 12 hours following said altering the treatment.

26. The method of clause 24, wherein steps (a) and (b) are repeated at least three times during the course of the disease.

27. The method of clause 11, wherein said first treatment comprises administering a therapeutic agent.

28. The method of clause 11, wherein said first therapeutic agent comprises a steroid agent.

29. The method of clause 27, wherein said therapeutic agent comprises an agent which reduces the activity or amount of IL-6.

30. The method of clause 27, wherein said altering the treatment comprises altering the dose of said therapeutic agent based on the level of IP10 and/or TRAIL.

31. The method of clause 11, wherein step (b) is effected no more than 12 hours following step (a).

32. The method of clause 11, wherein altering the treatment comprises terminating said first treatment.

33. The method of clause 11, wherein altering the treatment comprises initiating a second treatment.

34. The method of clause 11, wherein altering the treatment further comprises initiating a second treatment.

35. A method of predicting onset of a cytokine storm in a subject having a disease which brings about a dysregulation of an immune and/or inflammatory response, the method comprising:

(a) measuring an amount of IP10 in a sample of the subject; and
(b) predicting a cytokine storm based on the level of IP10.

36. A method of distinguishing between a system shutdown diagnosis and a cytokine storm diagnosis in a subject with a viral disease, comprising measuring the expression level of TRAIL in a sample of the subject, wherein when said level of TRAIL is above a predetermined level, a cytokine storm diagnosis is ruled in and/or when said level of TRAIL is below a predetermined level, a system shutdown diagnosis is ruled in.

37. The method of clauses 35 or 36, further comprising analyzing the level of at least one additional determinant set forth in Tables 1 or 2.

38. The method of clause 35, wherein the amount of IP10 above 1000 pg/ml is indicative of onset of cytokine storm.

39. The method of clauses 35 or 38, wherein the predicting comprises an estimation of the time of onset.

40. The method of clauses 35 or 38, wherein said cytokine storm is the result of an immune-based therapy selected from the group consisting of Tumor-Infiltrating Lymphocyte (TIL) Therapy, Engineered T Cell Receptor (TCR) Therapy, Chimeric Antigen Receptor (CAR) T Cell Therapy and Natural Killer (NK) Cell Therapy.

41. The method of clause 35, wherein said disease is selected from the group consisting of infectious disease, Ulcerative colitis, Crohn's disease, Rheumatoid arthritis, Sjogren syndrome, Kawasaki disease, Guillain-Barre syndrome, Lupus, Asthma, COPD, Multiple sclerosis, vasculitis and Psoriasis.

42. The method of clause 41, wherein said infectious disease is a viral disease.

43. A method of diagnosing the severity of a viral disease of a subject, comprising measuring the expression level of a first determinant set forth in Table 1 and the level of a clinical measurement set forth in Table 2 in a sample of the subject, wherein said levels are indicative of the severity of the viral disease.

44. The method of clause 43, wherein a virus of said viral infection is selected from the group consisting of Adenovirus, Bocavirus, Coronavirus, Enterovirus, Influenza virus, Metapneumovirus, Parainfluenza virus, Respiratory syncytial virus and Rhinovirus.

45. The method of clauses 36, 42 or 43, wherein said viral disease is a respiratory viral disease.

46. The method of clause 36, 42 or 43, wherein said viral disease is transmitted via an influenza virus.

47. The method of clause 36, 42 or 43, wherein said viral disease is transmitted via a coronavirus.

48. The method of clause 47, wherein said coronavirus is selected from the group consisting of severe acute respiratory syndrome coronavirus (SARS-CoV-2) and Middle East respiratory syndrome coronavirus (MERS-CoV).

49. The method of any one of clauses 35-48, further comprising measuring the amount of at least one additional determinant selected from the group consisting of IL-6, PCT and CRP.

50. A method of analyzing the contagiousness of a non-symptomatic subject suffering from an infection, comprising measuring the level of a determinant selected from the group consisting of TRAIL and IP10 in a body liquid sample of the subject, wherein:

(i) when said level of TRAIL is higher than the level of TRAIL in a subject who does not have an infection, it is indicative that the non-symptomatic subject is contagious; and/or
(ii) when said level of IP10 is higher than the level of IP-10 in a subject who does not have an infection, it is indicative that the non-symptomatic subject suffering from an infection is contagious.

51. A method of analyzing the degree of contagiousness of a test subj ect suffering from an infection comprising measuring the level of a determinant selected from the group consisting of TRAIL and IP10 in a body liquid sample of the subject, wherein the level of TRAIL and/or IP10 is proportional to the degree of contagiousness of the test subject suffering from the infection.

52. The method of clause 51, wherein the test subject is non-symptomatic.

53. The method of clause 51, wherein the test subject is symptomatic.

54. The method of clause 52, wherein said non-symptomatic subject has been exposed or is suspected of being exposed to a subject known to have a viral inf ection.

55. The method of clause 52, wherein said non-symptomatic subject is recovering from a viral infection.

56. The method of any one of clauses 50-55, further comprising recommending isolation of said subject upon diagnosis as being contagious.

57. The method of any one of clauses 50-55, further comprising treating the non-symptomatic subject who has been diagnosed as being contagious with a treatment selected from the group consisting of an anti-viral agent, mechanical ventilation, invasive monitoring, last-resort drug, sedation, intensive care admission, surgical intervention, hospital admittance and isolation.

58. The method of clause 50, wherein:

(i) when said level of TRAIL is at least 10 % higher than the level of TRAIL in a subject who does not have an infection, it is indicative that the non-symptomatic subject is contagious; and/or
(ii) when said level of IP10 is at least 25 % higher than the level of TRAIL in a subject who does not have an infection, it is indicative that the non-symptomatic subject is contagious.

59. A method of analyzing biological data, the biological data containing expression values of the TRAIL protein, the IP10 protein, and the CRP protein in a body liquid sample of the subject, the method comprising:

by a hardware processor, receiving the expression values, calculating a distance between a segment of a curved line and an axis defined by a direction, determining a score classifying a severity of a coronaviral infection of the subject based on said distance, and generating an output indicative of said score;
wherein said distance is calculated at a point over said curved line defined by a coordinate $\delta$ along said direction, said coordinate $\delta$ being defined based on the expression values,
wherein at least 90% of said segment is between a lower bound line $f(\delta)-\varepsilon_0$ and an upper bound line $f(\delta)+\varepsilon_1$, wherein each of said $\varepsilon_0$ and said $\varepsilon_1$ is less than 0.5, and wherein said $f(\delta)$ comprises a multiplication between a saturation function of said $\delta$ coordinate, and a saturation function of the expression value of the CRP protein.

60. The method according to clause 59, wherein said saturation function of said $\delta$ coordinate is linearly proportional to $1/(1+Exp(-\delta))$.

61. The method according to any of clauses 59 and 60, wherein said saturation function of the expression value of the CRP protein is linearly proportional to $1/(1 +(CRP/ CRP_0)^{-h})$, where $CRP_0$ and h are predetermined shift and width parameters.

62. The method according to clause 61, wherein said predetermined shift parameter $CRP_0$ is from about 1 mg/L to about 1000 mg/L.

63. The method according to any of clauses 61 and 62, wherein said predetermined width parameter h is from about 2 to about 100.

64. The method according to any of clauses 59-63, wherein said $f(\delta)$ equals $p(1-w)+w$, said p being said saturation function of said $\delta$ coordinate, and said w being said saturation function of the expression value of the CRP protein.

65. The method according to any of clauses 59-64, wherein said coordinate $\delta$ is defined at least based on a linear combination of the expression values.

66. The method according to clause 59, comprising comparing each of the expression values to at least one respective threshold, wherein said coordinate $\delta$ is defined at least based on a result of said comparison.

67. The method according to any of clauses 65 and 66, wherein an offset term of said linear combination is from about 0.9-$\kappa$ to about 0.9+$\kappa$, said $\kappa$ being defined as $\log(p_{max}/(1-p_{max})) - \log(p_{min}/(1-p_{min}))$, said $p_{max}$ being more than 0.9 and less than 1, and said $p_{min}$ being from about 0.01 to about 0.5.

68. The method according to any of clauses 65-67, wherein a coefficient of the expression value of the TRAIL protein in said linear combination is from about -0.04-$\kappa$/ TRAIL$_{min}$ ml/pg to about -0.04+$\kappa$/TRAIL$_{min}$ ml/pg, said $\kappa$ being defined as $\log(p_{max}/(1-p_{max})) - \log(p_{min}/(1-p_{min}))$, said $p_{max}$ being more than 0.9 and less than 1, said $p_{min}$ being from about 0.01 to about 0.5, and said TRAIL$_{min}$ being from about 0 to about 20 pg/ml.

69. The method according to any of clauses 65-68, wherein a coefficient of the expression value of the IP-10 protein in said linear combination is from about 0.0006-$\kappa$/ IP10$_{min}$ ml/pg to about 0.0006+$\kappa$/IP10$_{min}$ ml/pg, said $\kappa$ being defined as $\log(p_{max}/(1-p_{max})) - \log(p_{min}/(1-p_{min}))$, said $p_{max}$ being more than 0.9 and less than 1, said $p_{min}$ being from about 0.01 to about 0.5, and said IP10$_{min}$ being from about 0 to about 150 pg/ml.

70. The method according to any of clauses 65-68, wherein a coefficient of the expression value of the CRP protein in said linear combination is from-about 0.003 $\kappa$/ CRP$_{min}$ L/mg to about 0.003+$\kappa$/CRP$_{min}$ L/mg, said $\kappa$ being defined as $\log(p_{max}/(1-p_{max})) - \log(p_{min}/(1-p_{min}))$, said $p_{max}$ being more than 0.9 and less than 1, said $p_{min}$ being from about 0.01 to about 0.5, and said CRP$_{min}$ being from about 0 to about 1.5 mg/L.

## Claims

1. A method of managing treatment of a disease which brings about a dysregulation of an immune and/or inflammatory response of a subject, said subject already being medicated with a first treatment to treat said disease comprising:

   (a) measuring an amount of IP10 and/or TRAIL in a sample of the subject; and
   (b) providing a recommendation to alter the treatment or treatment course when the level of IP10 is above a predetermined threshold and/or when the level of TRAIL is below a predetermined threshold in said sample.

2. The method of claim 1, wherein said disease is a viral disease.

3. The method of claim 2, wherein said viral disease is a respiratory viral disease.

4. The method of claim 3, wherein the virus of said respiratory viral disease is selected from the group consisting of acute respiratory distress syndrome (ARDS), RSV, Flu A, Flu B, HCoV and SARS-Cov-2.

5. The method of claim 1, wherein said predetermined threshold for IP10 is above 1000 pg/ml.

6. The method of claim 5, further comprising determining the length of time for which the level of IP10 is above said predetermined threshold, wherein said length of time is indicative that the treatment or treatment course should be altered.

7. The method of claim 1, wherein said predetermined threshold for TRAIL is below 25 pg/ml.

8. The method of claim 7, further comprising determining the length of time for which the level of TRAIL is below a predetermined threshold, wherein said length of time is indicative that the treatment or treatment course should be altered.

9. The method of claim 1, wherein said subject is hospitalized.

10. The method of claim 1, wherein said first treatment comprises a first therapeutic agent.

11. The method of claim 10, wherein said first therapeutic agent comprises a steroid or an agent which reduces the activity or amount of IL-6.

**12.** The method of claim 10, wherein said recommendation comprises altering the dose of said first therapeutic agent based on the level of IP 10 and/or TRAIL.

**13.** The method of claim 1, wherein step (b) is effected no more than 12 hours following step (a).

**14.** The method of claim 1, wherein said recommendation comprises terminating said first treatment.

**15.** The method of claim 1, wherein said recommendation comprises initiating a second treatment.

FIG. 1A

FIG. 1B

**TRAIL Dynamics**

TRAIL %change from baseline

Time from symptoms (hours)

**IP‑10 Dynamics**

IP‑10 %change from baseline

Time from symptoms (hours)

FIG. 1C

CRP Dynamics

EP 4 484 956 A2

FIG. 2A

FIG. 2B

TRAIL

IP10

FIG. 3

## FIG. 4

| GOALS | Improve O2 delivery | Control Inflammation | Viral Clearance |
|---|---|---|---|
| **GUIDANCE TOOLS** | D-dimer P/ F ratio Troponin US CT Echocardiography NICaS | Ferritin CRP IP-10 | Viral load Plasma Abs TRAIL |
| **INTERVENTIONS** | Fluids NO Vasopressors Enoxaparin Heparin TPA Prone position Invasive Mechanical Ventilation ECMO | Tocilizumab Corticosteroids Theranova dialyzer | Hydroxychloroquine Remdesivir Lopinavir/ Ritonavir Azithromycin Convalescent Plasma Zinc |

FIG. 5

FIG. 6A

Patient 11

FIG. 6B

## Patient 8

EP 4 484 956 A2

FIG. 6C

Patient 2

# FIG. 7

```
                          Total patients recruited
                                 N=518
```

```
                                              Excluded patients, N=124
                                              Unavailable serum, n=48
                                              Age <18 years, n=5
                                              Blood sample > 7 days from ED arrival, n=68
                                              Missing data, n=3
```

```
                      Study population (derivation cohort)
                                 N=394
```

```
        Not severe patients              Severe patients
             N=281                           N=113
```

# FIG. 8A

FIG. 8B

# FIG. 9

TRAIL (AUC=0.82, N=394)
IP-10 (AUC=0.75, N=394)
CRP (AUC=0.78, N=394)
IL6 (AUC=0.77, N=139)
PCT (AUC=0.77, N=106)
Ferritin (AUC=0.65, N=71)
Lactate (AUC=0.52, N=47)
Age (AUC=0.58, N=394)
**COVID−19 Severity (AUC=0.86, N=394)**

310
begin

311
calculate a distance between a segment of a curved line and an axis

312
determine a score classifying the severity of the infection

313
generate an output indicative of the score

314
treat the subject

315
end

FIG. 10

$S_{UB}$

$S$

$S_{LB}$

$S_{ROI}$

$d$

$\pi$

$\delta_{MIN}$  $P(\delta)$  $\delta_{MAX}$  $\underline{\delta}$

$\pi_{ROI}$

FIG. 11

FIG. 12A

320 — 322

$S_{ROI}$

FIG. 12B

320 —

$S_{ROI}$

FIG. 12C

320 —

$S_{ROI}$

FIG. 12D

$S_{ROI}$

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62967409
- US 62975802
- US 63019451
- US 63006758
- US 63014214 B
- US 63030937 B
- US 63085189 B
- WO 2013117746 A
- WO 2016024278 A
- WO 2018060998 A
- WO 2018060999 A
- US 4727022 A, Skold
- US 4659678 A, Forrest
- US 4376110 A, David
- US 4275149 A, Litman
- US 4233402 A, Maggio
- US 4230767 A, Boguslaski
- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A

### Non-patent literature cited in the description

- **E. MAGGIO.** Enzyme-Immunoassay. CRC Press, Inc, 1980
- The Immunoassay Handbook. 2005
- **NUOVO GJ et al.** Intracellular localization of polymerase chain reaction (PCR)-amplified hepatitis C cDNA. *Am J Surg Pathol.,* 1993, vol. 17, 683-90
- **KOMMINOTH P et al.** Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. *Pathol Res Pract.,* 1994, vol. 190, 1017-25
- DNA Microarrays: Analyzing Genome-Wide Expression. Molecular Cell Biology. W. H. Freeman, 2000
- **D'AGOSTINO et al.** *JAMA,* 2001, vol. 286, 180-187
- **O'MARCAIGH AS ; JACOBSON RM.** Estimating The Predictive Value Of A Diagnostic Test, How To Prevent Misleading Or Confusing Results. *Clin. Ped.,* 1993, vol. 32 (8), 485-491
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- **FRESHNEY.** Culture of Animal Cells - A Manual of Basic Technique. Wiley-Liss, 1994
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- A Practical Guide to Molecular Cloning. 1984

- Methods in Enzymology. Academic Press, vol. 1-317
- PCR Protocols: A Guide To Methods And Applications. Academic Press, 1990

- **MARSHAK et al.** Strategies for Protein Purification and Characterization - A Laboratory Course Manual. CSHL Press, 1996